(19) Europäisches Patentamt
European Patent Office
Office européen des brevets

(11) EP 4 418 842 A1

(12) **EUROPEAN PATENT APPLICATION**

(43) Date of publication:
**21.08.2024 Bulletin 2024/34**

(21) Application number: **23156500.3**

(22) Date of filing: **14.02.2023**

(51) International Patent Classification (IPC):
***H10K 85/60*** *(2023.01)* *H10K 50/16* *(2023.01)*

(52) Cooperative Patent Classification (CPC):
**H10K 85/654; H10K 85/6572;** H10K 50/166

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Novaled GmbH**
**01099 Dresden (DE)**

(72) Inventors:
- **SCHOLZ, Johannes**
  **01099 Dresden (DE)**
- **GANIER, Jermone**
  **01099 Dresden (DE)**
- **GALÁN GARCÍA, Elena**
  **01099 Dresden (DE)**

(74) Representative: **Bittner, Thomas L.**
**Boehmert & Boehmert**
**Anwaltspartnerschaft mbB**
**Pettenkoferstrasse 22**
**80336 München (DE)**

(54) **ORGANIC LIGHT EMITTING DIODE, DISPLAY DEVICE COMPRISING THE SAME AND COMPOUND**

(57) The present invention relates to an organic light emitting diode and a display device comprising the same. The invention further relates to a compound which can be used in the organic light emitting diode.

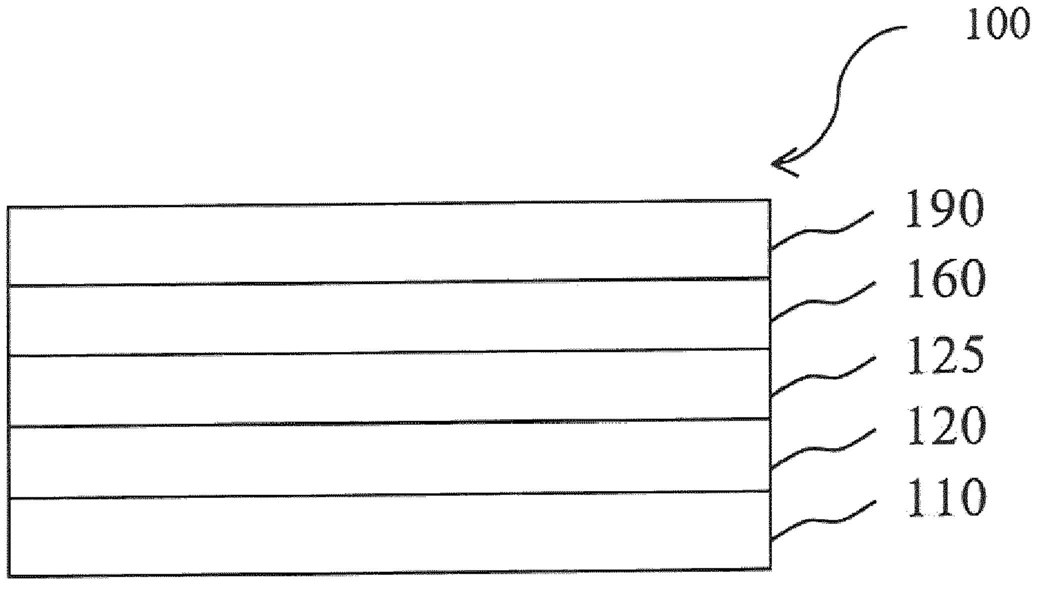


Fig.1



Processed by Luminess, 75001 PARIS (FR)

## Description

TECHNICAL FIELD

The present invention relates to an organic light emitting diode and a device comprising the same. The invention further relates to a compound which can be used in the organic light emitting diode.

BACKROUND OF THE INVENTION

Organic semiconducting devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode may be affected by characteristics of the organic semiconductor layer, and among them, may be affected by characteristics of an organic material of the organic semiconductor layer.

Particularly, development of an organic semiconductor layer being capable of increasing electron mobility and simultaneously increasing electrochemical stability is needed so that the organic semiconducting device, such as an organic light emitting diode, may be applied to a large-size flat panel display.

It is, therefore, the object of the present invention to provide organic light emitting diodes and compounds for preparing the same overcoming drawbacks of the prior art, in particular providing compounds for use in organic light emitting diodes comprising the same helpful to improve the performance thereof, especially with respect to a efficiency.

DISCLOSURE

The object is achieved by an organic light emitting diode comprising a non-transparent substrate, an anode, a cathode, an emission layer, and an electron transport layer stack;
wherein

- the electron transport layer stack is arranged between the emission layer and the cathode;
- the electron transport layer stack comprises optionally a first electron transport layer;
- the electron transport layer stack comprises a second electron transport layer;
- the second electron transport layer is arranged between the emission layer and the cathode;
- the second electron transport layer comprises a compound of formula (II)

$$(Ar^2)_m\text{-}(Z_k\text{-}G)_n \qquad \text{(II)}$$

- n is 2 or more;
- m is 1 or 2;
- k is o, 1 or 2;
- $Ar^2$ is independently selected from the group consisting of substituted or unsubstituted $C_2$ to $C_{42}$ heteroaryl and substituted or unsubstituted $C_6$ to $C_{60}$ aryl;
    - wherein, if $Ar^2$ is substituted, the one or more substituent(s) are independently selected from the group consisting of D, $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially

or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

- wherein each $C_6$ to $C_{12}$ aryl substituent on $Ar^2$ and each $C_3$ to $C_{11}$ heteroaryl substituent on X may be substituted with D, $C_1$ to $C_4$ alkyl or halogen;

- Z is independently selected from substituted or unsubstituted $C_6$ to $C_{30}$ aryl and substituted or unsubstituted $C_2$ to $C_{42}$ heteroaryl;

  - wherein, if Z is substituted, the one or more substituent(s) are independently selected from the group consisting of D, $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;
  - wherein each $C_6$ to $C_{12}$ aryl substituent on Z and each $C_3$ to $C_{11}$ heteroaryl substituent on X may be substituted with D, $C_1$ to $C_4$ alkyl or halogen;

- G comprises at least one 6-membered heteroaromatic ring, wherein the 6-membered heteroaromatic ring comprises one N-atom;

- G is chosen so that the dipole moment of a compound G-phenyl is $\geq 1$ D and $\leq 7$ D; and

- the first electron transport layer and the second electron transport layer are free of an electrical dopant.

The object is further achieved by a display device comprising the organic light emitting diode according to the present invention.

The object is further achieved by a compound of formula (IV)

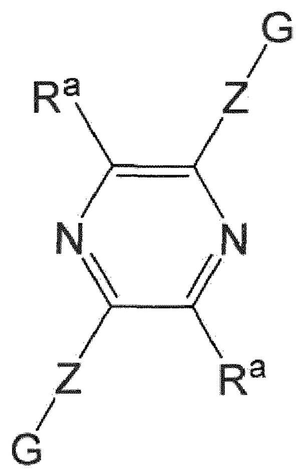

(IV)

wherein

$R^a$ is independently selected from the group consisting of substituted or unsubstituted $C_6$ to $C_{24}$ aryl and substituted or unsubstituted $C_3$ to $C_{18}$ heteroaryl, wherein if $R^a$ is substituted, the one or more substituents are independently elected from the group consisting of D, phenyl, and $C_1$ to $C_{12}$ alkyl;

Z is independently selected from the group consisting of a single bond and $C_6$ to $C_{24}$ arylene;

G is independently selected from (IVa-1) to (IVa-3)

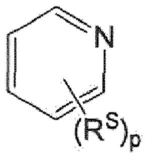 (IVa-1) 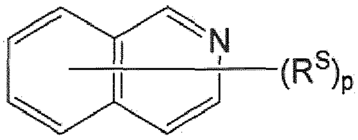 (IVa-2) 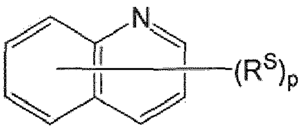 (IVa-3);

p is an integer from o to 4 for IVa-1;

p is an integer from o to 6 for IVa-2 and IVa-3; and

$R^S$ is independently selected from the group consisting of D, $C_1$ to $C_{12}$ aryl, $C_1$ to $C_{12}$ alkyl, and pyridyl, wherein the respective $C_1$ to $C_{12}$ aryl and pyridyl can be unsubstituted or substituted with one or more of D and $C_1$ to $C_4$ alkyl.

Second electron transport layer

The organic light emitting diode in accordance with the present invention mandatorily comprises a second electron transport layer. The second electron transport layer is free of an electrical dopant, such as an n-type dopant, especially a redox n-type dopant.

The term "free of" in this regard does not exclude impurities. Impurities have no technical effect with respect to the object achieved by the present invention. Impurities are not deliberately added to the layer during processing.

The term "free of" a compound means that such compound is not deliberately added to the layer during processing.

Under electrical dopant, especially n-type dopant it is understood a compound which, if embedded into an electron transport matrix, improves, in comparison with the neat matrix under the same physical conditions, the electron properties of the formed organic material, particularly in terms of electron injection and/or electron conductivity.

In the context of the present invention "embedded into an electron transport matrix" means homogenously mixed with the electron transport matrix.

The electrical dopant as referred to herein is especially selected from elemental metals, metal salts, metal complexes and organic radicals.

In one embodiment, the electrical dopant is selected from alkali metal salts and alkali metal complexes; preferably from lithium salts and lithium organic complexes; more preferably from lithium halides and lithium organic chelates; even more preferably from lithium fluoride, a lithium quinolinolate, lithium borate, lithium phenolate, lithium pyridinolate or from a lithium complex with a Schiff base ligand; most preferably,

- the lithium complex has the formula II, III or IV:

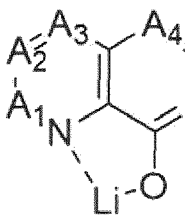 (II), 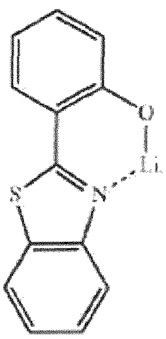 (III), 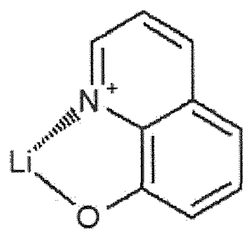 (IV)

wherein

$A_1$ to $A_6$ are same or independently selected from CH, CR, N, O;

R is same or independently selected from hydrogen, halogen, alkyl or aryl or heteroaryl with 1 to 20 carbon atoms; and more preferred $A_1$ to $A_6$ are CH,

- the borate based organic ligand is a tetra(1H-pyrazol-1-yl)borate,

- the phenolate is a 2-(pyridin-2-yl)phenolate, a 2-(diphenylphosphoryl)phenolate, an imidazol phenolate, 2-(pyridin-2-yl)phenolate or 2-(1-phenyl-1H-benzo[d]imidazol-2-yl)phenolate,

- the pyridinolate is a 2-(diphenylphosphoryl)pyridin-3-olate,

- the lithium Schiff base has the structure 100, 101, 102 or 103:

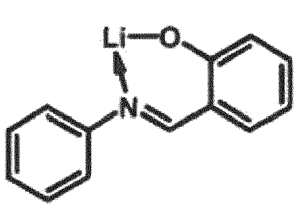

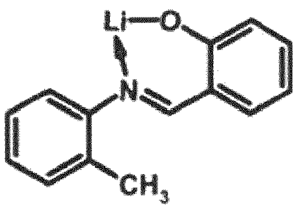

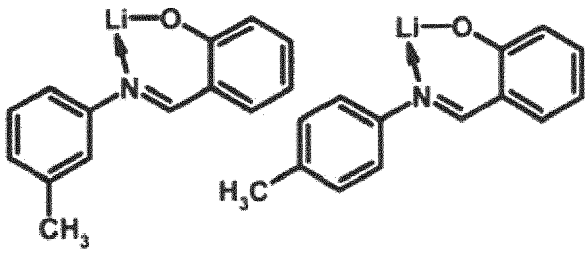

100 101 102 103

According to one embodiment of the invention, the electron transport layer of the present invention is free of a lithium organic complex, alternatively 8-hydroxyquinolinolato-lithium (= LiQ).

According to one embodiment of the present invention the electron transport layer is free of a metal, preferably selected from alkali metals, alkaline earth metals, rare earth metals and metals of the first transition period Ti, V, Cr and Mn, especially selected from Li, Na, K, Rb, Cs, Mg, Ca, Sr, Ba, Sm, Eu, Tm, Yb; more preferably from Li, Na, K, Rb, Cs, Mg and Yb, even more preferably from Li, Na, Cs and Yb, most preferably from Li, Na and Yb.

The most practical benchmark for the strength of an n-dopant is the value of its redox potential. There is no particular limitation in terms how negative the value of the redox potential can be.

As reduction potentials of usual electron transport matrices used in organic semiconductors are, if measured by cyclic voltammetry against ferrocene/ferrocenium reference redox couple, roughly in the range from about - 0.8 V to about - 3.1V; the practically applicable range of redox potentials for n-type dopants which can effectively n-dope such matrices is in a slightly broader range, from about - 0.5 to about - 3.3 V.

The measurement of redox potentials is practically performed for a corresponding redox couple consisting of the reduced and of the oxidized form of the same compound.

In case that the n-type dopant is an electrically neutral metal complex and/or an electrically neutral organic radical, the measurement of its redox potential is actually performed for the redox couple formed by

(i) the electrically neutral metal complex and its cation radical formed by an abstraction of one electron from the electrically neutral metal complex, or

(ii) the electrically neutral organic radical and its cation formed by an abstraction of one electron from the electrically neutral organic radical.

Preferably, the redox potential of the electrically neutral metal complex and/or of the electrically neutral organic radical may have a value which is more negative than - 0.5 V, preferably more negative than - 1.2 V, more preferably more negative than - 1.7 V, even more preferably more negative than - 2.1 V, most preferably more negative than - 2.5 V, if measured by cyclic voltammetry against ferrocene/ferrocenium reference redox couple for a corresponding redox couple consisting of

(i) the electrically neutral metal complex and its cation radical formed by an abstraction of one electron from the electrically neutral metal complex, or

(ii) the electrically neutral organic radical and its cation formed by an abstraction of one electron from the electrically neutral organic radical.

In a preferred embodiment, the redox potential of the n-dopant is between the value which is about 0.5 V more positive and the value which is about 0.5 V more negative than the value of the reduction potential of the chosen electron transport matrix.

Electrically neutral metal complexes suitable as n-type dopants may be e.g. strongly reductive complexes of some transition metals in low oxidation state. Particularly strong n-type dopants may be selected for example from Cr(II), Mo(II) and/or W(II) guanidinate complexes such as $W_2(hpp)_4$, as described in more detail in WO2005/086251.

Electrically neutral organic radicals suitable as n-type dopants may be e.g. organic radicals created by supply of additional energy from their stable dimers, oligomers or polymers, as described in more detail in EP 1837 926 B1, WO2007/107306, or WO2007/107356. Under an elemental metal, it is understood a metal in a state of a neat metal, of a metal alloy, or in a state of free atoms or metal clusters. It is understood that metals deposited by vacuum thermal evaporation from a metallic phase, e.g. from a neat bulk metal, vaporize in their elemental form. It is further understood that if the vaporized elemental metal is deposited together with a covalent matrix, the metal atoms and/or clusters are embedded in the covalent matrix. In other words, it is understood that any metal-doped covalent material prepared by vacuum thermal evaporation contains the metal at least partially in its elemental form.

For the use in consumer electronics, only metals containing stable nuclides or nuclides having very long halftime of radioactive decay might be applicable. As an acceptable level of nuclear stability, the nuclear stability of natural potassium can be taken.

In one embodiment, the electrical may be selected from electropositive metals selected from alkali metals, alkaline earth metals, rare earth metals and metals of the first transition period Ti, V, Cr and Mn. Preferably, the n-dopant may be selected from Li, Na, K, Rb, Cs, Mg, Ca, Sr, Ba, Sm, Eu, Tm, Yb; more preferably from Li, Na, K, Rb, Cs, Mg and Yb, even more preferably from Li, Na, Cs and Yb, most preferably from Li, Na and Yb.

The second electron transport layer may be arranged between the first electron transport layer and the electron injection layer. The second electron transport layer may be arranged in direct contact with the first electron transport layer. The second electron transport layer may be arranged "contacting sandwiched" between the first electron transport layer and the electron injection layer.

The second electron transport layer may have a thickness of < 100 nm, optionally between 10 and 90 nm, optionally between 10 and 60nm, optionally between 10 and 50 nm.

Compound of formula (II)

The second electron transport layer comprises a compound of formula (II)

$$(Ar^2)_m\text{-}(Z_k\text{-}G)_n \qquad (II).$$

The second electron transport layer may consist of the compound of formula (II). The second electron transport layer may comprise or consist of the compound of formula (II) and one or more further compounds, such as the compound (III) as defined herein, provided that none of the further compounds is an electrical dopant. The second electron transport layer may comprise more than one compound of formula (II). The electron transport layer may consist of a mixture of the compound of formula (II) and the compound (III). Exemplary further electron transport matrix compounds which may be contained are disclosed below.

In the compound of formula (II), the group "Z" is a spacer moiety connecting (if present, that is in case that k > 1) the groups $Ar^2$ and G. For each of the groups ($Z_k$-G) the groups may or may not independently comprise the spacer Z.

In formula (II) n is 2 or more. In formula (II) n may be 2 to 4. In formula (II), n may be 2.

In formula (II), m is independently 1 or 2. In formula (II), m may be 1.

In formula (II), k is independently o, 1 or 2. In formula (II), k may be independently 1 or 2.

$Ar^2$ may be independently selected from the group consisting of substituted or unsubstituted $C_2$ to $C_{39}$ heteroaryl and substituted or unsubstituted $C_6$ to $C_{54}$ aryl, optionally substituted or unsubstituted $C_2$ to $C_{36}$ heteroaryl and substituted or unsubstituted $C_6$ to $C_{48}$ aryl, optionally substituted or unsubstituted $C_3$ to $C_{30}$ heteroaryl and substituted or unsubstituted $C_6$ to $C_{42}$ aryl, and optionally substituted or unsubstituted $C_3$ to $C_{28}$ heteroaryl and substituted or unsubstituted $C_6$ to $C_{30}$ aryl.

$Ar^2$ may be independently selected from the group consisting of substituted or unsubstituted $C_2$ to $C_{39}$ N-containing heteroaryl and substituted or unsubstituted $C_6$ to $C_{54}$ aryl, optionally substituted or unsubstituted $C_2$ to $C_{36}$ N-containing heteroaryl and substituted or unsubstituted $C_6$ to $C_{48}$ aryl, optionally substituted or unsubstituted $C_3$ to $C_{30}$ N-containing heteroaryl and substituted or unsubstituted $C_6$ to $C_{42}$ aryl, and optionally substituted or unsubstituted $C_3$ to $C_{28}$ N-containing heteroaryl and substituted or unsubstituted $C_6$ to $C_{30}$ aryl. In this regard, it may be provided that a respective N-containing heteroaryl comprises one or more N-atoms as the only heteroatom(s).

$Ar^2$ may comprise at least two annelated 5- or 6-membered rings.

$Ar^2$ may independently selected from the group consisting of pyridinyl, triazinyl, pyrimidinyl, pyrazinyl, quinoxalinyl; quinazolinyl, benzoquinazolinyl, benzimidazolyl, quinolinyl, benzoquinolinyl, benzoacridinyl, dibenzoacridinyl, fluoranthenyl, anthracenyl, naphthyl, triphenylenyl, phenathrolinyl, and dinaphthofuranyl, which may be substituted or unsubstituted, respectively.

If the respective group is substituted, the one or more substituent(s) may be independently selected from the group consisting of D, $C_6$ to $C_{24}$ aryl, $C_3$ to $C_{21}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, wherein each $C_6$ to $C_{24}$ aryl substituent on $Ar^2$ and each $C_3$ to $C_{21}$ heteroaryl substituent on $Ar^2$ may be substituted with D, $C_1$ to $C_4$ alkyl or halogen.

If the respective group is substituted, the one or more substituent(s) may be independently selected from the group consisting of

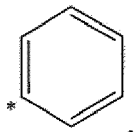 , 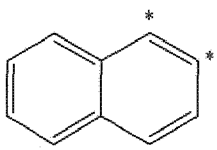 , 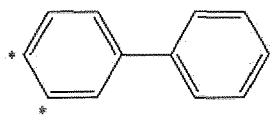 , 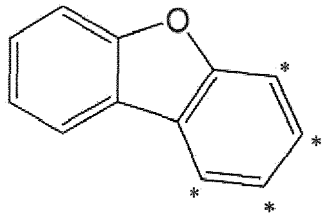 ,

wherein the respective substituent is bonded at one of the * to $Ar_2$.

$Ar^2$ may be substituted pyrazinyl, wherein the one or more substituent(s) may be independently selected from the group consisting of D, $C_6$ to $C_{24}$ aryl, $C_3$ to $C_{21}$ heteroaryl, and $C_1$ to $C_6$ alkyl, wherein each $C_6$ to $C_{24}$ aryl substituent on $Ar^2$ and each $C_3$ to $C_{21}$ heteroaryl substituent on $Ar^2$ may be substituted with D, $C_1$ to $C_4$ alkyl or halogen.

$Ar^2$ may be substituted pyrazinyl, wherein the one or more substituent(s) may be independently selected from the group consisting of $C_6$ to $C_{24}$ aryl and $C_3$ to $C_{21}$ heteroaryl, wherein each $C_6$ to $C_{24}$ aryl substituent on $Ar^2$ and each $C_3$ to $C_{21}$ heteroaryl substituent on $Ar^2$ may be substituted with D, $C_1$ to $C_4$ alkyl or halogen.

$Ar^2$ may be substituted pyrazinyl, wherein the one or more substituent(s) may be independently selected from the group consisting of $C_6$ to $C_{24}$ aryl and $C_3$ to $C_{21}$ heteroaryl.

$Ar^2$ including all substituents may be independently selected from the following structures

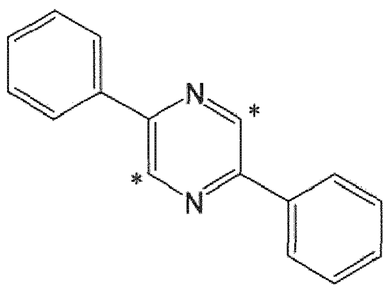 ; 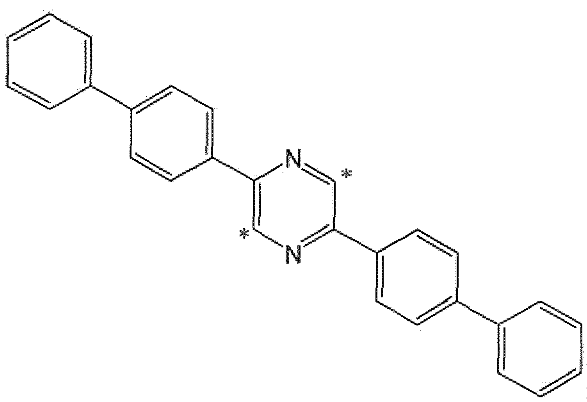 ;

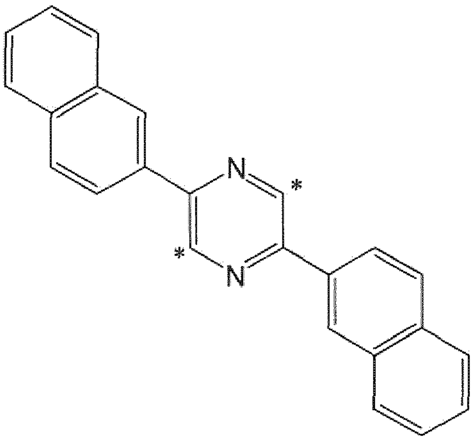;
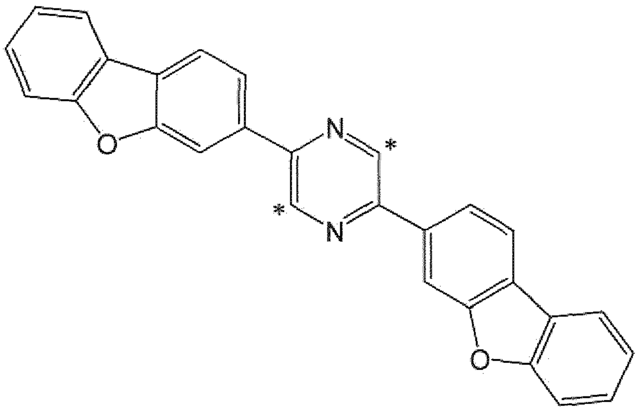

wherein two ($Z_k$-G) are bonded to the respective $Ar^2$ at *, respectively.

$Ar^2$ including all substituents may be independently selected from the following structures

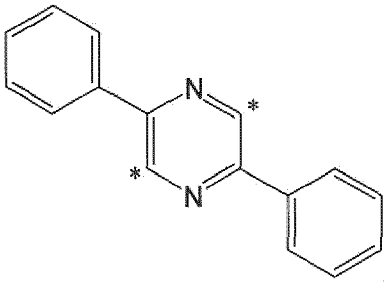;

wherein two ($Z_k$-G) are bonded to the respective $Ar^2$ at *, respectively.

Z may be independently selected from substituted or unsubstituted $C_6$ to $C_{24}$ aryl and substituted or unsubstituted $C_2$ to $C_{21}$ heteroaryl. Z may be independently selected from substituted or unsubstituted $C_6$ to $C_{18}$ aryl and substituted or unsubstituted $C_2$ to $C_{15}$ heteroaryl. Z may be independently selected from substituted or unsubstituted $C_6$ to $C_{12}$ aryl and substituted or unsubstituted $C_2$ to $C_{11}$ heteroaryl. If the respective group is substituted, the one or more substituent(s) are independently selected from the group consisting of D, $C_1$ to $C_{12}$ alkyl and phenyl.

Z may be independently selected from the following groups

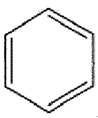; 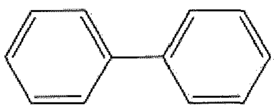; 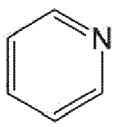; 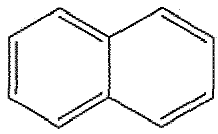; 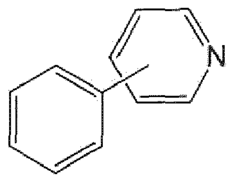;
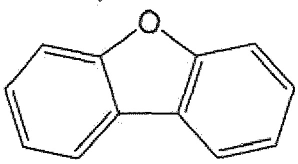;

wherein the respective structure may be unsubstituted or substituted with one or more substituent(s) selected from the group consisting of D, $C_1$ to $C_{12}$ alkyl and phenyl.

Z may be independently selected from the following groups

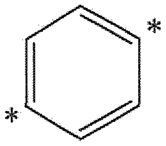 ; 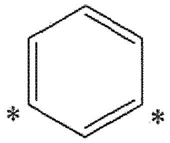 ; 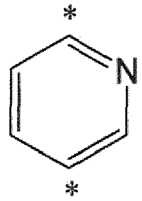 ; 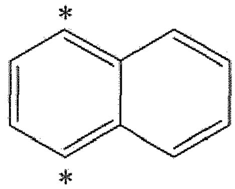 ; 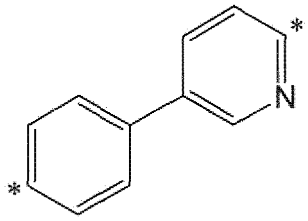 ;

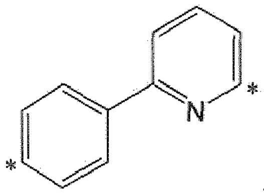 , 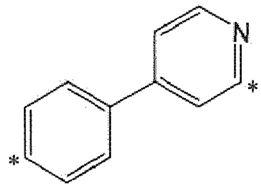

wherein Z is bonded to $Ar^2$ and G at *, respectively.

Z may be independently selected from the following groups

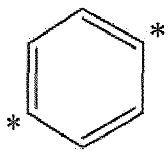 ; 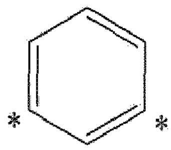 ,

wherein Z is bonded to $Ar^2$ and G at *, respectively.

G comprises at least one 6-membered heteroaromatic ring, wherein the 6-membered heteroaromatic ring comprises one N-atom. The 6-membered heteroaromatic ring comprising one N-atom may be unsubstituted pyridine (G is pyridine), substituted pyridine (G comprises besides the pyridine ring one or more further group(s), wherein the one or more further group(s) is/are bonded to the pyridine ring by single bonds, respectively), or may be part of a ring system of two or more annelated rings, preferably of two or more annelated (hetero)aromatic rings, most preferred two annelated (hetero)aromatic rings, for example may be quinoline.

G may be selected from substituted or unsubstituted $C_5$ to $C_{42}$ heteroaryl comprising at least one 6-membered heteroaromatic ring, wherein the 6-membered heteroaromatic ring comprises one N-atom. G may be selected from substituted or unsubstituted $C_5$ to $C_{35}$ heteroaryl comprising at least one 6-membered heteroaromatic ring, wherein the 6-membered heteroaromatic ring comprises one N-atom. G may be selected from substituted or unsubstituted $C_5$ to $C_{29}$ heteroaryl comprising at least one 6-membered heteroaromatic ring, wherein the 6-membered heteroaromatic ring comprises one N-atom. G may be selected from substituted or unsubstituted $C_5$ to $C_{23}$ heteroaryl comprising at least one 6-membered heteroaromatic ring, wherein the 6-membered heteroaromatic ring comprises one N-atom. G may be selected from substituted or unsubstituted $C_5$ to $C_{17}$ heteroaryl comprising at least one 6-membered heteroaromatic ring, wherein the 6-membered heteroaromatic ring comprises one N-atom. G may be selected from substituted or unsubstituted $C_5$ to $C_{15}$ heteroaryl comprising at least one 6-membered heteroaromatic ring, wherein the 6-membered heteroaromatic ring comprises one N-atom. If the respective G is substituted, the one or more substituents independently selected from the group consisting of D, phenyl, naphthyl, $C_1$ to $C_{12}$ alky, and pyridyl.

G may be selected from the group consisting of

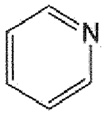 ; 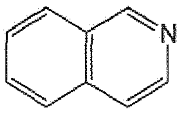 ; and 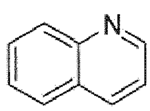 ,

wherein the respective structure may be unsubstituted or substituted with one or more substituents independently selected from the group consisting of D, phenyl, naphthyl, $C_1$ to $C_{12}$ alky, and pyridyl.

G including all substituents may be selected from the following groups

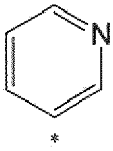 ; 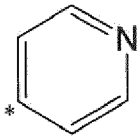 ; 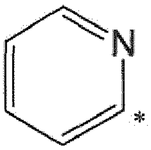 ; 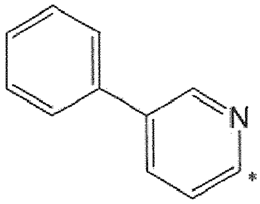 ; 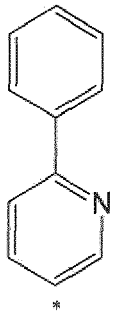 ; 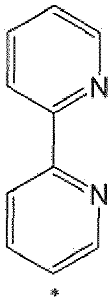 ; 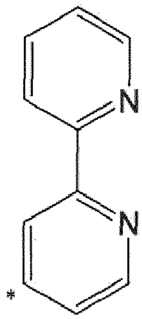 ;

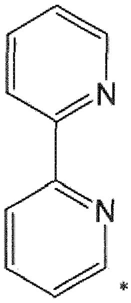 ; 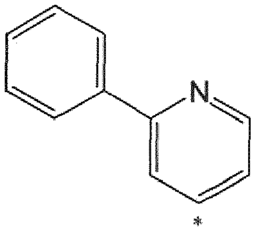 ; 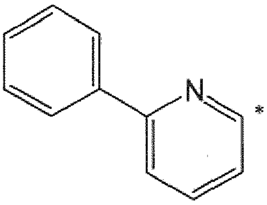 ; 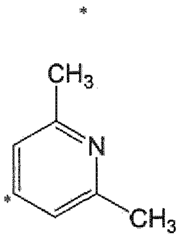 ; 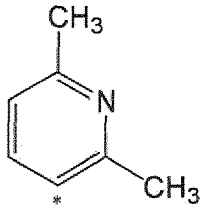 ;

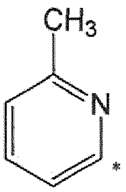 ; 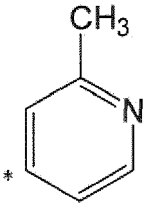 ; 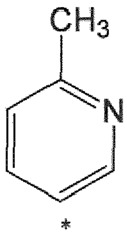 ; 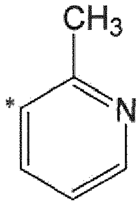 ; 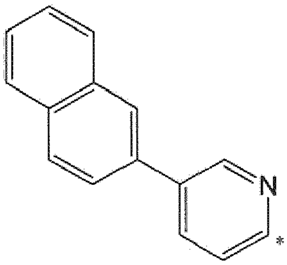 ; 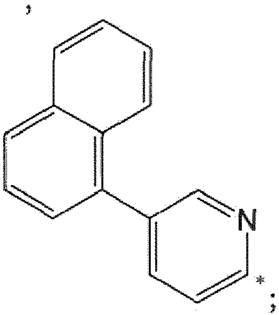 ;

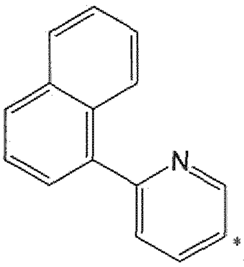 ; 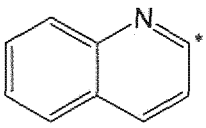 ; 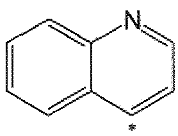 ; 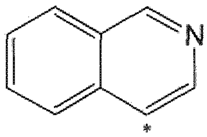 ; 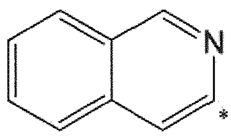 ,

wherein the respective G is bonded to Z at *.

G including all substituents may be

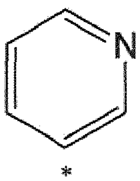

wherein G is bonded to Z at *.

G is chosen so that the dipole moment of a compound G-phenyl is ≥ 1 D and ≤ 7 D

G is chosen so that the dipole moment, computed by the TURBOMOLE V6.5 program package using hybrid functional B3LYP and Gaussian 6-31G* basis set, of a compound G-phenyl is ≥ 1 D and ≤ 7 D. The unit for the dipole

moment "Debye" is abbreviated with the symbol "D". The inventors have found that it is advantageous if the compound of Formula (II) comprises two groups having a certain polarity, that is a specific dipole moment within the above range or the ranges mentioned below. It was further found that it is still advantageous that the compound of Formula (II) comprises such polar groups in a geometry of the first polar group and the second polar group in the structure of the compound of Formula (II) in a way that the total dipole moment of the compound of Formula (II) is low, for example, in case that the compound is a symmetrical molecule comprising a first polar group and a second polar group which are the same, the dipole moment could be o Debye. Therefore, the compound of Formula (II) cannot be characterized be referring to the total dipole moment of the compound. As a consequence, reference is made instead to an artificial compound comprising the polar group "G" and an unpolar group "phenyl". In this regard, the dipole moment $|\vec{\mu}|$ of a compound containing N atoms is given by:

$$\vec{\mu} = \sum_{i}^{N} q_i \vec{r_i}$$

$$|\vec{\mu}| = \sqrt{\mu_x^2 + \mu_y^2 + \mu_z^2}$$

where $q_i$ and $\vec{r_i}$ are the partial charge and position of atom i in the molecule. The dipole moment is determined by a semi-empirical molecular orbital method. The geometries of the molecular structures are optimized using the hybrid functional B3LYP with the 6-31G* basis set in the gas phase as implemented in the program package TURBOMOLE V6.5 (TURBOMOLE GmbH, Litzenhardtstrasse 19, 76135 Karlsruhe, Germany). If more than one conformation is viable, the conformation with the lowest total energy is selected to determine the bond lengths of the molecules. In this regard, the entire moiety G encompasses all possible substituents which may be comprised.

G may be selected independently so that the dipole moment of a compound G-phenyl is > 1 D; optionally ≥ 1.2 D; and optionally ≥ 1.5 D,. G may be chosen so that the dipole moment of a compound G-phenyl is ≤ 7 D, optionally ≤ 6 D, optionally ≤ 5 D, optionally ≤ 4 D, optionally ≤ 3.6 D. If more than one conformational isomer of the compound G-phenyl is viable then the average value of the dipole moments of the conformational isomers of G-phenyl is selected to be in this range. Conformational isomerism is a form of stereoisomerism in which the isomers can be interconverted just by rotations about formally single bonds.

By selecting the G such that the dipole moment of a compound G-phenyl lies in the above range it is provided that the electron injection from the adjacent, distinct electron injection layer (EIL) is improved and voltage of the OLED device is decreased and the cd/A efficiency of the OLED device is increased.

Exemplary compounds "G-phenyl" are listed in the following Table 1, wherein the moiety

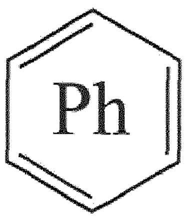

in the respective compound specifies the "phenyl" part in "G-phenyl"

Table 1:

| | Structure of G-phenyl | HOMO [eV] | LUMO [eV] | Dipole moment [D] |
|---|---|---|---|---|
| G-phenyl-1 | 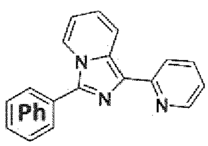 | -5.04 | -1.18 | **3.86** |

(continued)

| | Structure of G-phenyl | HOMO [eV] | LUMO [eV] | Dipole moment [D] |
|---|---|---|---|---|
| G-phenyl-2 | 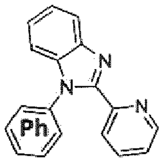 | -5.86 | -1.19 | **5.14** |
| G-phenyl-3 | 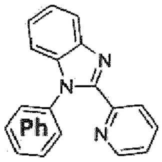 | -5.76 | -1.33 | **2.61** |
| G-phenyl-4 | 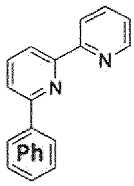 | -5.96 | -1.35 | **2.69** |
| G-phenyl-5 | 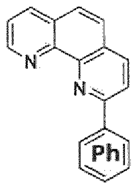 | -5.83 | -1.59 | **2.67** |
| G-phenyl-6 | 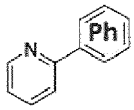 | -6.12 | -1.13 | **1.71** |
| G-phenyl-7 | 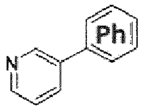 | -6.32 | -0.98 | **2.31** |
| G-phenyl-8 | 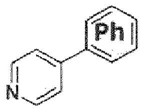 | -6.57 | -1.19 | **2.75** |
| G-phenyl-9 | 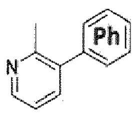 | -6.28 | -0.77 | **2.00** |
| G-phenyl-10 | 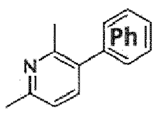 | -6.12 | -0.69 | **1.50** |
| G-phenyl-11 | 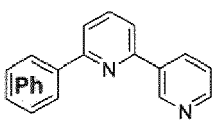 | -6.10 | -1.41 | **3.51** |

(continued)

| | Structure of G-phenyl | HOMO [eV] | LUMO [eV] | Dipole moment [D] |
|---|---|---|---|---|
| G-phenyl-12 | 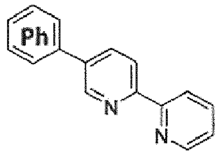 | -6.10 | -1.38 | **2.98** |
| G-phenyl-13 | 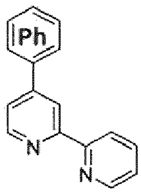 | -6.47 | -1.31 | **3.46** |
| G-phenyl-14 | 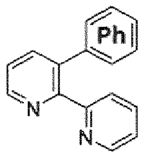 | -6.19 | -1.03 | **3.02** |
| G-phenyl-15 | 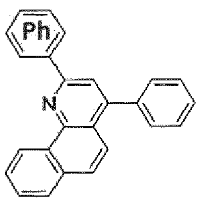 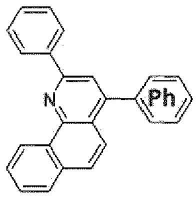 | -5.63 | -1.56 | **1.84** |
| G-phenyl-16 | 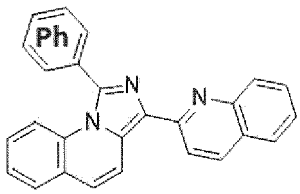 | -5-07 | -1.58 | **2.29** |
| G-phenyl-17 | 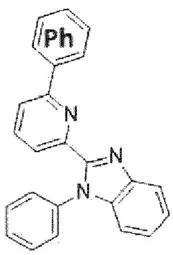 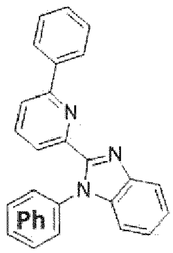 | -5.81 | -1.19 | **4.61** |
| G-phenyl-18 | 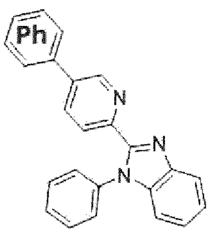 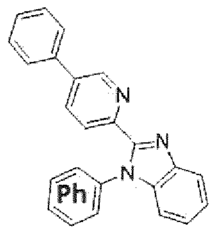 | -5.78 | -1.42 | **5.20** |

(continued)

| | Structure of G-phenyl | HOMO [eV] | LUMO [eV] | Dipole moment [D] |
|---|---|---|---|---|
| G-phenyl-19 | 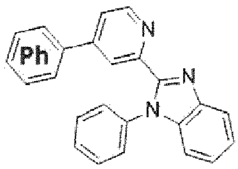 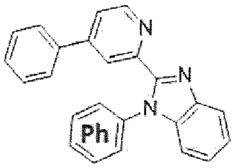 | -5.84 | -1.38 | **5.63** |
| G-phenyl-20 | 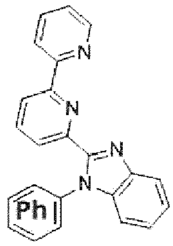 | -5.83 | -1.35 | **3.37** |
| G-phenyl-21 | 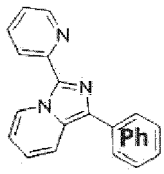 | -4.94 | -1.15 | **1.81** |
| G-phenyl-22 | 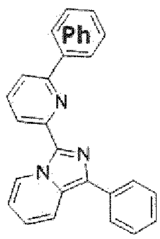 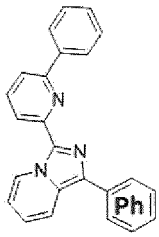 | -4.94 | -1.16 | **2.12** |
| G-phenyl-23 | 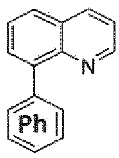 | -5.84 | -1.47 | **1.94** |
| G-phenyl-24 | 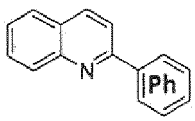 | -5-97 | -1.56 | **1.53** |
| G-phenyl-25 | 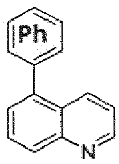 | -6.01 | -1.42 | **2.31** |
| G-phenyl-26 | 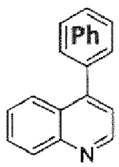 | -6.09 | -1.47 | **2.57** |

(continued)

| | Structure of G-phenyl | HOMO [eV] | LUMO [eV] | Dipole moment [D] |
|---|---|---|---|---|
| G-phenyl-27 | 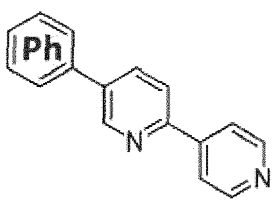 | -6.17 | -1.85 | **4.56** |
| G-phenyl-28 | 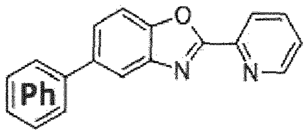 | -5.95 | -1.70 | **1.25** |
| G-phenyl-29 | 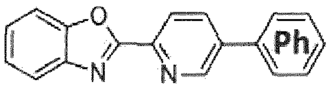 | -5.92 | -1.91 | **1.83** |
| G-phenyl-30 | 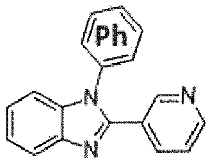 | -5.88 | -1.27 | **2.59** |
| G-phenyl-31 | 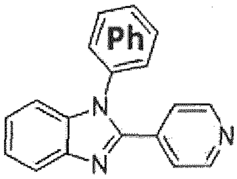 | -6.02 | -1.48 | **4.35** |

The compound of formula (II) may have the following structure (IIa)

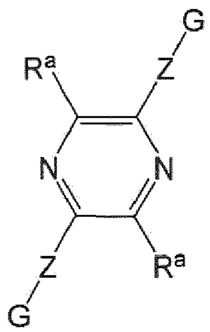

(IIa).

The two $R^a$ may be selected different or the same. The two $R^a$ may be selected the same. The two Z may be selected different or the same. The two Z may be selected the same.

The two G may be selected different or the same. The two G may be selected the same.

$R^a$ is independently selected from the group consisting of substituted or unsubstituted $C_6$ to $C_{24}$ aryl and substituted or unsubstituted $C_3$ to $C_{21}$ heteroaryl, wherein if $R^a$ is substituted, the one or more substituents are independently selected from the group consisting of D, phenyl, and $C_1$ to $C_{12}$ alkyl.

$R^a$ may be independently selected from the group consisting of substituted or unsubstituted $C_6$ to $C_{18}$ aryl and substituted or unsubstituted $C_3$ to $C_{17}$ heteroaryl, wherein if $R^a$ is substituted, the one or more substituents are independently selected from the group consisting of D, and $C_1$ to $C_{12}$ alkyl.

$R^a$ may be independently selected from the group consisting of substituted or unsubstituted $C_6$ to $C_{12}$ aryl and substituted or unsubstituted $C_5$ to $C_{11}$ heteroaryl, wherein if $R^a$ is substituted, the one or more substituents are independently selected from the group consisting of D, phenyl, and $C_1$ to $C_4$ alkyl.

$R^a$ may be independently selected from the group consisting of

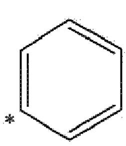, 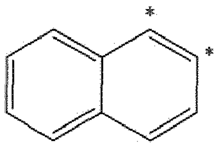, 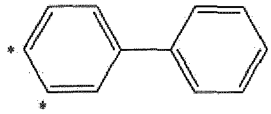, 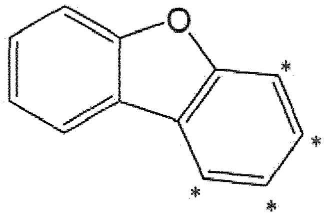,

wherein the respective group is bonded at one of the * to

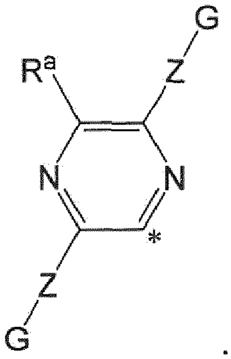.

Z is independently selected from the group consisting of a single bond and $C_6$ to $C_{24}$ arylene. Z may be independently selected from the group consisting of a single bond and $C_6$ to $C_{18}$ arylene. Z may be independently selected from the group consisting of a single bond and $C_6$ to $C_{12}$ arylene. Z may be independently selected from the group consisting of a single bond and $C_6$ to $C_{10}$ arylene.

Z may be independently selected from

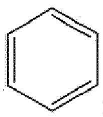; 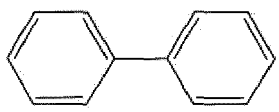; and 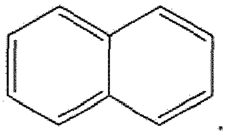.

Z may be independently selected from

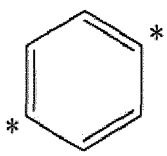; 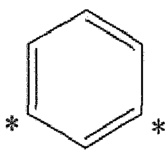;

and;

wherein Z is bonded to

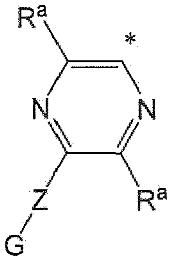

and *G at *, respectively.

G is independently selected from (IIa-1) to (IIa-3)

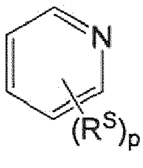 (IIa-1) 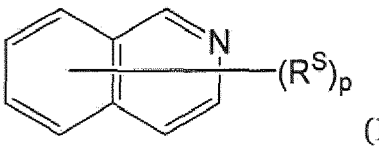 (IIa-2) 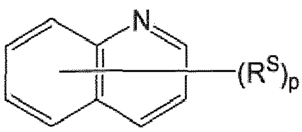 (IIa-3);

In accordance with the present disclosure, in a formula showing the following binding situation,

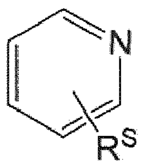

the group $R^S$ may be bonded to any suitable binding position, that is, to any of the carbon atoms not bonded to Z.

In a situation where it is shown that the bond of $R^S$ crosses more than one ring, such as in

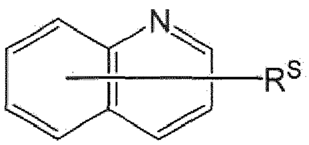 or 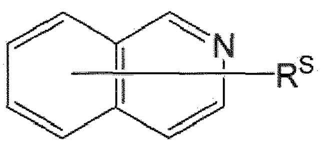

the group $R^S$ may be bonded to any suitable binding position of each ring crossed with the bond, that is, to any of the carbon atoms not bonded to Z.

Z may be bonded in ortho-, meta-, or para-position with respect to the N-atom of the pyridine ring in formula IIa-1 and the remaining positions each may or may not have a substituent $R^s$. Z may be bonded in ortho-, or meta-position with respect to the N-atom of the pyridine ring in formula IIa-2 or to any of the carbon atoms of the ring annelated to the pyridine ring and the remaining positions each may or may not have a substituent $R^s$.

Z may be bonded in meta- or para-position with respect to the N-atom of the pyridine ring in formula IIa-3 or to any of the carbon atoms of the ring annelated to the pyridine ring and the remaining positions each may or may not have a substituent $R^s$.

$R^S$ is independently selected from the group consisting of D, $C_1$ to $C_{12}$ aryl, $C_1$ to $C_{12}$ alkyl, and pyridyl, wherein the respective $C_1$ to $C_{12}$ aryl and pyridyl can be unsubstituted or substituted with one or more of D and $C_1$ to $C_4$ alkyl.

$R^S$ may be independently selected from the group consisting of D, $C_1$ to $C_{10}$ aryl, $C_1$ to $C_4$ alkyl, and pyridyl, wherein the respective $C_1$ to $C_{10}$ aryl and pyridyl can be unsubstituted or substituted with one or more of D and $C_1$ to $C_4$ alkyl.

$R^S$ may be independently selected from the group consisting of D, phenyl, naphthyl, $C_1$ to $C_{12}$ alkyl, and pyridyl.

$R^S$ may be independently selected from the group consisting of D, phenyl, naphthyl, $C_1$ to $C_4$ alkyl, and pyridyl.

$R^s$ may be independently selected from the group consisting of

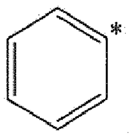 ; 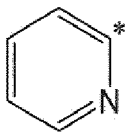 ; 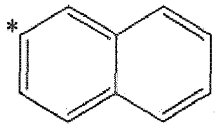 ; 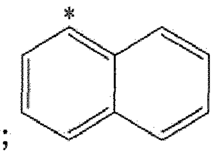 ; *$CH_3$

wherein the respective $R^s$ is bonded at * to Z.

p is an integer from o to 4 for IIa-1. p may by an integer from o to 2 for IIa-1. p may by an integer from o to 1 for IIa-1.

p is an integer from o to 6 for IIa-2 and IIa-3. p may be an integer from o to 4 for IIa-2 and IIa-3. p may be an integer from o to 2 for IIa-2 and IIa-3. p may be an integer from o to 1 for IIa-2 and IIa-3.

The compound of formula (II) may be selected from B-1 to B-44

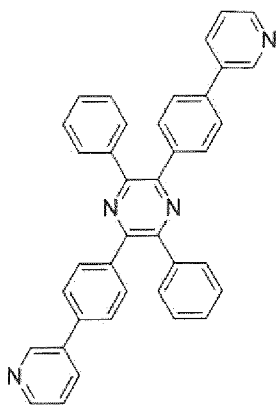

B-1

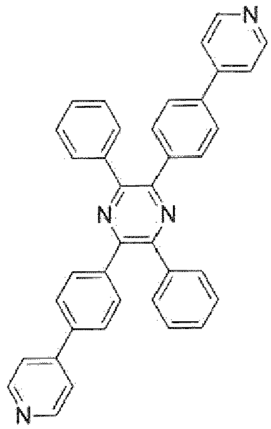

B-2

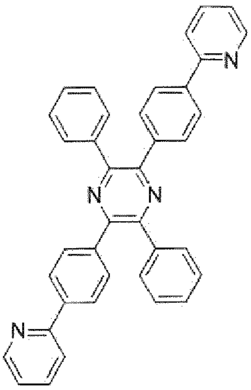

B-3

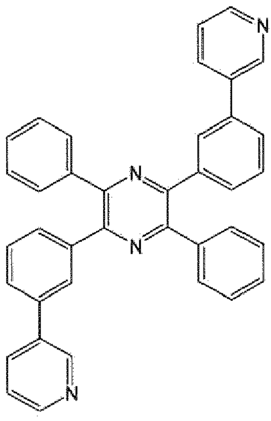

B-4

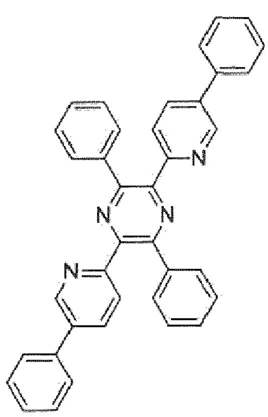

B-5

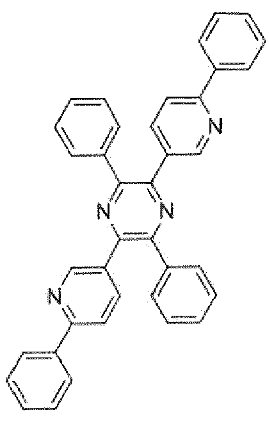

B-6

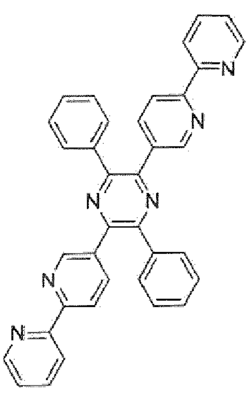

B-7

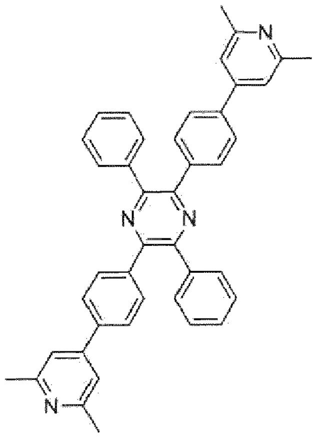

B-8

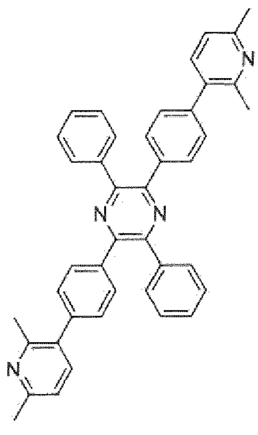

B-9

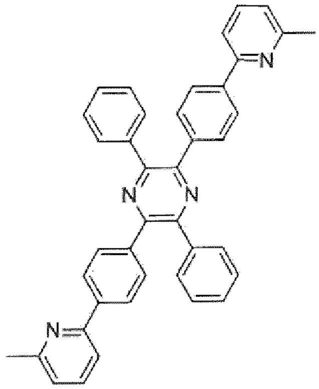

B-10

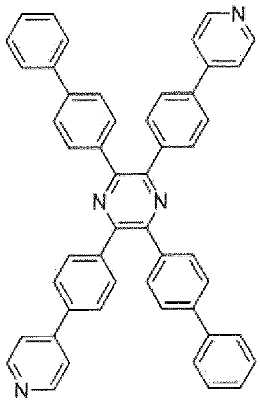

B-11

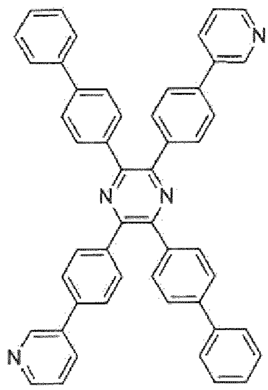

B-12

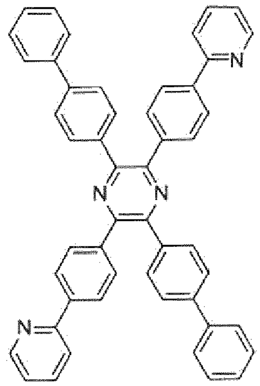

B-13

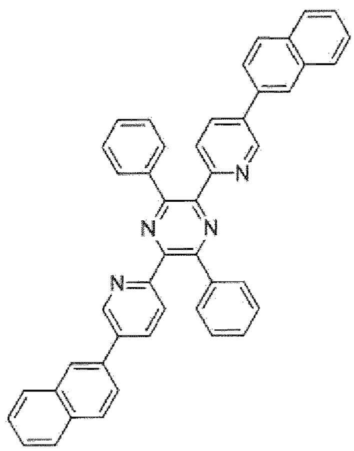

B-14

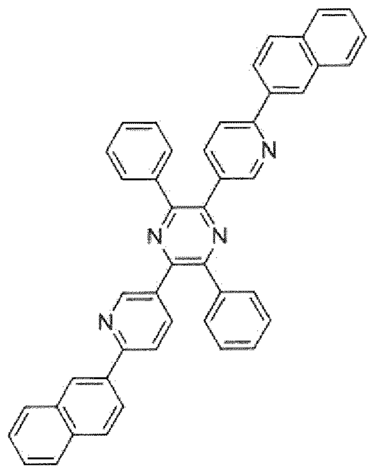

B-15

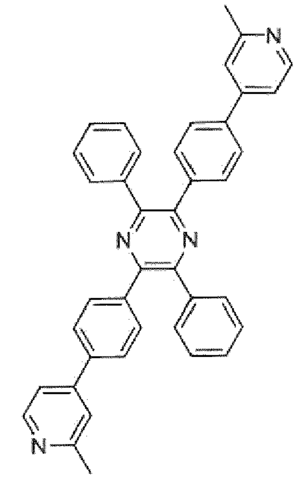

B-16

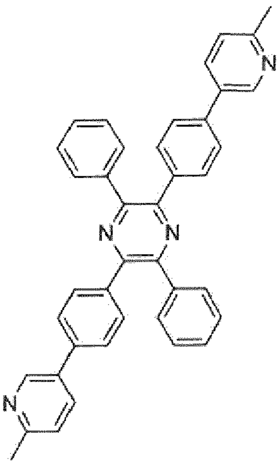

B-17

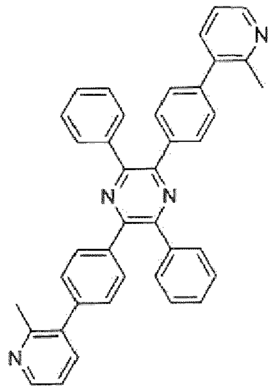

B-18

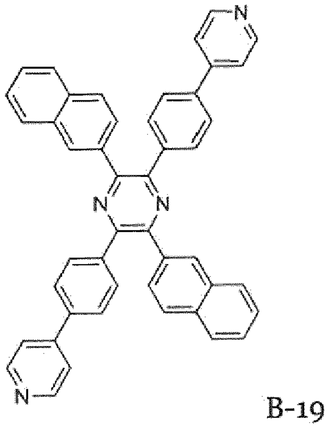

B-19

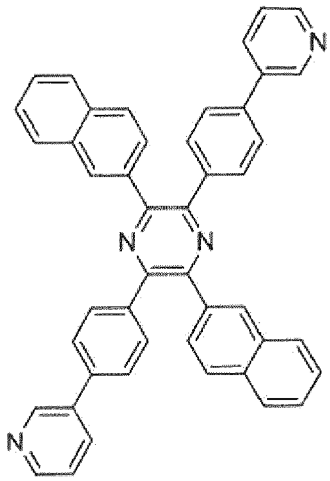

B-20

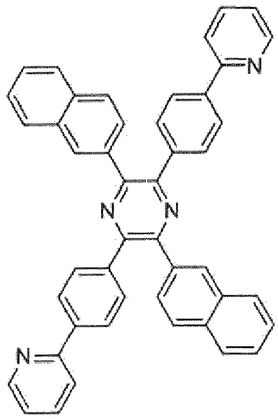

B-21

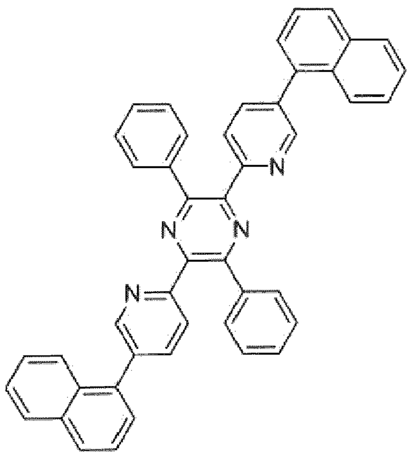

B-22

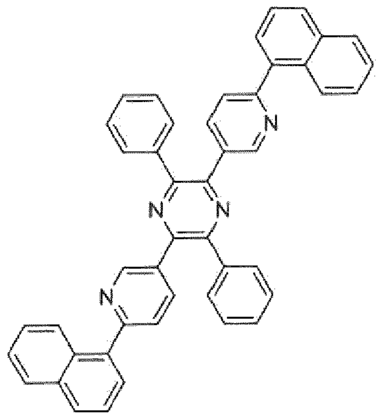

B-23

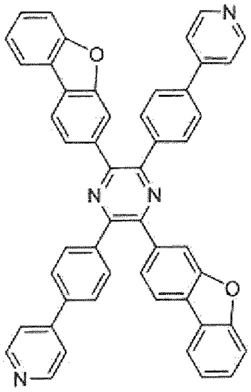

B-24

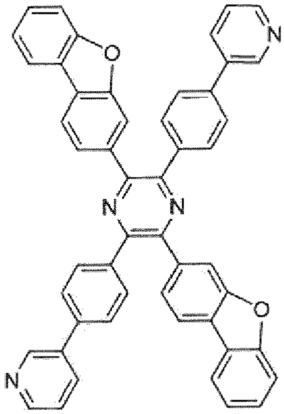

B-25

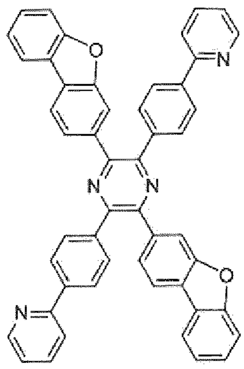

B-26

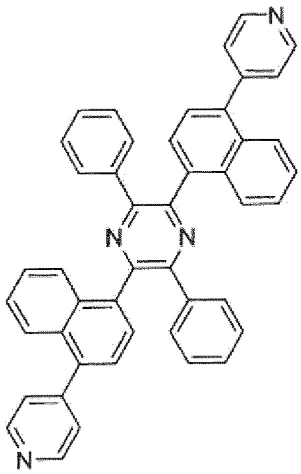

B-27

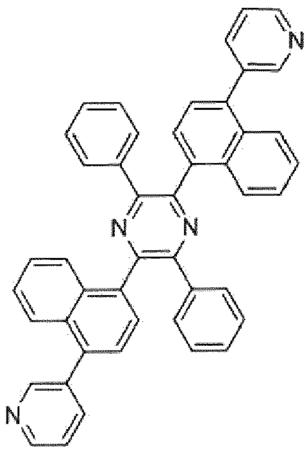

B-28

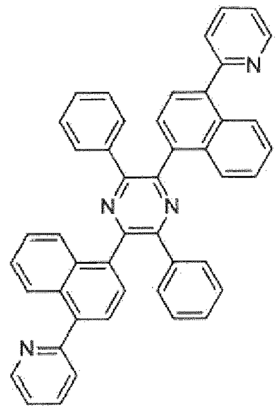

B-29

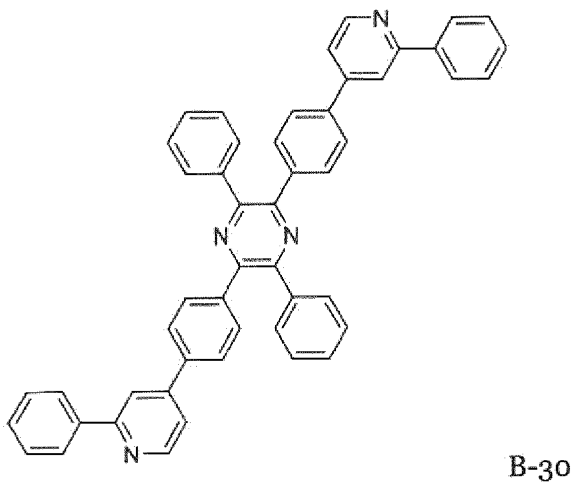

B-30

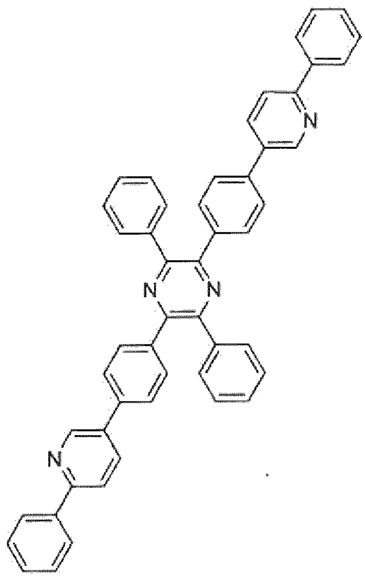

B-31

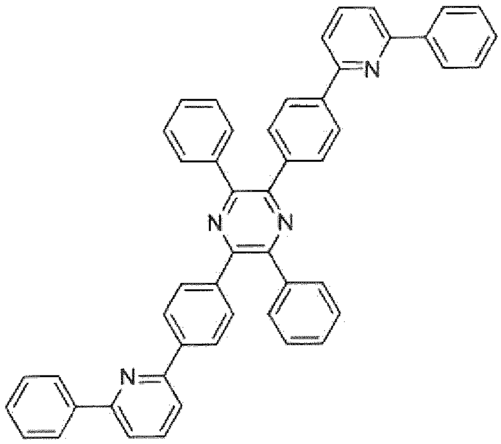

B-32

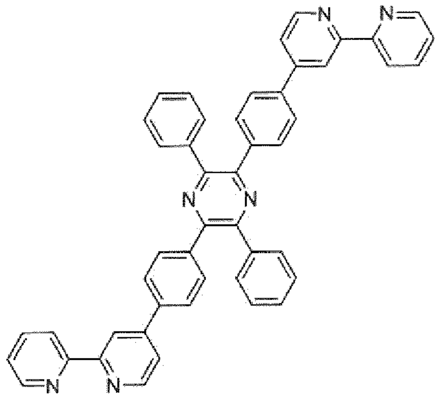

B-33

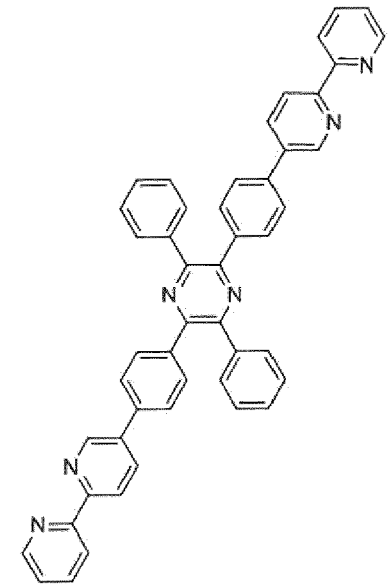

B-34

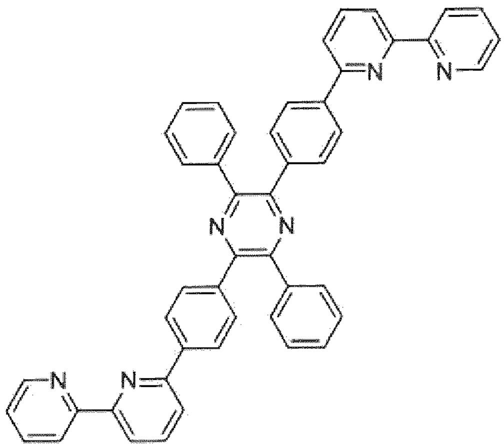

B-35

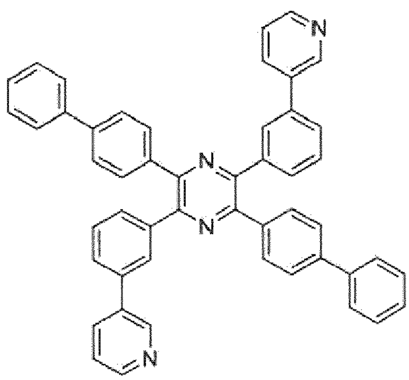

B-36

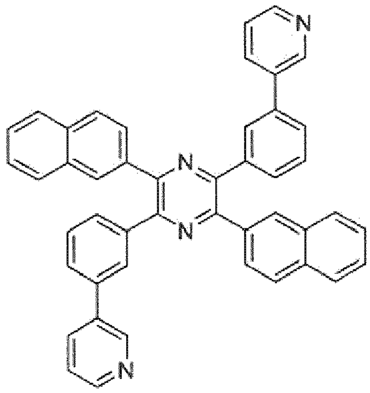

B-37

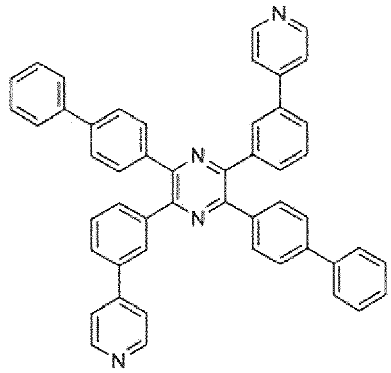

B-38

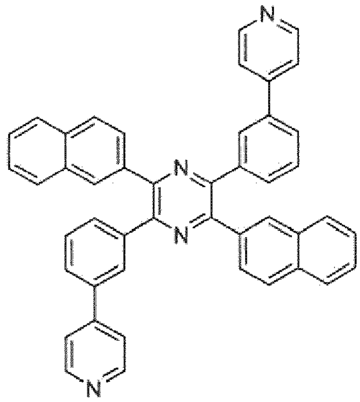

B-39

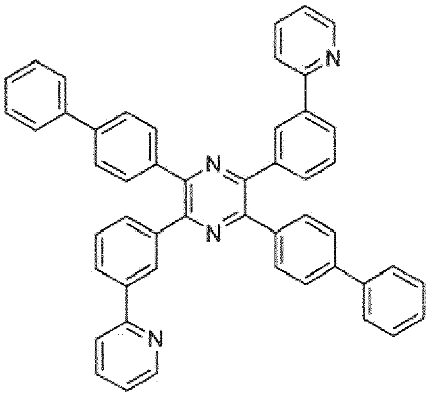

B-40

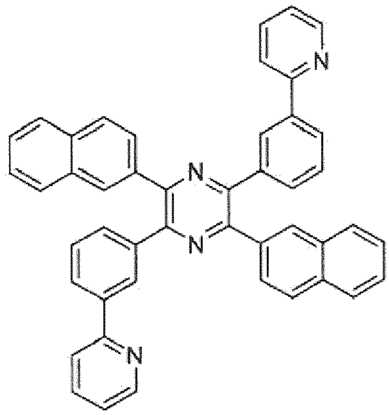

B-41

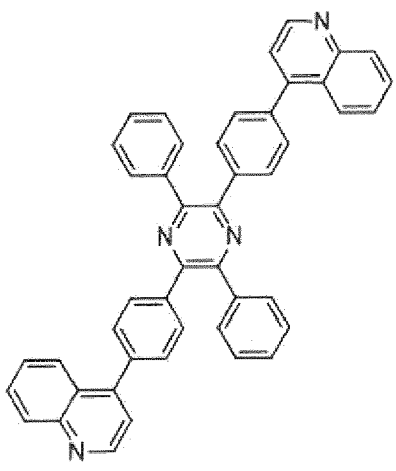

B-42

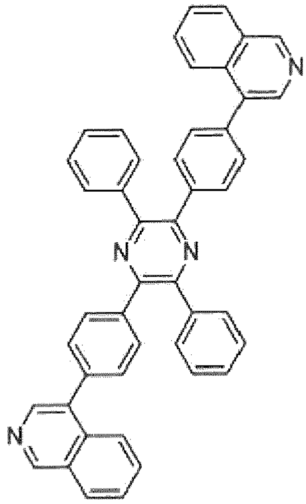

B-43

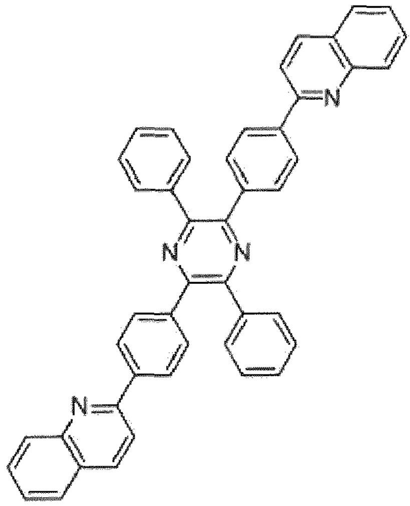

B-44.

The compound of formula (II) may be selected from B-1 and B-4

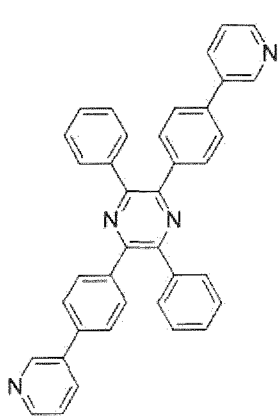

B-1

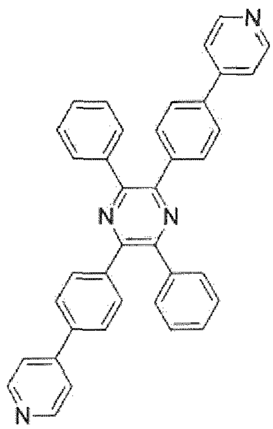

B-2

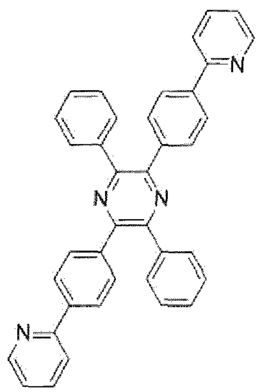

B-3

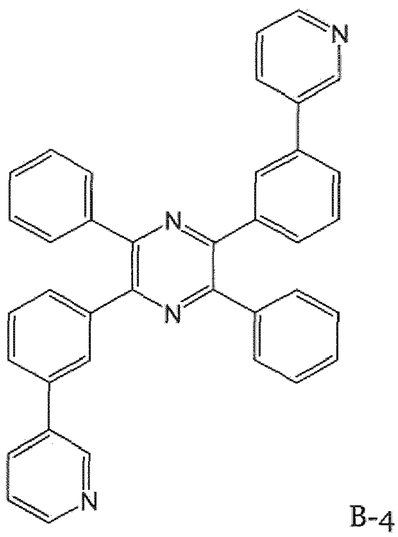

B-4

Compound (III)

The second electron transport layer may further comprise a compound (III), wherein the compound (III) comprises 8 to 13 aromatic or heteroaromatic rings, optionally 8 to 11 aromatic or heteroaromatic rings, optionally 9 to 11 aromatic or heteroaromatic rings, and optionally 9 aromatic or heteroaromatic rings, wherein one or more of the aromatic or heteroaromatic rings may be substituted with $C_1$ to $C_4$ alkyl. In this regard, an aromatic, respectively heteroaromatic ring is a single aromatic ring, for example a 6-membered aromatic ring such as phenyl, a 6-membered heteroaromatic ring such as pyridyl, a 5-membered heteroaromatic ring such as pyrrolyl etc. In a system of condensed (hetero)aromatic rings, each ring is considered as a single ring in this regard. For example, naphthalene comprises two aromatic rings.

The compound (III) may comprise at least one heteroaromatic ring, optionally 1 to 5 heteroaromatic rings, optionally 1 to 4 heteroaromatic rings, optionally 1 to 3 heteroaromatic rings, and optionally 1 or 2 heteroaromatic rings.

The aromatic or heteroaromatic rings of the compound (III) may be 6-membered rings.

The heteroaromatic rings of the compound (III) may be a N-containing heteroaromatic ring, optionally all of the heteroaromatic rings are N-containing heteroaromatic rings, optionally all of the heteroaromatic rings heteroaromatic rings contain N as the only type of heteroatom.

The compound (III) may comprise at least one 6-membered heteroaromatic ring containing one to three N-atoms in each heteroaromatic ring, optionally one to three 6-membered heteroaromatic rings containing one to three N-atoms in each heteroaromatic ring, respectively.

The at least one 6-membered heteroaromatic ring comprised in the compound (III) may be an azine. The at least one 6-membered heteroaromatic ring comprised in the compound (III) may be triazine, diazine, pyrazine.

If the compound (III) comprises two or more heteroaromatic rings, the heteroaromatic rings may be separated from each other by at least one aromatic ring which is free of a heteroatom.

In an embodiment, the heteroatoms in the heteroaromatic rings of compound (III) are bound into the molecular structure of compound (III) by at least one double bond.

The molecular dipole moment, computed by the TURBOMOLE V6.5 program package using hybrid functional B3LYP and Gaussian 6-31G* basis set, of the compound (III) may be ≥ o D and ≤ 4 D; alternatively ≥ 0.1 D and ≤ 3.9 D; alternatively ≥ 0.2 D and ≤ 3.7 D; alternatively ≥ 0.3 D and ≤ 3.5 D.

By choosing compound (III) according to these embodiments it is provided that the electron mobility of the second electron transport layer is further improved and voltage of the OLED device is decreased and the cd/A efficiency of the OLED device is increased.

In an embodiment the compound (III) is not a compound of formula (II). The compound of Formula (III) may be selected from the compounds C-1 to C-6 of the following Table 2.

Table 2:

| Name | Structure | HOMO [eV] | LUMO [eV] | Dipole moment [D] |
|---|---|---|---|---|
| C-1 | 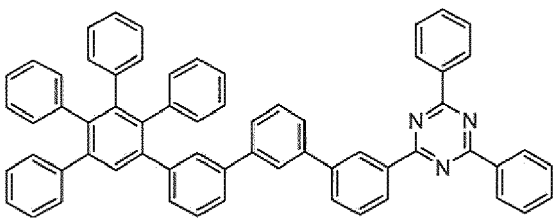 | -5.72 | -1.82 | 0.30 |
| C-2 | 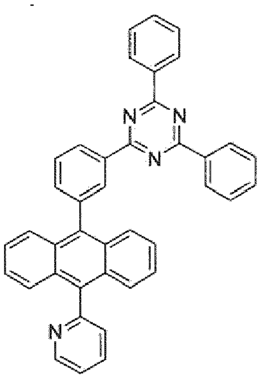 | -5.11 | -1.83 | 1.98 |

(continued)

| Name | Structure | HOMO [eV] | LUMO [eV] | Dipole moment [D] |
|---|---|---|---|---|
| C-3 | 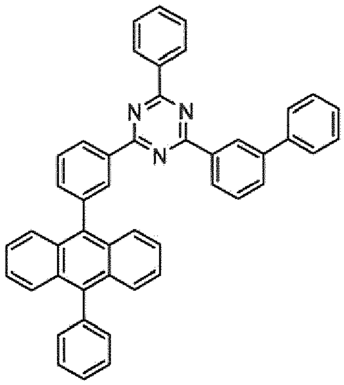 | -5.19 | -1.84 | 0.37 |
| C-4 | 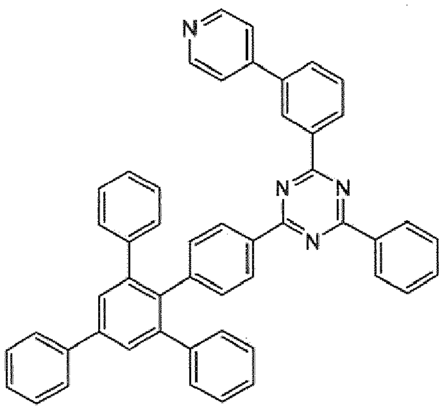 | -5.87 | -1.94 | 3.24 |
| C-5 | 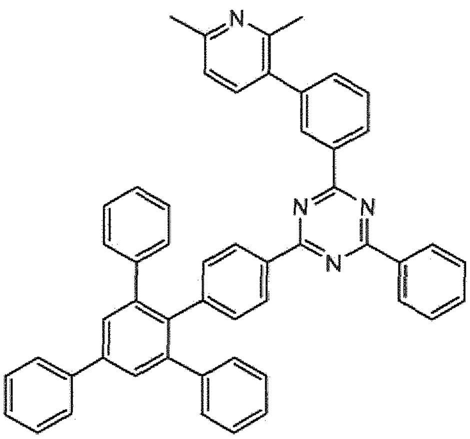 | -5.83 | -1.86 | 1.61 |
| C-6 | 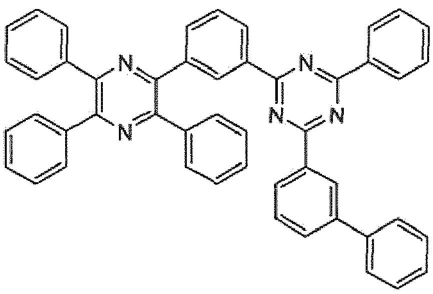 | -5.67 | -1.85 | 0.40 |

In case that the second electron transport layer comprises both the compound of Formula (II) and compound (III), the weight ratio of Formula (II) to compound (III) may be 1:99 to 99:1, alternatively 10:90 to 60:40, alternatively

20:80 to 50:50, alternatively 25:75 to 40:60, alternatively about 30:70.

In an embodiment the LUMO energy level of the compound of formula (III) in the absolute scale taking vacuum energy level as zero, computed by the TURBOMOLE V6.5 program package using hybrid functional B3LYP and Gaussian 6-31G* basis set, is in the range from - 2.00 eV to -1.70 eV, preferably from -1.95 eV to -1.80 eV.

In an embodiment the compound (III) comprises one nitrogen-containing six-membered ring.

In another embodiment the compound (III) comprises two nitrogen-containing six-membered rings.

In an embodiment the compound of formula (I) is not a compound of formula (II). In a further embodiment the compound of formula (II) is not a compound (III). In another embodiment the compound of formula (I) is not a compound (III). In a further embodiment of the invention all three compounds, namely the compound of formula (I), the compound of formula (II) and compound (III), are different from each other in that they have different molecular structure formulas.

First electron transport layer

The organic electron transport layer stack comprises optionally a first electron transport layer, that is, may comprise the first electron transport layer or may not comprise the first electron transport layer. In case that the organic electron transport layer stack does not comprise the first electron transport layer, the layer stack may consist only of the second electron transport layer. The first electron transport layer may be referred to as a hole blocking layer, auxiliary electron transport layer or $\alpha$-ETL.

The first electron transport layer may be arranged between the second electron transport layer and the emission layer. The first electron transport layer may be arranged between and in direct contact with both the second electron transport layer and the emission layer.

The first electron transport layer may comprise a compound of Formula (I)

$$(Ar^1\text{-}A_c)_a\text{-}X_b \qquad \text{(I)}$$

- a is 1 or 2;
- b is 1 or 2;
- c is o or 1;
- $Ar^1$ is independently selected from substituted or unsubstituted $C_6$ to $C_6$. aryl or substituted or unsubstituted $C_2$ to $C_{42}$ heteroaryl;
  - wherein, if $Ar^1$ is substituted, the one or more substituent(s) are independently selected from the group consisting of D, $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;
  - wherein each $C_6$ to $C_{12}$ aryl substituent on $Ar^1$ and each $C_3$ to $C_{11}$ heteroaryl substituent on $Ar^1$ may be substituted with D, $C_1$ to $C_4$ alkyl or halogen;
- A is independently selected from substituted or unsubstituted $C_6$ to $C_{30}$ aryl;
  - wherein, if A is substituted, the one or more substituent(s) are independently selected from the group consisting of D, $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

- wherein each $C_6$ to $C_{12}$ aryl substituent on A and each $C_3$ to $C_{11}$ heteroaryl substituent on X may be substituted with D, $C_1$ to $C_4$ alkyl or halogen;

- X is independently selected from the group consisting of substituted or unsubstituted $C_2$ to $C_{42}$ heteroaryl and substituted or unsubstituted $C_6$ to $C_{60}$ aryl

  - wherein, if X is substituted, the one or more substituent(s) are independently selected from the group consisting of D, $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

  - wherein each $C_6$ to $C_{12}$ aryl substituent on X and each $C_3$ to $C_{11}$ heteroaryl substituent on X may be substituted with D, $C_1$ to $C_4$ alkyl or halogen; and

- the compound of formula (I) has a dipole moment of ≥ o D and ≤ 4 D.

The first electron transport layer may consist of the compound of Formula (I). Alternatively, the first electron transport layer may consist of a mixture of the compound of Formula (I) and one or more further compounds. The first electron transport layer may comprise more than one compound of Formula (I). In particular, the first electron transport layer may consist of a mixture of the compound of Formula (I) and further compounds known in the art as electron transport matrix compounds. Exemplary further electron transport matrix compounds which may be contained are disclosed below. Preferably, the first electron transport layer is free of an electrical dopant, such as an n-type dopant, especially a redox n-type dopant.

The term "free of" in this regard does not exclude impurities. Impurities have no technical effect with respect to the object achieved by the present invention. Impurities are not deliberately added to the layer during processing.

The term "free of' a compound means that such compound is not deliberately added to the layer during processing.

In the compound of Formula (I), the group "A" is spacer moiety connecting (if present, that is in case that c > 1) the group $Ar^1$ and X. In case that the compound of Formula (I) comprises more than one groups ($Ar^1$-$A_c$), the groups may or may not independently comprise the spacer A.

In the compound of Formula (I), a and b are independently 1 or 2. Alternatively, a and b may both be 1.

In the compound of Formula (I), c is independently o or 1.

$Ar^1$ is independently selected from $C_6$ to $C_{60}$ aryl or $C_2$ to $C_{42}$ heteroaryl, alternatively $C_6$ to $C_{54}$ aryl or $C_2$ to $C_{39}$ heteroaryl, alternatively $C_6$ to $C_{48}$ aryl or $C_2$ to $C_{36}$ heteroaryl, alternatively $C_6$ to $C_{42}$ aryl or $C_2$ to $C_{36}$ heteroaryl, alternatively $C_6$ to $C_{36}$ aryl or $C_2$ to $C_{30}$ heteroaryl, alternatively $C_6$ to $C_{30}$ aryl or $C_2$ to $C_{24}$ heteroaryl.

$Ar^1$ may be independently $C_6$ to $C_{54}$ aryl, optionally $C_6$ to $C_{48}$ aryl, optionally $C_6$ to $C_{42}$ aryl, optionally $C_6$ to $C_{36}$ aryl, optionally $C_6$ to $C_{30}$ aryl, and optionally $C_6$ to $C_{24}$ aryl.

$Ar^1$ may be independently $C_2$ to $C_{42}$ hetroaryl, optionally $C_2$ to $C_{40}$ hetroaryl, optionally $C_2$ to $C_{36}$ hetroaryl, optionally $C_2$ to $C_{30}$ hetroaryl, and optionally $C_2$ to $C_{24}$ hetroaryl.

In an embodiment $Ar^1$ is different from X.

$Ar^1$ may comprise a system of two or more anellated aromatic rings, preferably three or more anellated aromatic rings.

$Ar^1$ may comprise at least one spa-hypridized carbon atom.

$Ar^1$ may comprise at least one carbon-carbon $sp^2$ alkene bond which is not integrated into an aromatic ring structure.In an embodiment where $Ar^1$ is independently selected from unsubstituted $C_2$ to $C_{42}$ heteroaryl, the heteroatoms are bound into the molecular structure of $Ar^1$ by single bonds.

$Ar^1$ may be independently selected from the group consisting of phenyl, naphthyl, anthracenyl, fluoranthenyl, xanthenyl, spiro-xanthenyl, fluorenyl, spiro-fluorenyl, triphenylsilyl, tetraphenylsilyl, dibenzo-furanyl, di-dibenzofuranyl, pyrimidinyl, pyrazinyl, aryl-alkenyl or a group having the formula (Ia)

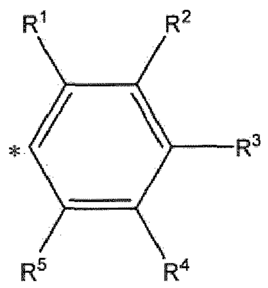

(Ia)

wherein

- the asterisk symbol "*" represents the binding position for binding the group of formula (Ia) to A; and
- $R^1$ to $R^5$ are independently selected from the group consisting of H, $C_6$ to $C_{12}$ aryl and $C_3$ to $C_{10}$, alternatively $C_4$ to $C_5$ heterorayl.

$Ar^1$ may be independently selected from the group consisting of phenyl, anthracenyl, fluorenyl or the group of the formula (Ia)

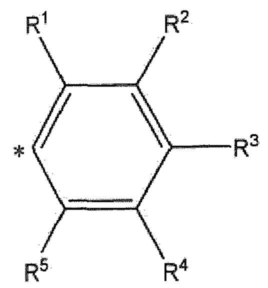

(Ia)

wherein $R^1$ to $R^5$ are independently selected from H and phenyl.

$Ar^1$ may be a group of Formula (Ia)

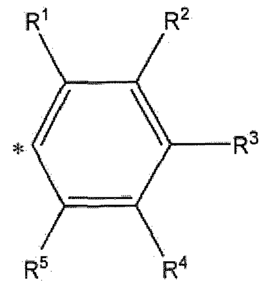

(Ia)

and at least two of $R^1$ to $R^5$ are not H.

In the group of Formula (Ia), at least two of $R^1$ to $R^5$ which are not H may be in ortho-position to each other. At least one of $R^1$ to $R^5$ which is not H may be in ortho-position to the *-position. In this regard, two groups are in ortho position to each other if bound to adjacent carbon atoms of the benzene ring in Formula (Ia), respectively.

$Ar^1$ may be selected independently from one of the following groups

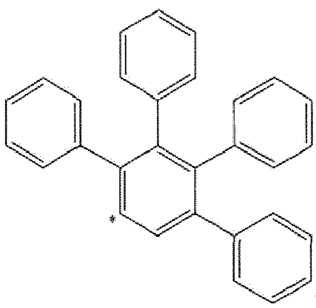; 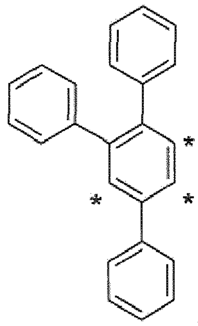; 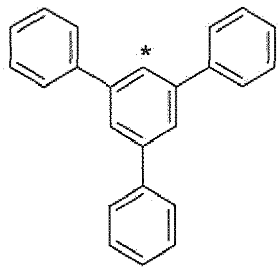; 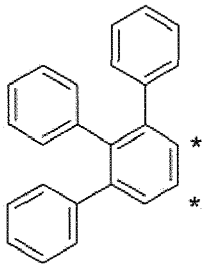;

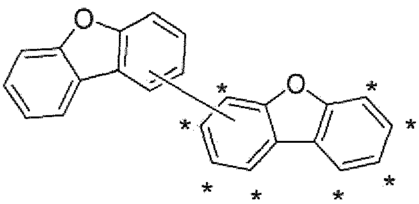; 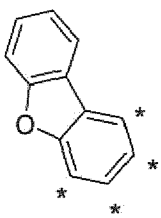; 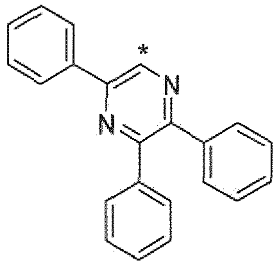; 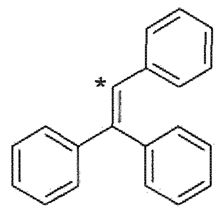;

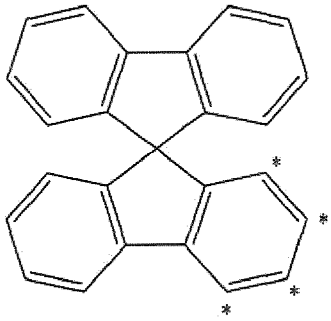; 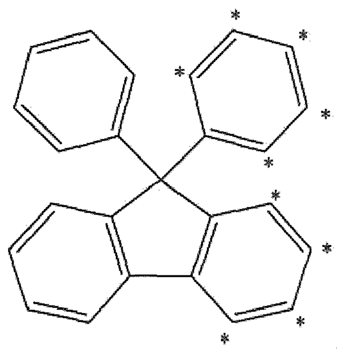; 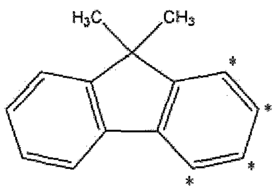

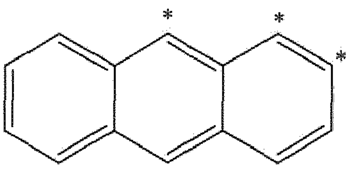; 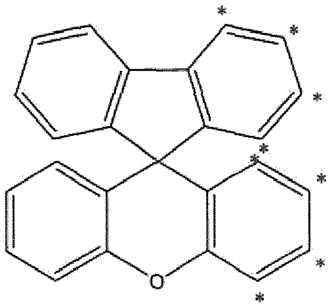; 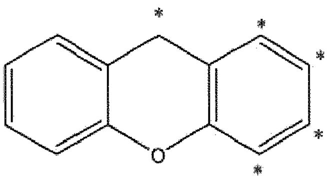;

wherein the asterisk symbol "*" represents the binding position for binding the to A, respectively.

In case that $Ar^1$ is substituted, each of the substituents may be independently selected from the group consisting of phenyl, naphtyl, biphenyl, pyridyl, picolinyl, lutidinyl, dibenzofuranyl, dibenzothiophene-yl, and benzothiophene-yl.

A may be independently selected from substituted or unsubstituted $C_6$ to $C_{30}$ aryl, alternatively $C_6$ to $C_{24}$ aryl, alternatively $C_6$ to $C_{18}$ aryl.

A may be selected independently from the group consisting of phenylene, naphthylene, biphenylene and terphenylene which may be substituted or unsubstituted, respectively.

A may be selected independently from one of the following groups or combinations thereof

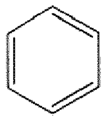;
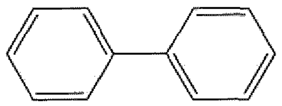;
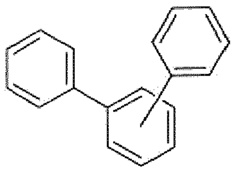; ;
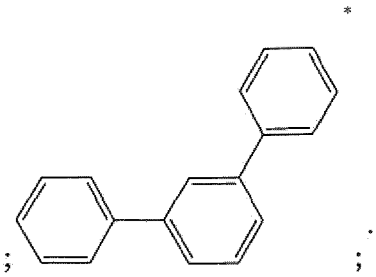;

wherein the binding positions for binding to $Ar^1$ and X can be freely selected, preferably

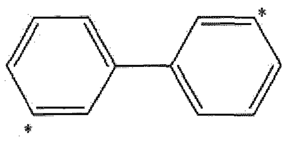;
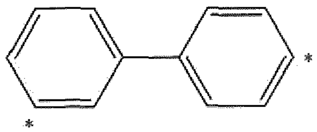
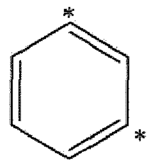;
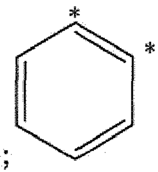;
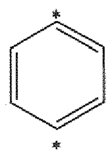;

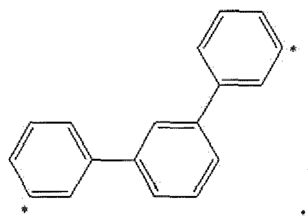;
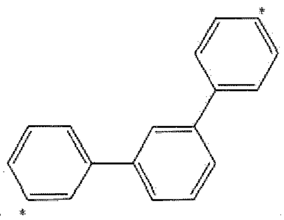;

wherein the asterisk symbol "*" indicate the binding positions.

In case that A is substituted, each substituent on A may be independently selected from the group consisting of phenyl and $C_1$ to $C_4$ alkyl.

X may be independently selected from the group consisting of $C_2$ to $C_{39}$ heteroaryl and $C_6$ to $C_{54}$ aryl, optionally $C_2$ to $C_{36}$ heteroaryl and $C_6$ to $C_{48}$ aryl, optionally $C_3$ to $C_{30}$ heteroaryl and $C_6$ to $C_{42}$ aryl, optionally $C_3$ to $C_{27}$ heteroaryl and $C_6$ to $C_{36}$ aryl, optionally $C_3$ to $C_{24}$ heteroaryl and $C_6$ to $C_{30}$ aryl, and optionally $C_3$ to $C_{21}$ heteroaryl and $C_6$ to $C_{24}$ aryl, wherein the respective group may be substituted or unsubstituted.

X may be independently selected from the group consisting of $C_2$ to $C_{39}$ N-containing heteroaryl, $C_2$ to $C_{39}$ O-containing heteroaryl and $C_6$ to $C_{54}$ aryl, optionally $C_2$ to $C_{36}$ N-containing heteroaryl, $C_2$ to $C_{36}$ O-containing heteroaryl and $C_6$ to $C_{48}$ aryl, optionally $C_3$ to $C_{30}$ N-containing heteroaryl, $C_3$ to $C_{30}$ O-containing heteroaryl and $C_6$ to $C_{42}$ aryl, optionally $C_3$ to $C_{27}$ N-containing heteroaryl, $C_3$ to $C_{27}$ O-containing heteroaryl and $C_6$ to $C_{36}$ aryl, optionally $C_3$ to $C_{24}$ N-containing heteroaryl, $C_3$ to $C_{24}$ O-containing heteroaryl and $C_6$ to $C_{30}$ aryl, and optionally $C_3$ to $C_{21}$ N-containing heteroaryl, $C_3$ to $C_{21}$ O-containing heteroaryl and $C_6$ to $C_{24}$ aryl, wherein the respective group may be substituted or unsubstituted.

X may be independently selected from the group consisting of $C_2$ to $C_{39}$ N-containing heteroaryl and $C_6$ to $C_{54}$ aryl, optionally $C_2$ to $C_{36}$ N-containing heteroaryl, and $C_6$ to $C_{48}$ aryl, optionally $C_3$ to $C_{30}$ N-containing heteroaryl and $C_6$ to $C_{42}$ aryl, optionally $C_3$ to $C_{27}$ N-containing heteroaryl and $C_6$ to $C_{36}$ aryl, optionally $C_3$ to $C_{24}$ N-containing heteroaryl and $C_6$ to $C_{30}$ aryl, and optionally $C_3$ to $C_{21}$ N-containing heteroaryl and $C_6$ to $C_{24}$ aryl, wherein the respective group may be substituted or unsubstituted. In this regard, it may be provided that a respective N-containing heteroaryl comprises one or more N-atoms as the only heteroatom(s).

X may be independently selected from the group consisting of triazinyl, pyrimidinyl, pyrazinyl quinazolinyl, benzoquinazolinyl, benzimidazolyl, quinolinyl, benzoquinolinyl benzoacridinyl, dibenzoacridinyl, fluoranthenyl, anthracenyl, naphthyl, triphenylenyl, phenathrolinyl, and dinaphthofuranyl which may be substituted or unsubstituted, respectively.

X may be independently selected from the group consisting of triazinyl, pyrimidinyl, pyrazinyl quinazolinyl, benzoquinazolinyl, benzoacridinyl, dibenzoacridinyl, fluoranthenyl, anthracenyl, naphthyl, triphenylenyl, phenathrolinyl, and dinaphthofuranyl which may be substituted or unsubstituted, respectively.

X may be independently selected from the group consisting of triazinyl, pyrimidinyl, pyrazinyl quinazolinyl, benzoquinazolinyl, benzoacridinyl, dibenzoacridinyl, fluoranthenyl which may be substituted or unsubstituted, respectively.

X may be selected independently from one of the following groups

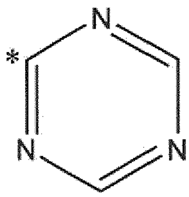 ; 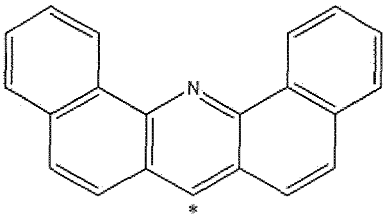 ; 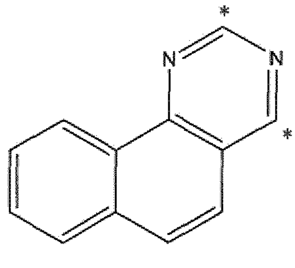 ; 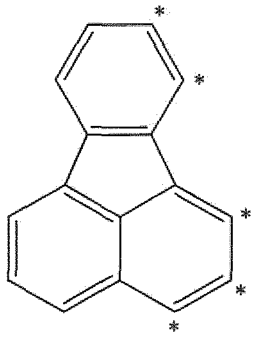

wherein the asterisk symbol "*" represents the binding position for binding the group to A, respectively, wherein the respective group may be substituted or unsubstituted.

In case that X is substituted, each substituent on X may be independently selected from the group consisting of phenyl, naphthyl and biphenyl-yl. In case that X is substituted, each substituent on X may be independently selected from the group consisting of phenyl and biphenyl-yl.

In case that X is substituted, respective substituted X groups (including all substituents) may be

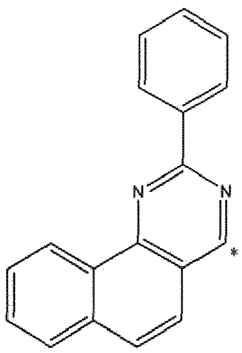 ; 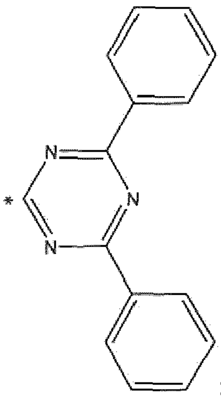 ; 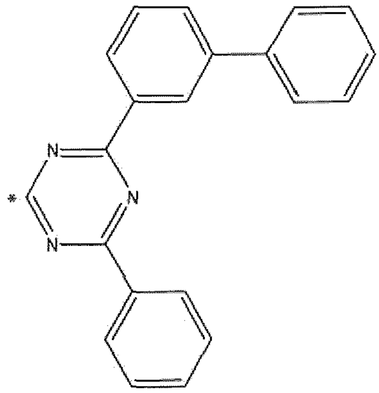 , 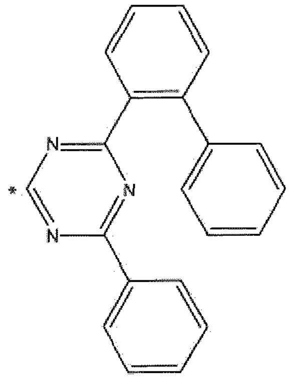 .

wherein the asterisk symbol "*" represents the binding position for binding the group to A, respectively.

It may be provided that the compound of Formula (I) does not contain a moiety P=O. It may be provided that the compound of Formula (I) does not contain $P(=O)Aryl_2$. It may be provided that the compound of Formula (I) does not contain $P(=O)Alkyl_2$. It may be provided that the compound of Formula (I) does not contain $P(=O)Ph_2$. It may be provided that the compound of Formula (I) does not contain $P(=O)(CH_3)_2$. It may be provided that the compound of Formula (I) does not contain R'P(=O)R" wherein R' and R" are connected with each other to form a ring, that is, does not contain ring-phosphine oxide. It may be provided that the compound of Formula (I) does not contain R'P(=O)R" wherein R' and R" are connected with each other to form a 7-membered ring.

It may be provided that the compound of Formula (I) does not contain two moieties P=O. It may be provided that wherein the compound of Formula (I) does not contain two $P(=O)Aryl_2$. It may be provided that wherein the compound of Formula (I) does not contain two $P(=O)Alkyl_2$. It may be provided that wherein the compound of Formula (I) does not contain two $P(=O)Ph_2$. It may be provided that wherein the compound of Formula (I) does not contain two $P(=O)(CH_3)_2$. It may be provided that wherein the compound of Formula (I) does not contain CN.

It may be provided that one or more of the following formulas are excluded from the scope of the Compound of Formula (I)

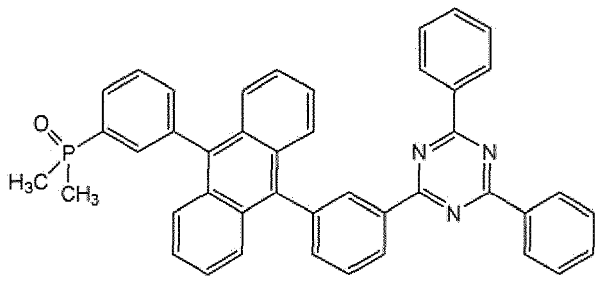 ; 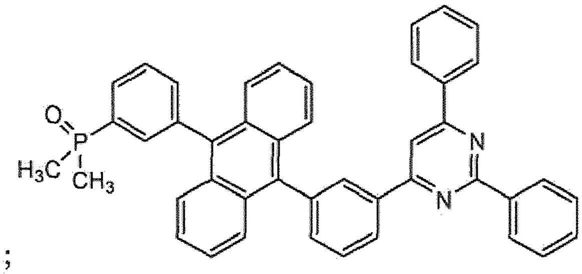 ;

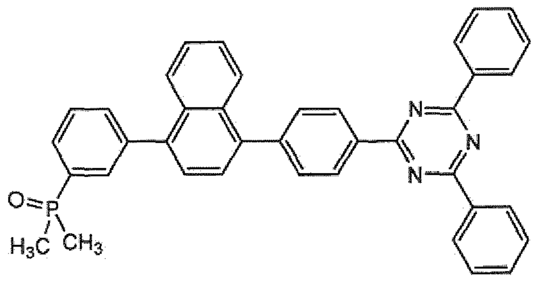 ; 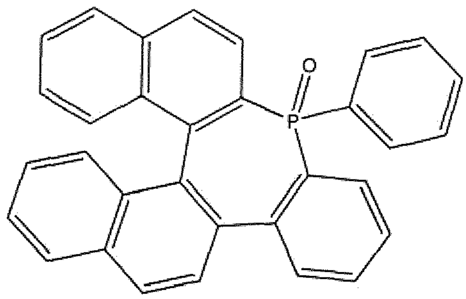 ;

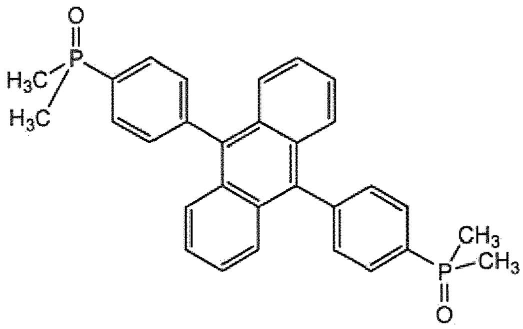 ; 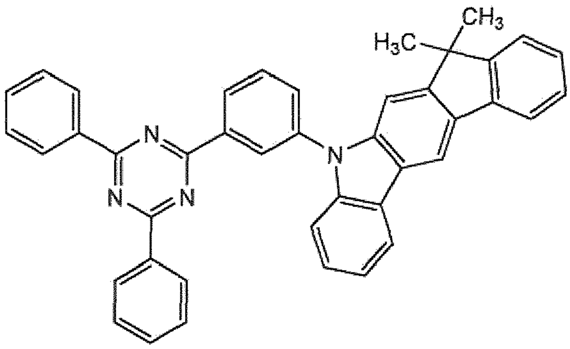 ;

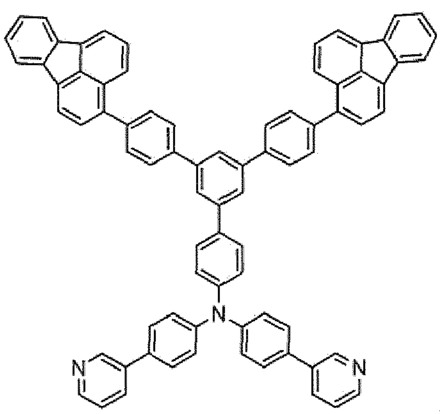 .

The compound of Formula (I) may comprise 6 to 14 aromatic or heteroaromatic rings, optionally 7 to 13 aromatic or heteroaromatic rings, optionally 7 to 12 aromatic or heteroaromatic rings, optionally 9 to 11 aromatic or heteroaromatic rings. In this regard, an aromatic, respectively heteroaromatic ring, is a single aromatic ring, for example a 6-membered aromatic ring such as phenyl, a 6-membered heteroaromatic ring, an example would be pyridyl, a 5-membered heteroaromatic ring an example would be pyrrolyl etc. In a system of condensed (hetero)aromatic rings, each ring is considered as a single ring in this regard. For example, naphthalene comprises two aromatic rings.

The molecular dipole moment, computed by the TURBOMOLE V6.5 program package using hybrid functional B3LYP and Gaussian 6-31G* basis set, of the compound of formula (I) may be ≥ o D and ≤ 4 D; alternatively ≥ o D and

≤ 3.5 D; alternatively ≥ o D and ≤ 3.0 D; alternatively ≥ o D and ≤ 2.5 D; alternatively ≥ o D and ≤ 2.0 D. In this regards, the dipole moment $|\vec{\mu}|$ of a molecule containing N atoms is given by:

$$\vec{\mu} = \sum_{i}^{N} q_i \vec{r_i}$$

$$|\vec{\mu}| = \sqrt{\mu_x^2 + \mu_y^2 + \mu_z^2}$$

where $q_i$ and $\vec{r_i}$ are the partial charge and position of atom i in the molecule. The dipole moment is determined by a semi-empirical molecular orbital method. The geometries of the molecular structures are optimized using the hybrid functional B3LYP with the 6-31G* basis set in the gas phase as implemented in the program package TURBOMOLE V6.5 (TURBOMOLE GmbH, Litzenhardtstrasse 19, 76135 Karlsruhe, Germany). If more than one conformation is viable, the conformation with the lowest total energy is selected to determine the bond lengths of the molecules.

In an embodiment the LUMO energy level of the compound of formula (I) in the absolute scale taking vacuum energy level as zero, computed by the TURBOMOLE V6.5 program package using hybrid functional B3LYP and Gaussian 6-31G* basis set, is in the range from -1.90 eV to -1.60 eV, preferably from -1.87 eV to -1.65 eV, preferably from -1.85 eV to -1.65 eV.

The compound of Formula (I) maybe selected from the compounds A-1 to A-29 of the following Table 3.

Table 3:

| Name | Structure | HOMO [eV] | LUMO [eV] | Dipole moment [D] |
|---|---|---|---|---|
| A-1 | 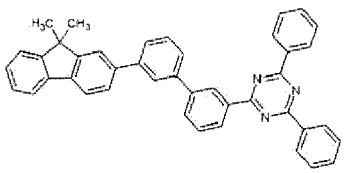 | -5.57 | -1.84 | 0.48 |
| A-2 | 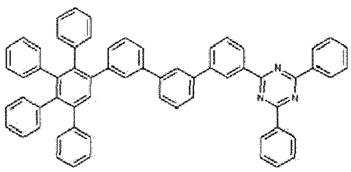 | -5.72 | -1.82 | 0.30 |
| A-3 | 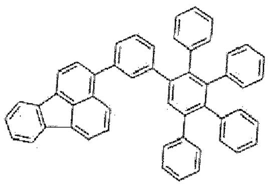 | -5.57 | -1.78 | 1.02 |
| A-4 | 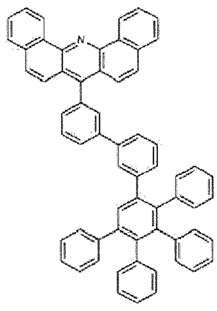 | -5.60 | -1.70 | 2.74 |

(continued)

| Name | Structure | HOMO [eV] | LUMO [eV] | Dipole moment [D] |
|---|---|---|---|---|
| A-5 | 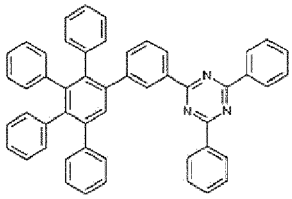 | -5.82 | -1.76 | 0.57 |
| A-6 | 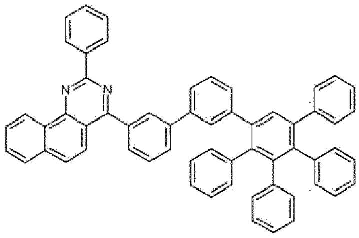 | -5.77 | -1.75 | 1.59 |
| A-7 | 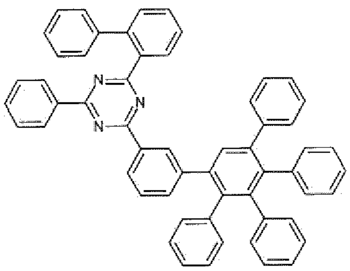 | -5.82 | -1.69 | 0.50 |
| A-8 | 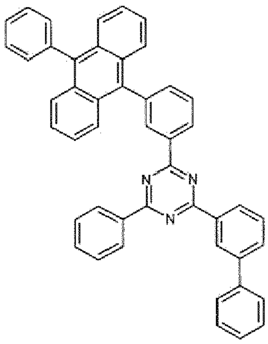 | -5.19 | -1.84 | 0.37 |
| A-9 | 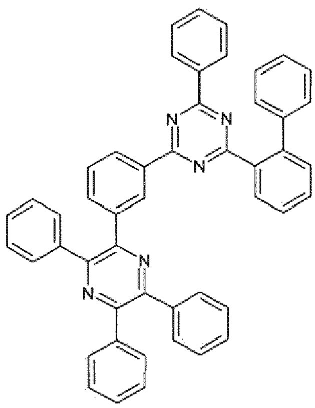 | -5.79 | -1.78 | 0.25 |

(continued)

| Name | Structure | HOMO [eV] | LUMO [eV] | Dipole moment [D] |
|---|---|---|---|---|
| A-10 | 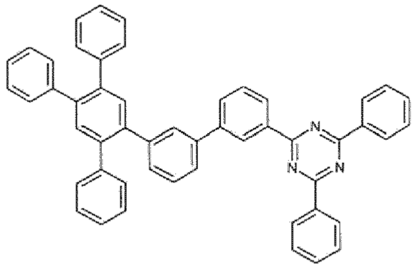 | -5.67 | -1.82 | 0.22 |
| A-11 | 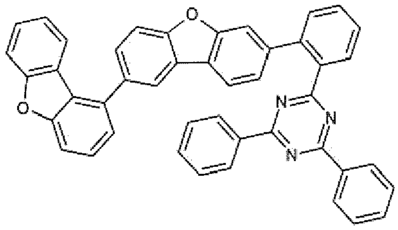 | -5.75 | -1.79 | 0.83 |
| A-12 | 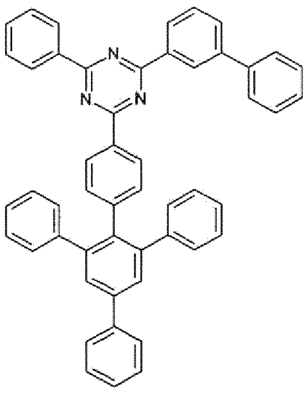 | -5.82 | -1.83 | 0.37 |
| A-13 | 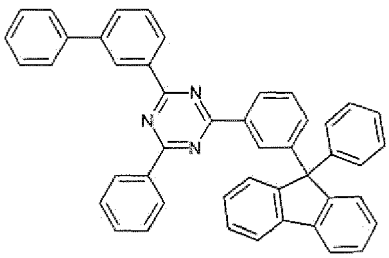 | -5.81 | -1.80 | 0.27 |
| A-14 | 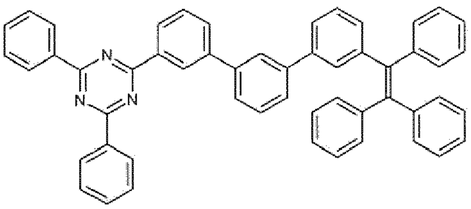 | -5.32 | -1.81 | 0.28 |
| A-15 | 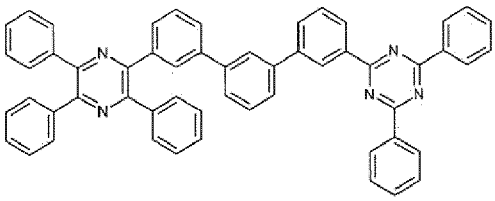 | -5.75 | -1.87 | 0.42 |

(continued)

| Name | Structure | HOMO [eV] | LUMO [eV] | Dipole moment [D] |
|---|---|---|---|---|
| A-16 | 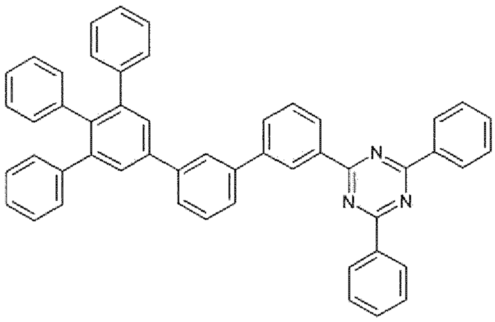 | -5.77 | -1.82 | 0.36 |
| A-17 | 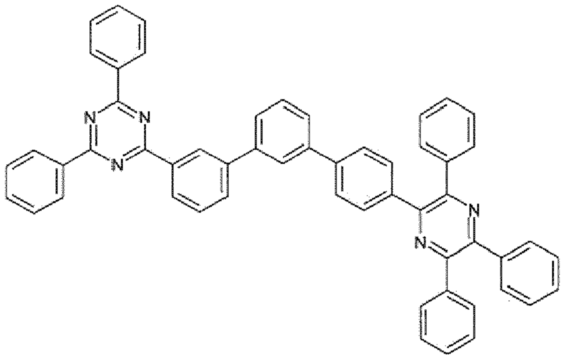 | -5-56 | -1.83 | 0.12 |
| A-18 | 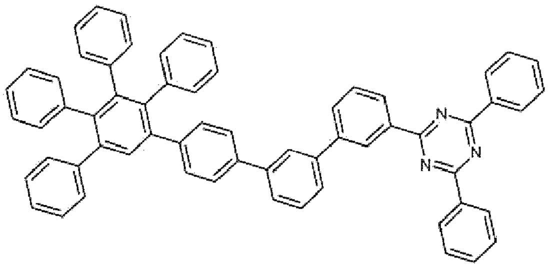 | -5.67 | -1.82 | 0.70 |
| A-19 | 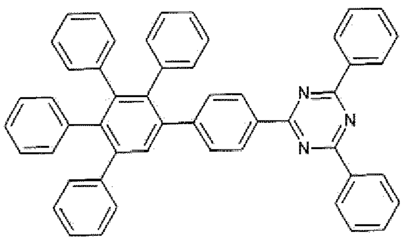 | -5.84 | -1.81 | 0.86 |
| A-20 | 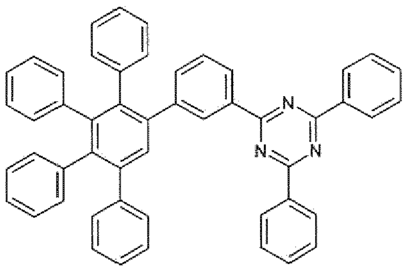 | -5.82 | -1.76 | 0.57 |

(continued)

| Name | Structure | HOMO [eV] | LUMO [eV] | Dipole moment [D] |
|---|---|---|---|---|
| A-21 | 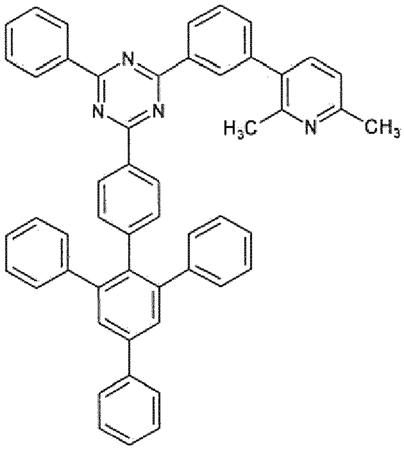 | -5.83 | -1.86 | 1.61 |
| A-22 | 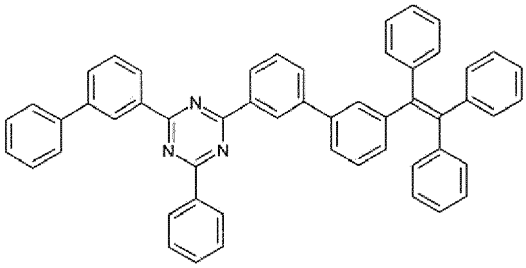 | -5.35 | -1.81 | 0.18 |
| A-23 | 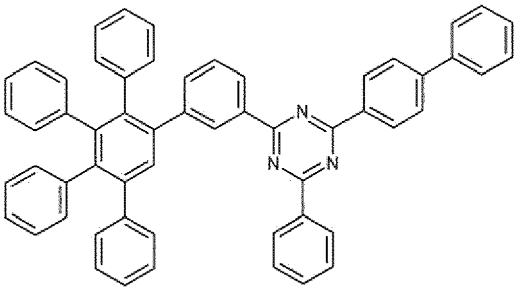 | -5.82 | -1.84 | 0.69 |
| A-24 | 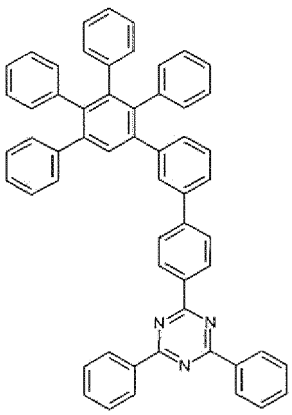 | -5.84 | -1.83 | 1.03 |

(continued)

| Name | Structure | HOMO [eV] | LUMO [eV] | Dipole moment [D] |
|---|---|---|---|---|
| A-25 | 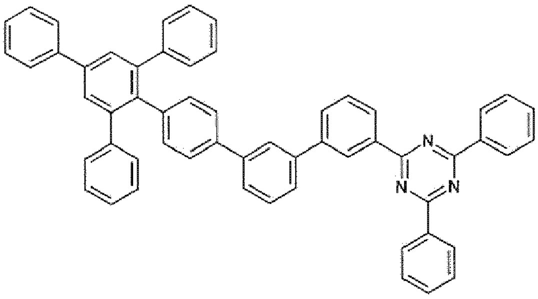 | -5.71 | -1-79 | 0.45 |
| A-26 | 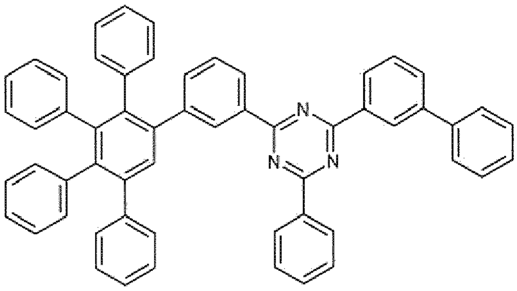 | -5.82 | -1.78 | 0.66 |
| A-27 | 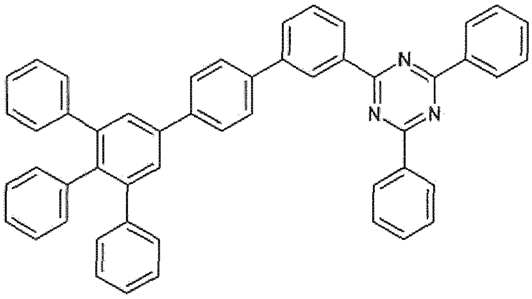 | -5.68 | -1.83 | 0.43 |
| A-28 | 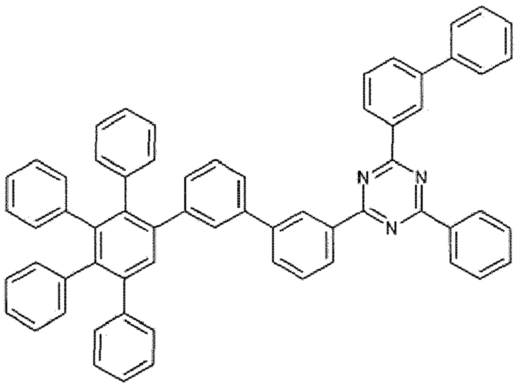 | -5.83 | -1.77 | 0.97 |

(continued)

| Name | Structure | HOMO [eV] | LUMO [eV] | Dipole moment [D] |
|---|---|---|---|---|
| A-29 | 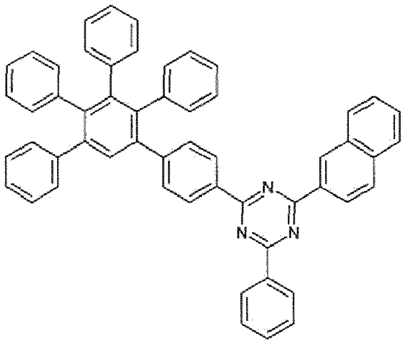 | -5.83 | -1.89 | 0.67 |

In an embodiment the LUMO energy level of the compound of formula (I) in the absolute scale taking vacuum energy level as zero, computed by the TURBOMOLE V6.5 program package using hybrid functional B3LYP and Gaussian 6-31G* basis set, is in the range from -1.90 eV to -1.60 eV, preferably from -1.85 eV to -1.65 eV.

The first electron transport layer may be arranged between the emission layer and the second electron transport layer. The first electron transport layer may be arranged in direct contact with the emission layer. The first electron transport layer may be arranged "contacting sandwiched" between the emission layer and the second electron transport layer.

The first electron transport layer may have a thickness of < 50 nm, optionally between 1 and 30 nm, optionally between 1 and 10nm, optionally between 1 and 5 nm.

Organic light emitting diode

The organic light emitting diode may further comprise an electron injection layer, wherein the electron injection layer is arranged between the electron layer stack and the cathode. The electron injection layer may be in direct contact with the electron transport layer stack. The electron injection layer may be in direct contact with the second electron transport layer. The electron injection layer may be in direct contact with the cathode. The electron injection layer may be contacting sandwiched between the second electron transport layer and the cathode.

It may be provided that the electron injection layer does not comprise the compound of formula (I). It may be provided that the electron injection layer does not comprise the compound of formula (II). It may be provided that the electron injection layer does not comprise the compound of formula (III). It may be provided that the electron injection layer does not comprise any of the compounds of formulas (I) and (II). It may be provided that the electron injection layer does not comprise any of the compounds of formulas (I), (II) and (III).

The electron injection layer may comprise a first electron injection sub-layer and a second electron injection sub-layer, wherein the first electron injection sub-layer and the second electron injection sub-layer are in direct contact with each other.

The first electron injection sub-layer may be in direct contact with the electron transport layer and the first electron injection sub-layer may comprise a metal salt or a metal complex, preferably a lithium salt or a lithium organic complex; more preferably a compound selected from lithium halides and lithium organic chelates; even more preferably from lithium fluoride, a lithium quinolinolate, lithium borate, lithium phenolate, lithium pyridinolate or from a lithium complex with a Schiff base ligand; most preferably,

- the lithium complex has the formula II, III or IV:

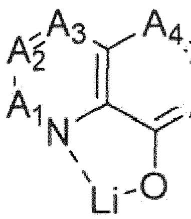
(II),
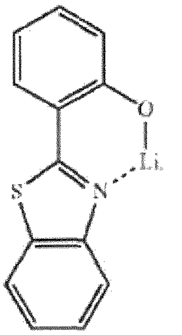
(III),
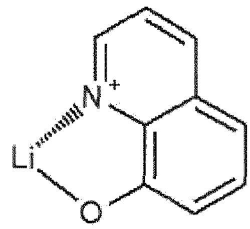
(IV)

wherein

$A_1$ to $A_6$ are same or independently selected from CH, CR, N, O;

R is same or independently selected from hydrogen, halogen, alkyl or aryl or heteroaryl with 1 to 20 carbon atoms; and more preferred $A_1$ to $A_6$ are CH,

- the borate based organic ligand is a tetra(1H-pyrazol-1-yl)borate,

- the phenolate is a 2-(pyridin-2-yl)phenolate, a 2-(diphenylphosphoryl)phenolate, an imidazol phenolate, 2-(pyridin-2-yl)phenolate or 2-(1-phenyl-1H-benzo[d]imidazol-2-yl)phenolate,

- the pyridinolate is a 2-(diphenylphosphoryl)pyridin-3-olate,

- the lithium Schiff base has the structure 100, 101, 102 or 103:

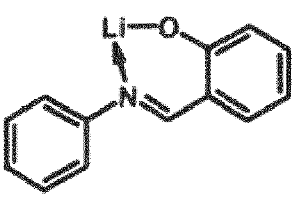
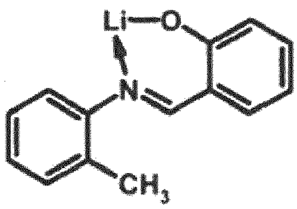
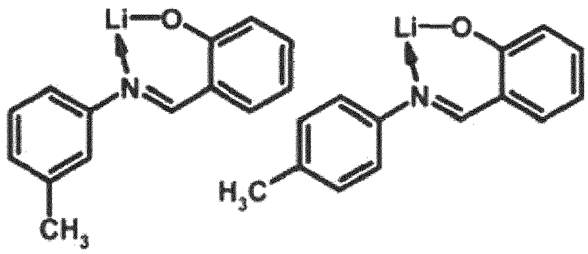

100 101 102 103

Preferably, the first electron injection sub-layer comprises 8-hydroxyquinolinolato-lithium (= LiQ).

The second electron injection sub-layer comprises a metal selected form the group consisting of alkali metals, alkaline earth metals, and rare earth metals, preferably the metal may be selected from Li, Na, K, Rb, Cs, Mg, Ca, Sr, Ba, Sm, Eu, Tm, Yb; more preferably from Li, Na, K, Rb, Cs, Mg and Yb, even more preferably from Li, Na, Cs and Yb, most preferably from Li, Na and Yb most preferred Yb.

The second electron injection sub-layer may be in direct contact with the cathode.

The organic light emitting diode may further comprise an n-type charge generation layer, wherein the n-type charge generation layer is arranged between the electron layer stack and the cathode. The n-type charge generation layer may be in direct contact with the electron transport layer stack. The n-type charge generation layer may be in direct contact with the second electron transport layer. The n-type charge generation layer may be in direct contact with the cathode. The n-type charge generation layer may be contacting sandwiched between the second electron transport layer and the cathode.

The organic light emitting diode may not comprise an electron injection layer.

The organic light emitting diode may not comprise an n-type charge generation layer.

*Further layers*

In accordance with the invention, the organic light emitting diode may comprise, besides the layers already

mentioned above, further layers. Exemplary embodiments of respective layers are described in the following:

*Substrate*

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate or a silicon substrate.

*Anode electrode*

Either a first electrode or a second electrode comprised in the inventive organic electronic device may be an anode electrode. The anode electrode may be formed by depositing or sputtering a material that is used to form the anode electrode. The material used to form the anode electrode may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide ($SnO_2$), aluminum zinc oxide (AlZO) and zinc oxide (ZnO), may be used to form the anode electrode. The anode electrode may also be formed using metals, typically silver (Ag), gold (Au), or metal alloys.

*Hole injection layer*

A hole injection layer (HIL) may be formed on the anode electrode by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of 10-8 to 10-3 Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise or consist of p-type dopant and the p-type dopant may be selected from tetrafluoro-tetracyanoquinonedimethane ($F_4$TCNQ), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto. The HIL may be selected from a hole-transporting matrix compound doped with a p-type dopant. Typical examples of known doped hole transport materials are: copper phthalocyanine (CuPc), which HOMO level is approximately -5.2 eV, doped with tetrafluoro-tetracyanoquinonedimethane ($F_4$TCNQ), which LUMO level is about -5.2 eV; zinc phthalocyanine (ZnPc) (HOMO = -5.2 eV) doped with $F_4$TCNQ; $\alpha$-NPD (N,N'-Bis(naphthalen-1-yl)-N,N'-bis(phenyl)-benzidine) doped with $F_4$TCNQ. $\alpha$-NPD doped with 2,2'-(perfluoronaphthalen-2,6-diylidene) dimalononitrile. The p-type dopant concentrations can be selected from 1 to 20 wt.-%, more preferably from 3 wt.-% to 10 wt.-%.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

*Hole transport layer*

A hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010

and incorporated by reference. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

When the thickness of the HTL is within this range, the HTL may have excellent hole transporting characteristics, without a substantial penalty in driving voltage.

*Electron blocking layer*

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

*Emission layer (EML)*

The emission layer in the organic light emitting diode in accordance with the invention may be a blue emission layer or a green emission layer.

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

It may be provided that the emission layer does not comprise the compound of Formula (II) or (I).

The emission layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc ($Zn(BTZ)_2$).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, $Ir(piq)_3$, and $Btp_2Ir(acac)$, but are not limited thereto. These compounds are phosphorescent emitters, however, fluorescent red emitter dopants could also be used.

Examples of phosphorescent green emitter dopants are $Ir(ppy)_3$ (ppy = phenylpyridine), $Ir(ppy)_2(acac)$, $Ir(mpyp)_3$.

Examples of phosphorescent blue emitter dopants are $F_2Irpic$, $(F_2ppy)_2Ir(tmd)$ and $Ir(dfppz)_3$ and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

*Hole blocking layer (HBL)*

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function. The hole blocking layer may be the first electron transport layer comprising or consisting of the compound of formula (I) as defined above.
The HBL may also be named auxiliary ETL or a-ETL.
When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, and phenanthroline derivatives.
The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.
The hole blocking layer may also be described as a-ETL or auxiliary ETL.
According to an embodiment, a hole blocking layer is arranged between the at least one emission layer and the second electron transport layer comprising the compound of formula (II).
The HBL may be free of the compound of formula (II). The HBL may be free of the compound (III). The HBL may be free of the compound of formula (II) and of the compound (III).
In an embodiment the OLED according to the present invention does not comprise a first electron transport layer. In an embodiment the OLED according to the present invention does not comprise a hole blocking layer (HBL).
In an embodiment the OLED according to the present invention does not comprise a first electron transport layer and the triplet energy level T1 of the compound of formula (II) is in the range of 2.45 to 3.00 eV, preferably between 2.47 to 3.00 eV, more preferred between 2.60 and 3.00 eV, more preferred between 2.65 to 2.99 eV.
In an embodiment the OLED according to the present invention does not comprise a first electron transport layer and the triplet energy level T1 of the compound of formula (IV) is in the range of 2.45 to 3.00 eV, preferably between 2.47 to 3.00 eV, more preferred between 2.60 and 3.00 eV, more preferred between 2.65 to 2.99 eV.

*Electron transport layer (ETL)*

The OLED according to the present invention comprises one or more electron transport layer(s) (ETL). In accordance with the invention, at least one of the electron transport layers is the inventive electron transport layer comprising the compound of formula (II) as defined herein.
According to various embodiments the OLED may comprise an electron transport layer or an electron transport layer stack comprising at least a first electron transport layer and at least a second electron transport layer.
The electron transport layer may be the second electron transport layer.
By suitably adjusting energy levels of particular layers of the ETL, the injection and transport of the electrons may be controlled, and the holes may be efficiently blocked. Thus, the OLED may have long lifetime.
The electron transport layer may comprise, besides the compound of formula (II) further ETM materials known in the art. Likewise, the electron transport layer may comprise as the only electron transport matrix material the compound of formula (II). In case that the inventive organic electronic device comprises more than one electron transport layers, the compound of formula (II) may be comprised in only one of the electron transport layers, in more than one of the electron transport layers or in all of the electron transport layers. Suitable further compounds for the ETM are not particularly limited. In one embodiment, the electron transport matrix compounds consist of covalently bound atoms. Preferably, the electron transport matrix compound comprises a conjugated system of at least 6, more preferably of at least 10 delocalized electrons. In one embodiment, the conjugated system of delocalized electrons may be comprised in aromatic or heteroaromatic structural moieties, as disclosed e.g. in documents EP 1 970 371 A1 or WO 2013/079217 A1.
According to the present invention the electron transport layer is free of an electrical dopant, such as an n-type dopant, especially a redox n-type dopant. The term "free of" in this regard does not exclude impurities. Impurities have no technical effect with respect to the object achieved by the present invention. Impurities are not deliberately added to the layer during processing.
The term "free of" a compound means that such compound is not deliberately added to the layer during processing.
The electron transport layer may comprise a compound (III).

*Electron injection layer (EIL)*

An EIL, which may facilitate injection of electrons from the cathode into the electron transport layer stack, may be formed on the electron transport layer stack, preferably directly on the electron transport layer stack, preferably directly on the second electron transport layer, preferably in direct contact with the second electron transport layer. Examples of materials for forming the EIL or being comprised in the EIL include lithium 8-hydroxyquinolinolate (LiQ), LiF, NaCl, CsF, Li2O, BaO, Ca, Ba, Yb, Mg which are known in the art. Deposition and coating conditions for forming the EIL are similar to those for formation of the HIL, although the deposition and coating conditions may vary, according to the material that is used to form the EIL. The EIL may comprise an organic matrix material doped with an n-type dopant. The matrix material may be selected from materials conventionally used as matrix materials for electron transport layers.

The EIL may consist of a number of individual EIL sublayers. In case the EIL consists of a number of individual EIL sublayers, the number of sublayers is preferably 2. The individual EIL sublayers may comprise different materials for forming the EIL.

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

The electron transport stack of the present invention is not part of the electron injection layer.

The electron injection layer may be in direct contact with the electron transport layer.

The electron injection layer may be is in direct contact with the second electron transport layer.

*Cathode electrode*

The cathode electrode is formed on the EIL if present, preferably directly on the EIL, preferably in direct contact with the EIL. In the sense of this invention the cathode and the EIL can be regarded as one functional part enabling the injection of electrons into the electron transport layer stack. The cathode electrode may be formed of a metal, an alloy, an electrically conductive compound, or a mixture thereof. The cathode electrode may have a low work function. For example, the cathode electrode may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode electrode may be formed of a transparent conductive oxide, such as ITO or IZO.

The thickness of the cathode electrode may be in the range from about 5 nm to about 1000 nm, for example, in the range from about 10 nm to about 100 nm. When the thickness of the cathode electrode is in the range from about 5 nm to about 50 nm, the cathode electrode may be transparent or semitransparent even if formed from a metal or metal alloy. The transparent or semitransparent cathode may facilitate light emission through the cathode.

It is to be understood that the cathode electrode and the electron injection layer are not part of the electron transport layer stack.

*Charge generation layer (CGL)*

The charge generation layer (CGL) may comprise a p-type charge generation layer (p-CGL) and an n-type charge generation layer (n-CGL). An interlayer may be arranged between the p-CGL and the -n-CGL.

Typically, the charge generation layer is a p-n junction joining an n-type charge generation layer (electron generating layer) and a hole generating layer. The n-side of the p-n junction generates electrons and injects them into the layer that is adjacent in the direction to the anode. Analogously, the p-side of the p-n junction generates holes and injects them into the layer that is adjacent in the direction to the cathode.

Charge generating layers are used in tandem and stacked devices, for example, in tandem or stacked OLEDs comprising, between two electrodes, two or more emission layers. In a tandem or stacked OLED comprising two emission layers, the n-type charge generation layer provides electrons for the first light emission layer arranged near the anode, while the hole generating layer provides holes to the second light emission layer arranged between the first emission layer and the cathode.

Suitable matrix materials for the hole generating layer may be materials conventionally used as hole injection and/or hole transport matrix materials. Also, p-type dopant used for the hole generating layer can employ conventional materials. For example, the p-type dopant can be one selected from a group consisting of tetrafluoro-7,7,8,8-tetracyanoquinodimethane ($F_4$-TCNQ), derivatives of tetracyanoquinodimethane, radialene derivatives, iodine, $FeCl_3$, $FeF_3$, and $SbCl_5$. Also, the host can be one selected from a group consisting of N,N'-di(naphthalen-1-yl)-N,N-diphenyl-benzidine (NPB), N,N'-diphenyl-N,N'-bis(3-methylphenyl)-1,1-biphenyl-4,4'-diamine (TPD) and N,N',N'-tetranaphthyl-benzidine (TNB). The p-type charge generation layer may consist of CNHAT.

The n-type charge generating layer may be the layer comprising the compound of Formula (I). The n-type charge generation layer can be layer of a neat n-type dopant, for example of a metal, or can consist of an organic matrix

material doped with the n-type dopant. In one embodiment, the n-type dopant can be alkali metal, alkali metal compound, alkaline earth metal, alkaline earth metal compound, a transition metal, a transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. More specifically, the n-type dopant can be one selected from a group consisting of Li, Cs, K, Rb, Mg, Na, Ca, Sr, Eu and Yb. Suitable matrix materials for the electron generating layer may be the materials conventionally used as matrix materials for electron injection or electron transport layers. The matrix material can be for example one selected from a group consisting of triazine compounds, hydroxyquinoline derivatives like tris(8-hydroxyquinoline)aluminum, benzazole derivatives, and silole derivatives.

The CGL may be free of the compound of formula (I).

The CGL may be free of the compound of formula (II).

The CGL may be free of the compound of formula (III).

The CGL may be free of the compound formula (I) and free of the compound of formula (II).

The CGL may be free of the compound formula (I) and free of the compound of formula (II) and free of the compound (III).

The hole generating layer may be arranged in direct contact to the n-type charge generation layer.

According to one aspect of the present invention, the electron transport layer is arranged between the first and second emission layer.

The CGL may be in direct contact with the second electron transport layer.

The n-CGL may be in direct contact with the second electron transport layer.

According to one aspect of the present invention, the electron transport layer comprising the compound of formula (II) is arranged between a first and a second emission layer and an electron transport layer comprising the compound of formula (II) is arranged between the second emission layer and the cathode.

According to one aspect of the present invention, there is provided an organic light-emitting diode (OLED) comprising: a substrate; an anode electrode formed on the substrate; a hole injection layer, a hole transport layer, an emission layer, an electron transport layer comprising the compound of formula (II) and a cathode electrode.

According to another aspect of the present invention, there is provided an OLED comprising: a substrate; an anode electrode formed on the substrate; a hole injection layer, a hole transport layer, an electron blocking layer, an emission layer, a hole blocking layer, an electron transport layer comprising the compound of formula (II) and a cathode electrode.

According to another aspect of the present invention, there is provided an OLED comprising: a substrate; an anode electrode formed on the substrate; a hole injection layer, a hole transport layer, an electron blocking layer, an emission layer, a hole blocking layer, a first electron transport layer comprising the compound of formula (I), a second electron transport layer comprising the compound of formula (II), an electron injection layer, and a cathode electrode.

According to various embodiments of the present invention, there may be provided OLEDs layers arranged between the above mentioned layers, on the substrate or on the top electrode.

According to one aspect, the OLED can comprise a layer structure of a substrate that is adjacent arranged to an anode electrode, the anode electrode is adjacent arranged to a first hole injection layer, the first hole injection layer is adjacent arranged to a first hole transport layer, the first hole transport layer is adjacent arranged to a first electron blocking layer, the first electron blocking layer is adjacent arranged to a first emission layer, the first emission layer is adjacent arranged to a first electron transport layer, the first electron transport layer is adjacent arranged to an n-type charge generation layer, the n-type charge generation layer is adjacent arranged to a hole generating layer, the hole generating layer is adjacent arranged to a second hole transport layer, the second hole transport layer is adjacent arranged to a second electron blocking layer, the second electron blocking layer is adjacent arranged to a second emission layer, between the second emission layer and the cathode electrode an optional electron transport layer and/or an optional injection layer are arranged.

For example, the OLED according to Fig. 2 may be formed by a process, wherein on a substrate (110), an anode (120), a hole injection layer (130), a hole transport layer (140), an electron blocking layer (145), an emission layer (150), a hole blocking layer (155), an electron transport layer (160), an electron injection layer (180) and the cathode electrode (190) are subsequently formed in that order.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device or a lighting device, most preferably a display device.

In one embodiment, the organic light emitting diode according to the invention further comprises a layer comprising a radialene compound and/or a quinodimethane compound.

In one embodiment, the radialene compound and/or the quinodimethane compound may be substituted with one or more halogen atoms and/or with one or more electron withdrawing groups. Electron withdrawing groups can be selected from nitrile groups, halogenated alkyl groups, alternatively from perhalogenated alkyl groups, alternatively from

perfluorinated alkyl groups. Other examples of electron withdrawing groups may be acyl, sulfonyl groups or phosphoryl groups.

Alternatively, acyl groups, sulfonyl groups and/or phosphoryl groups may comprise halogenated and/or perhalogenated hydrocarbyl. In one embodiment, the perhalogenated hydrocarbyl may be a perfluorinated hydrocarbyl. Examples of a perfluorinated hydrocarbyl can be perfluormethyl, perfluorethyl, perfluorpropyl, perfluorisopropyl, perfluorobutyl, perfluorophenyl, perfluorotolyl; examples of sulfonyl groups comprising a halogenated hydrocarbyl may be trifluoromethylsulfonyl, pentafluoroethylsulfonyl, pentafluorophenylsulfonyl, heptafluoropropylsufonyl, nonafluorobutylsulfonyl, and like.

In one embodiment, the radialene and/or the quinodimethane compound may be comprised in a hole injection, hole transporting and/or a hole generation layer.

In one embodiment, the radialene compound may have Formula (XX) and/or the quinodimethane compound may have Formula (XXIa) or (XXIb):

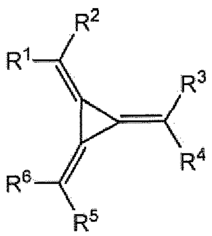
(XX)
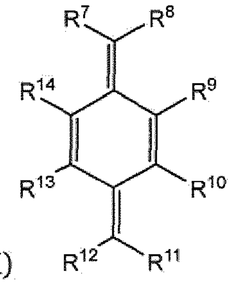
(XXIa)
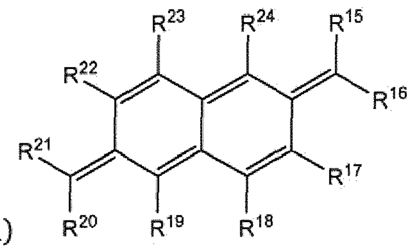
(XXIb),

wherein (as an exception different to the description above) $R^1$, $R^2$, $R^3$, $R^4$, $R^5$, $R^6$, $R^7$, $R^8$, $R^{11}$, $R^{12}$, $R^{15}$, $R^{16}$, $R^{20}$, $R^{21}$ are independently selected from above mentioned electron withdrawing groups and $R^9$, $R^{10}$, $R^{13}$, $R^{14}$, $R^{17}$, $R^{18}$, $R^{19}$, $R^{22}$, $R^{23}$ and $R^{24}$ are independently selected from H, halogen and above mentioned electron withdrawing groups.

## Process for preparing the organic electronic device

According to a further aspect, the invention is related to a process for preparing the organic electronic device according to the present invention, wherein the process comprises a step of depositing the compound of formula (II) according to the present invention on a solid support.

The method for depositing may comprise:

- deposition via vacuum thermal evaporation;
- deposition via solution processing, preferably the processing is selected from spincoating, printing, casting; and/or
- slot-die coating.

## Display device

According to a further aspect, the invention is related to a display device comprising the organic light emitting diode according to the invention, preferably comprises at least two organic light emitting diodes according to the invention.

## Compound of formula (IV)

According to a further aspect, the invention is related to a compound of formula (IV)

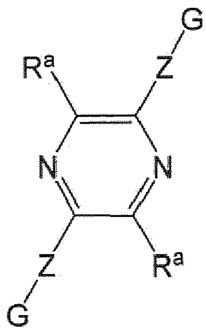

(IV).

The two $R^a$ may be selected different or the same. The two $R^a$ may be selected the same. The two Z may be selected different or the same. The two Z may be selected the same.

The two G may be selected different or the same. The two G may be selected the same.

$R^a$ is independently selected from the group consisting of substituted or unsubstituted $C_6$ to $C_{24}$ aryl and substituted or unsubstituted $C_3$ to $C_{18}$ heteroaryl, wherein if R is substituted, the one or more substituents are independently selected from the group consisting of D, phenyl, and $C_1$ to $C_{12}$ alkyl.

$R^a$ may be independently selected from the group consisting of substituted or unsubstituted $C_6$ to $C_{18}$ aryl and substituted or unsubstituted $C_3$ to $C_7$ heteroaryl, wherein if $R^a$ is substituted, the one or more substituents are independently selected from the group consisting of D, and $C_1$ to $C_{12}$ alkyl.

$R^a$ may be independently selected from the group consisting of substituted or unsubstituted $C_6$ to $C_{12}$ aryl and substituted or unsubstituted $C_5$ to $C_{11}$ heteroaryl, wherein if $R^a$ is substituted, the one or more substituents are independently selected from the group consisting of D, phenyl, and $C_1$ to $C_4$ alkyl,

$R^a$ may be independently selected from the group consisting of

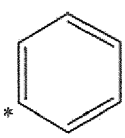 , 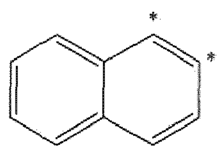 , 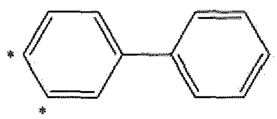 , 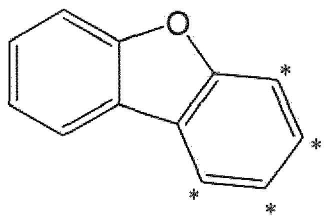 ,

wherein the respective group is bonded at one of the * to

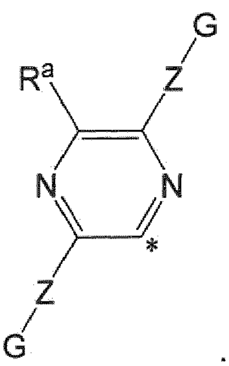

.

Z is independently selected from the group consisting of a single bond and $C_6$ to $C_{24}$ arylene. Z may be independently selected from the group consisting of a single bond and $C_6$ to $C_{18}$ arylene. Z may be independently selected from the group consisting of a single bond and $C_6$ to $C_{12}$ arylene. Z may be independently selected from the group consisting of a single bond and $C_6$ to $C_{10}$ arylene.

Z may be independently selected from

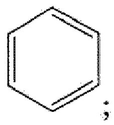 ; 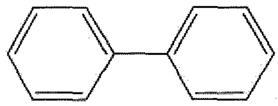 ; and 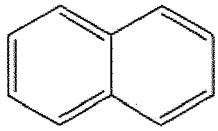 .

Z may be independently selected from

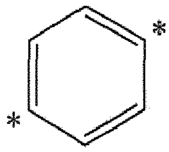 ; 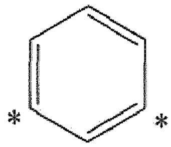 ;

and;
wherein Z is bonded to

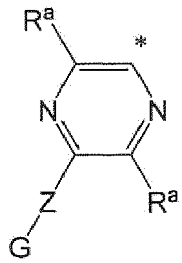

and *G at *, respectively.

G is independently selected from (IVa-1) to (IVa-3)

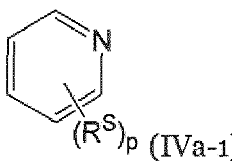 (IVa-1) 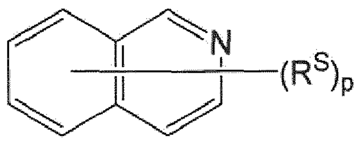 (VIa-2) 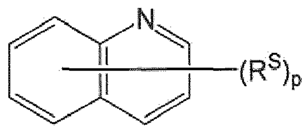 (VIa-3);

In accordance with the present disclosure, in a formula showing the following binding situation,

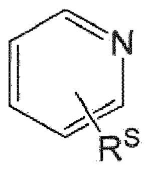

the group $R^s$ may be bonded to any suitable binding position, that is, to any of the carbon atoms not bonded to Z.

In a situation where it is shown that the bond of $R^s$ crosses more than one ring

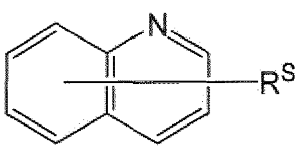 or 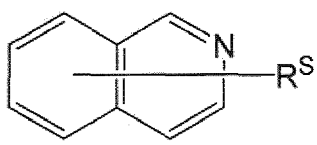

the group $R^s$ may be bound to any suitable binding position of each ring crossed with the bond, that is, to any of the carbon atoms not bonded to Z.

Z may be bonded in ortho-, meta-, or para-position with respect to the N-atom of the pyridine ring in formula IIa-1 and the remaining positions each may or may not have a substituent $R^s$.

Z may be bonded in ortho-, or meta-position with respect to the N-atom of the pyridine ring in formula IIa-2 or to any of the carbon atoms of the ring annelated to the pyridine ring and the remaining positions each may or may not have a substituent $R^s$.

Z may be bonded in meta- or para-position with respect to the N-atom of the pyridine ring in formula IIa-3 or to any of the carbon atoms of the ring annelated to the pyridine ring and the remaining positions each may or may not have a substituent $R^s$.

$R^S$ is independently selected from the group consisting of D, $C_1$ to $C_{12}$ aryl, $C_1$ to $C_{12}$ alkyl, and pyridyl, wherein the respective C, to $C_{12}$ aryl and pyridyl can be unsubstituted or substituted with one or more of D and $C_1$ to $C_4$ alkyl.

$R^S$ may be independently selected from the group consisting of D, $C_1$ to $C_{10}$ aryl, $C_1$ to $C_4$ alkyl, and pyridyl, wherein the respective $C_1$ to $C_{10}$ aryl and pyridyl can be unsubstituted or substituted with one or more of D and $C_1$ to $C_4$ alkyl.

$R^S$ may be independently selected from the group consisting of D, phenyl, naphthyl, $C_1$ to $C_{12}$ alkyl, and pyridyl.

$R^S$ may be independently selected from the group consisting of D, phenyl, naphthyl, $C_1$ to $C_4$ alkyl, and pyridyl.

$R^s$ may be independently selected from the group consisting of

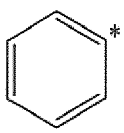 ; 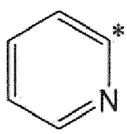 ; 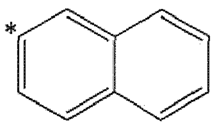 ; 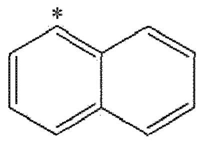 ; *$CH_3$

wherein the respective $R^s$ is bonded at * to Z.

p is an integer from o to 4 for IVa-1. p may by an integer from o to 2 for Iva-1. p may by an integer from o to 1 for IVa-1.

p is an integer from o to 6 for IVa-2 and IVa-3. p may be an integer from o to 4 for IVa-2 and IVa-3. p may be an integer from o to 2 for IVa-2 and IVa-3. p may be an integer from o to 1 for IVa-2 and IVa-3.

The compound of formula (IV) may be selected from B-1 to B-44

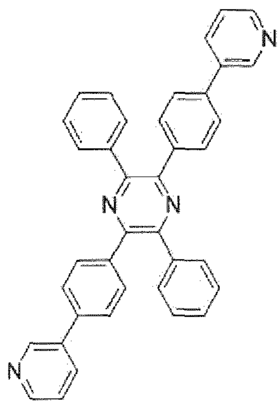
B-1

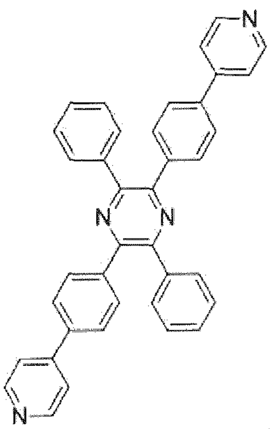
B-2

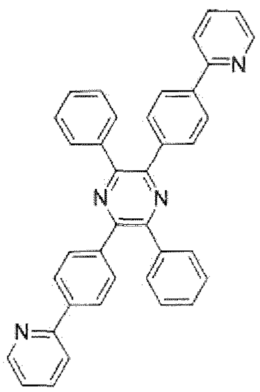
B-3

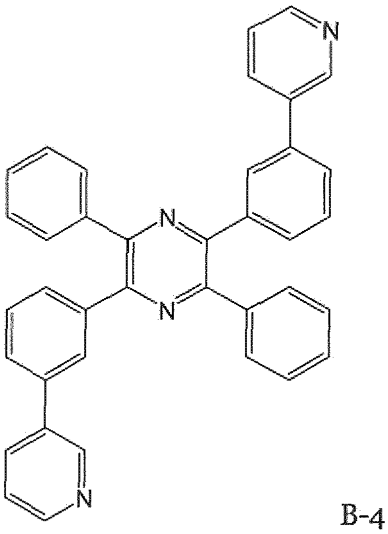

B-4

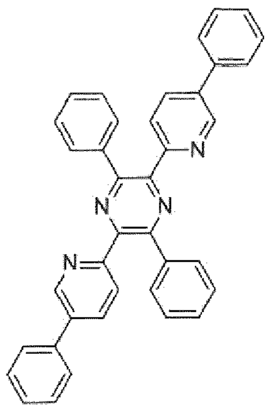

B-5

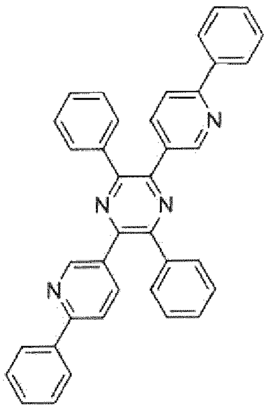

B-6

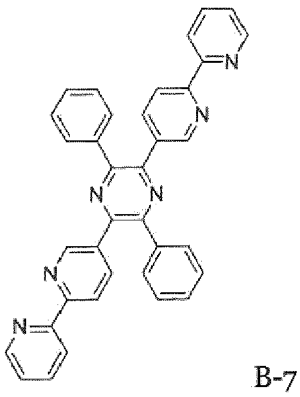

B-7

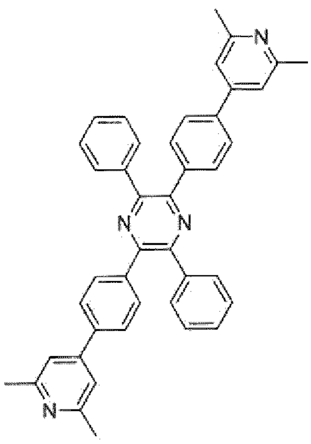

B-8

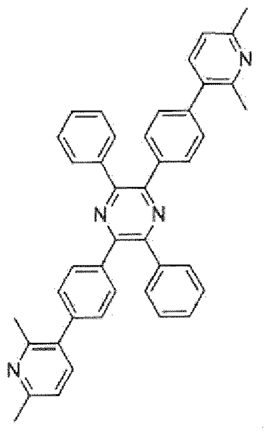

B-9

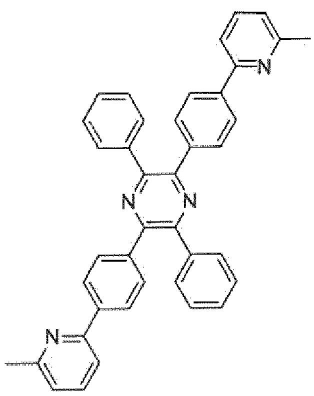

B-10

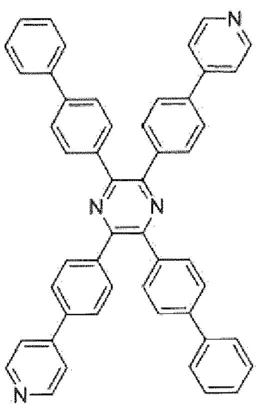

B-11

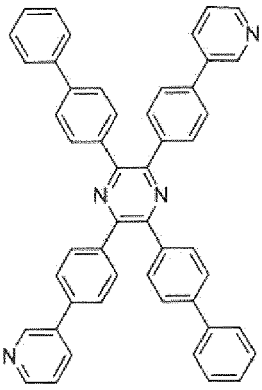

B-12

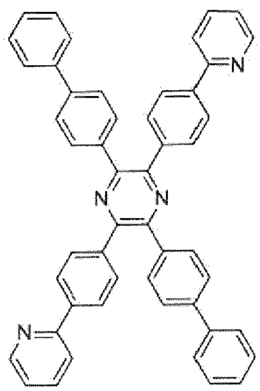

B-13

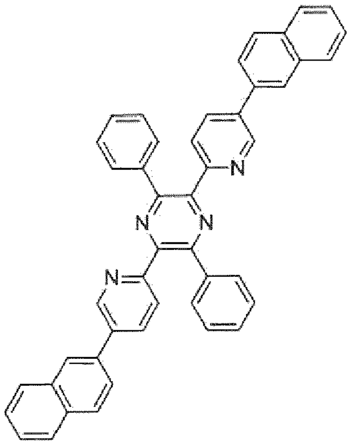

B-14

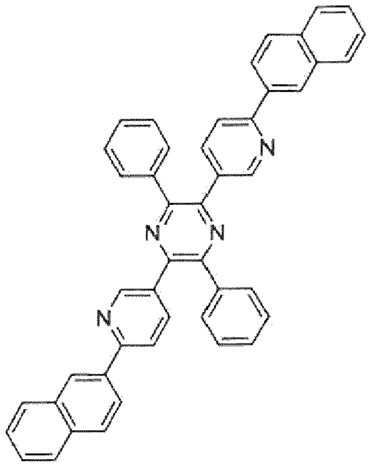

B-15

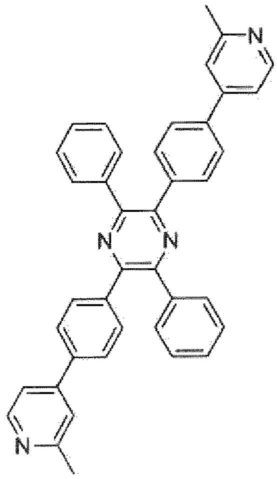

B-16

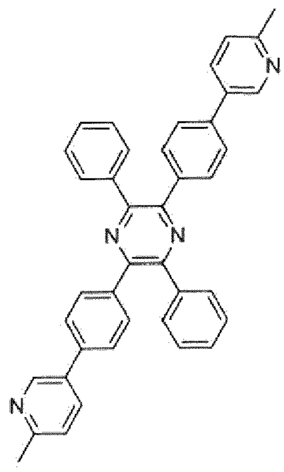

B-17

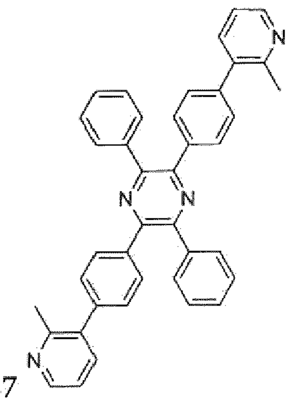

B-18

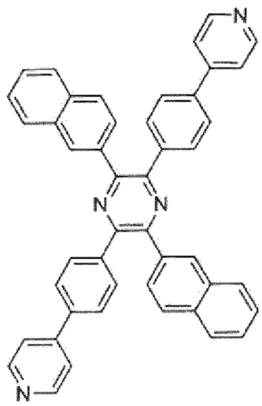

B-19

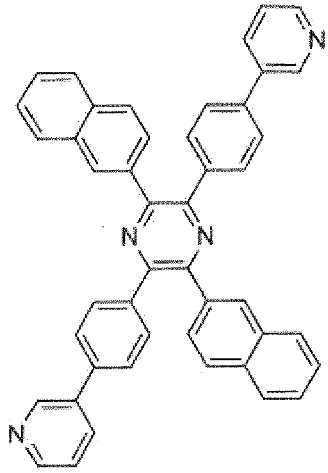

B-20

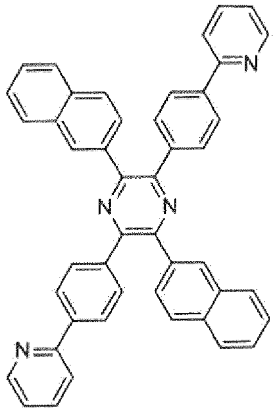

B-21

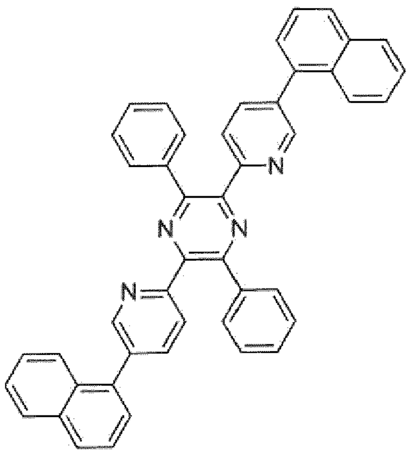

B-22

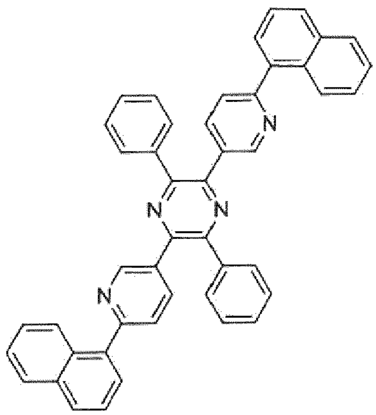

B-23

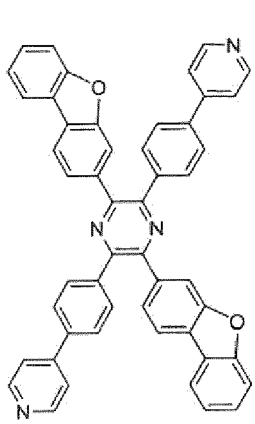

B-24

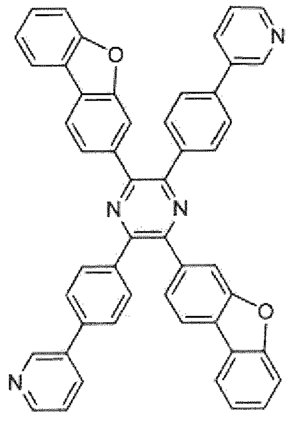

B-25

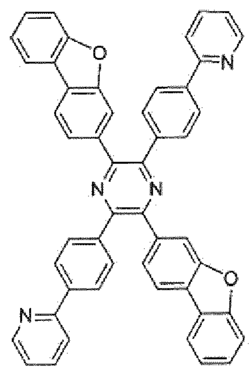

B-26

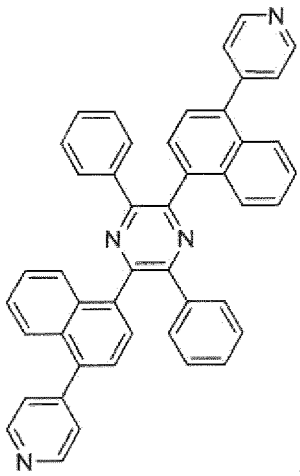
B-27

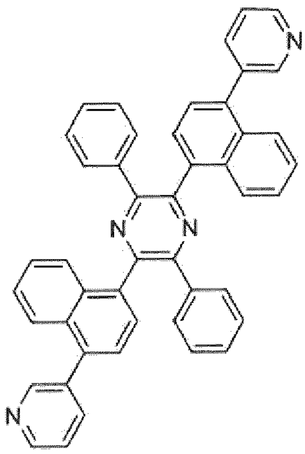
B-28

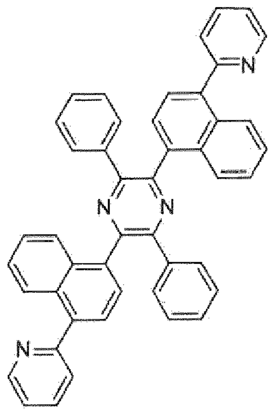
B-29

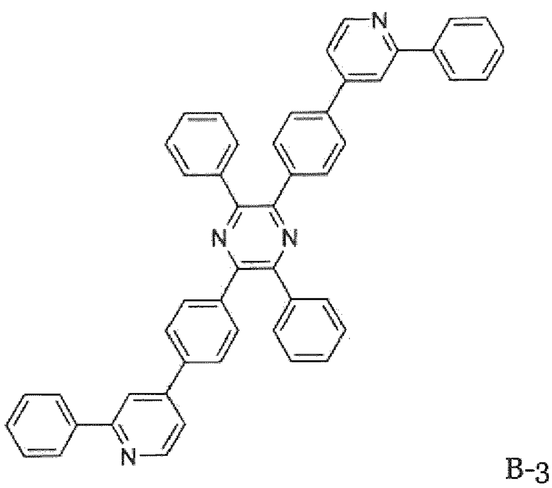
B-30

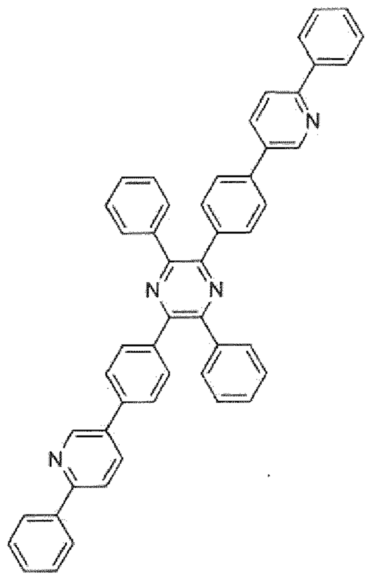
B-31

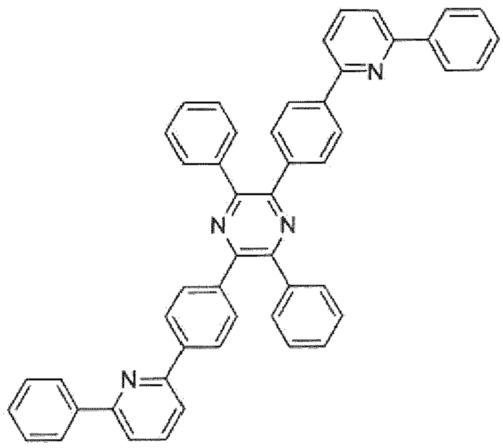
B-32

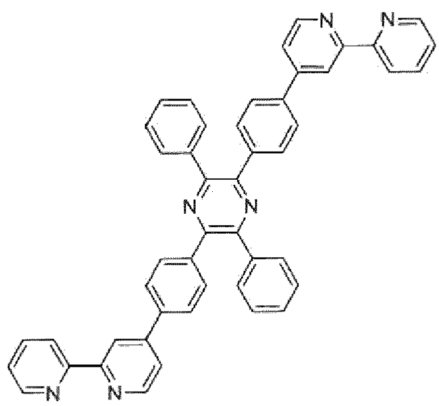
B-33

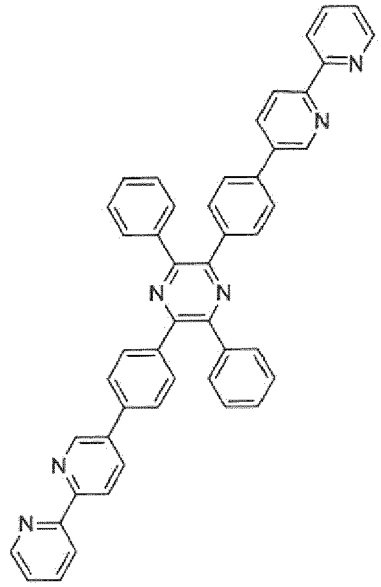

B-34

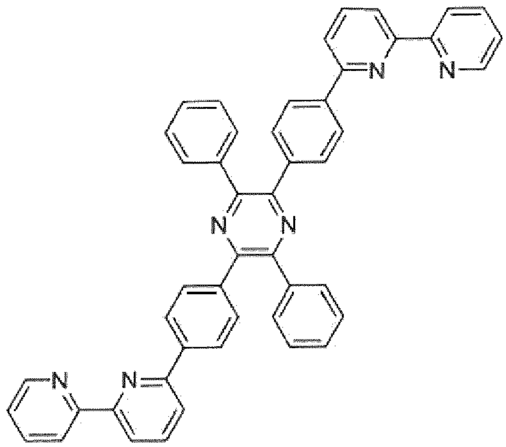

B-35

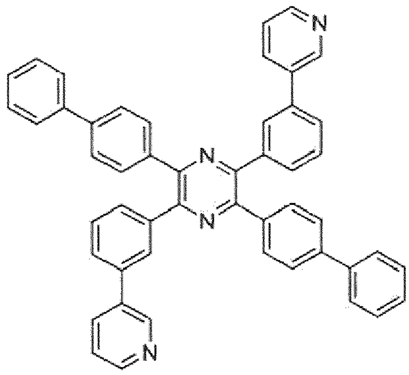

B-36

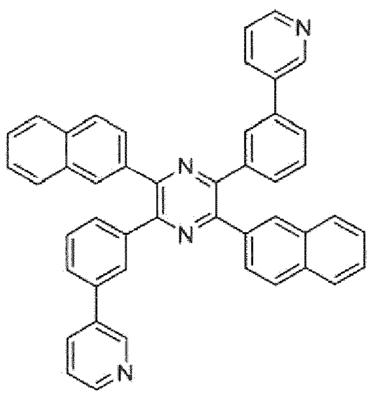

B-37

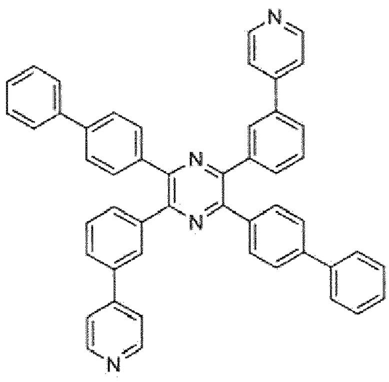

B-38

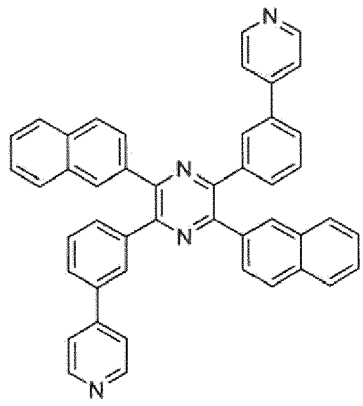

B-39

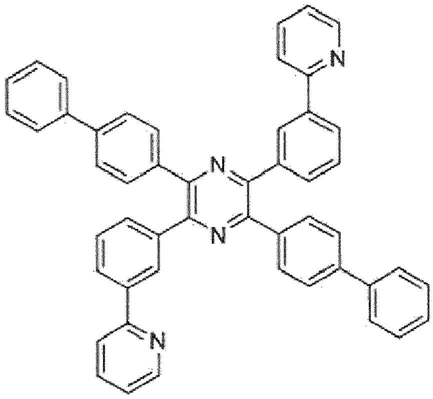

B-40

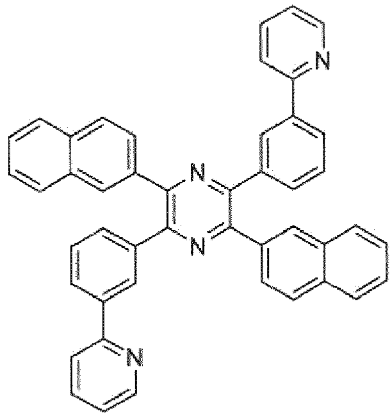

B-41

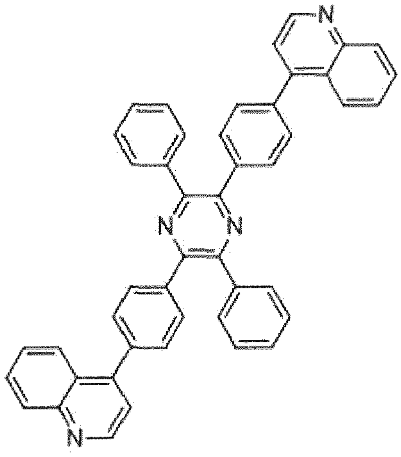

B-42

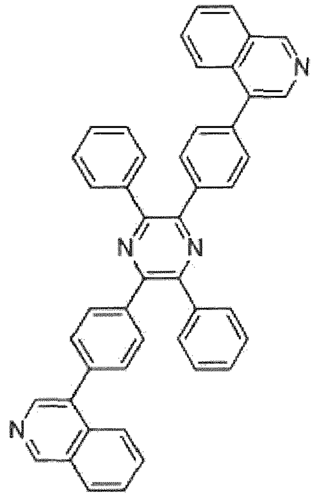

B-43

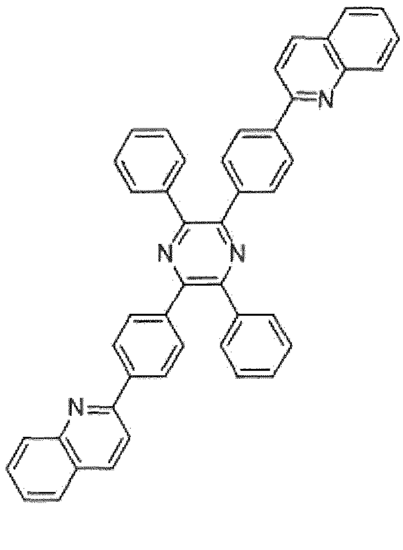

B-44.

The compound of formula (IV) may be selected from B-1 and B-4

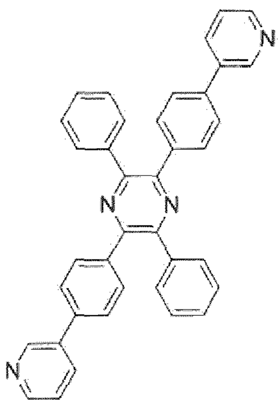

B-1

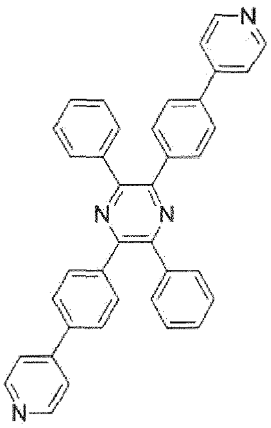

B-2

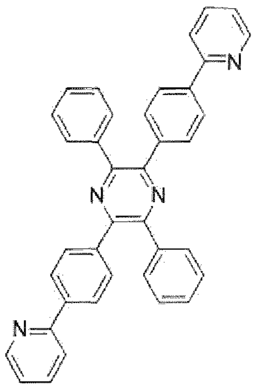

B-3

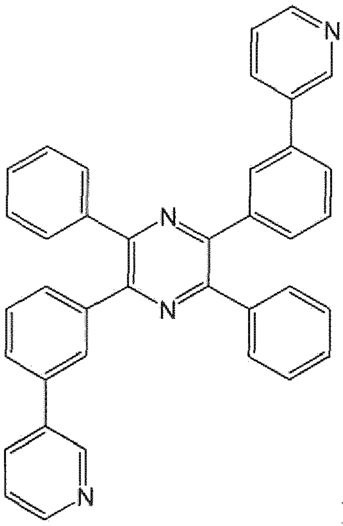

B-4.

GENERAL DEFINITIONS

If not explicitly mentioned else, each moiety of the compounds described herein, especially of the compounds of formulas (I) and (II) and compound (III) may be substituted with one or more D (deuterium) and/or $C_1$ to $C_4$ alkyl.

In the present specification, when a definition is not otherwise provided, an "alkyl group" may refer to an aliphatic hydrocarbon group. The alkyl group may refer to "a saturated alkyl group" without any double bond or triple bond. The term "alkyl" as used herein shall encompass linear as well as branched and cyclic alkyl. For example, $C_3$-alkyl may be selected from n-propyl and iso-propyl. Likewise, $C_4$-alkyl encompasses n-butyl, sec-butyl and t-butyl. Likewise, $C_6$-alkyl encompasses n-hexyl and cyclo-hexyl.

As used herein if not explicitly mentioned else, the asterisk symbol "*" represents a binding position at which the moiety labelled accordingly is bond to another moiety.

The subscribed number n in $C_n$ relates to the total number of carbon atoms in the respective alkyl, arylene, heteroarylene or aryl group.

The term "aryl" or "arylene" as used herein shall encompass phenyl ($C_6$-aryl), fused aromatics, such as naphthalene, anthracene, phenanthrene, tetracene etc.. Further encompassed are biphenyl and oligo- or polyphenyls, such as terphenyl, phenyl-substituted biphenyl, phenyl-substituted terphenyl (such as tetraphenyl benzene groups) etc.. "Arylene" respectively "heteroarylene", refers to groups to which two further moieties are attached. In the present specification, the term "aryl group" or "arylene group" may refer to a group comprising at least one hydrocarbon aromatic moiety, and all the elements of the hydrocarbon aromatic moiety may have p-orbitals which form conjugation, for example a phenyl group, a napthyl group, an anthracenyl group, a phenanthrenyl group, a pyrenyl group, a fluorenyl group and the like. Further encompoassed are spiro compounds in which two aromatic moieties are connected with each other via a spiro-atom, such as 9,9'-spirobi[9H-fluorene]yl. The aryl or arylene group may include a monocyclic or fused ring polycyclic (i.e., links sharing adjacent pairs of carbon atoms) functional group.

The term "heteroaryl" as used herein refers to aryl groups in which at least one carbon atom is substituted with

a heteroatom. The term "heteroaryl" may refer to aromatic heterocycles with at least one heteroatom, and all the elements of the hydrocarbon heteroaromatic moiety may have p-orbitals which form conjugation. The heteroatom may be selected from N, O, S, B, Si, P, Se, preferably from N, O and S. A heteroarylene ring may comprise at least 1 to 3 heteroatoms. Preferably, a heteroarylene ring may comprise at least 1 to 3 heteroatoms individually selected from N, 5 and/or O. Just as in case of "aryl"/"arylene", the term "heteroaryl" comprises, for example, spiro compounds in which two aromatic moieties are connected with each other, such as spiro[fluorene-9,9'-xanthene]. Further exemplary heteroaryl groups are diazine, triazine, dibenzofurane, dibenzothiofurane, acridine, benzoacridine, dibenzoacridine etc.

The term "alkenyl" as used herein refers to a group $-CR^1 = CR^2R^3$ comprising a carbon-carbon double bond.

The term "perhalogenated" as used herein refers to a hydrocarbyl group wherein all of the hydrogen atoms of the hydrocarbyl group are replaced by halogen (F, Cl, Br, I) atoms.

The term "alkoxy" as used herein refers to a structural fragment of the Formula -OR with R being hydrocarbyl, preferably alkyl or cycloalkyl.

The term "thioalkyl" as used herein refers to a structural fragment of the Formula -SR with R being hydrocarbyl, preferably alkyl or cycloalkyl.

The subscripted number n in $C_n$-heteroaryl merely refers to the number of carbon atoms excluding the number of heteroatoms. In this context, it is clear that a $C_3$ heteroarylene group is an aromatic compound comprising three carbon atoms, such as pyrazol, imidazole, oxazole, thiazole and the like.

The term "heteroaryl" as used herewith shall encompass pyridine, quinoline, benzoquinoline, quinazoline, benzoquinazoline, pyrimidine, pyrazine, triazine, benzimidazole, benzothiazole, benzo[4,5]thieno[3,2-d]pyrimidine, carbazole, xanthene, phenoxazine, benzoacridine, dibenzoacridine and the like.

In the present specification, the term single bond refers to a direct bond.

The term "fluorinated" as used herein refers to a hydrocarbon group in which at least one of the hydrogen atoms comprised in the hydrocarbon group is substituted by a fluorine atom. Fluorinated groups in which all of the hydrogen atoms thereof are substituted by fluorine atoms are referred to as perfluorinated groups and are particularly addressed by the term "fluorinated".

In terms of the invention, a group is "substituted with" another group if one of the hydrogen atoms comprised in this group is replaced by another group, wherein the other group is the substituent.

In accordance with the present disclosure, as an illustrative example using an arbitrary group A in a formula showing the following binding situation,

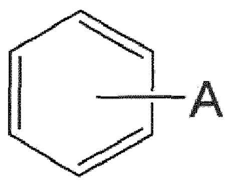

the group A may be bound to any suitable binding position. In a situation where it is shown that the bond of A crosses more than one ring

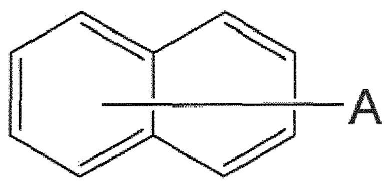

the group A may be bound to any suitable binding position of each ring crossed with the bond.

In terms of the invention, the expression "between" with respect to one layer being between two other layers does not exclude the presence of further layers which may be arranged between the one layer and one of the two other layers. In terms of the invention, the expression "in direct contact" with respect to two layers being in direct contact with each other means that no further layer is arranged between those two layers. One layer deposited on the top of another layer is deemed to be in direct contact with this layer.

The term "contacting sandwiched" refers to an arrangement of three layers whereby the layer in the middle is in direct contact with the two adjacent layers.

With respect to the inventive electron transport layer stack the compounds mentioned in the experimental part are most preferred.

A lighting device may be any of the devices used for illumination, irradiation, signaling, or projection. They are

correspondingly classified as illuminating, irradiating, signaling, and projecting devices. A lighting device usually consists of a source of optical radiation, a device that transmits the radiant flux into space in the desired direction, and a housing that joins the parts into a single device and protects the radiation source and light-transmitting system against damage and the effects of the surroundings.

According to another aspect, the organic electroluminescent device according to the present invention comprises two or three or more emission layers. An OLED comprising more than one emission layer is also described as a tandem OLED or stacked OLED.

The organic electroluminescent device (OLED) may be a bottom- or top-emission device. The organic electroluminescent device (OLED) may emit the light through a transparent anode or through a transparent cathode.

Another aspect is directed to a device comprising at least one organic electroluminescent device (OLED).

A device comprising organic light-emitting diodes is for example a display or a lighting panel.

In the present invention, the following defined terms, these definitions shall be applied, unless a different definition is given in the claims or elsewhere in this specification.

In the context of the present specification the term "different" or "differs" in connection with the matrix material means that the matrix material differs in their structural Formula.

The terms "OLED" and "organic light-emitting diode" are used simultaneously and have the same meaning. The term "organic electroluminescent device" as used herein may comprise both organic light emitting diodes as well as organic light-emitting transistors (OLETs).

As used herein, "weight percent", "wt.-%", "percent by weight", "% by weight", and variations thereof refer to a composition, component, substance or agent as the weight of that component, substance or agent of the respective electron transport layer divided by the total weight of the respective electron transport layer thereof and multiplied by 100. It is under-stood that the total weight percent amount of all components, substances and agents of the respective electron transport layer and electron injection layer are selected such that it does not exceed 100 wt.-%.

As used herein, "volume percent", "vol.-%", "percent by volume", "% by volume", and variations thereof refer to a composition, component, substance or agent as the volume of that component, substance or agent of the respective electron transport layer divided by the total volume of the respective electron transport layer thereof and multiplied by 100. It is understood that the total volume percent amount of all components, substances and agents of the cathode layer are selected such that it does not exceed 100 vol.-%.

All numeric values are herein assumed to be modified by the term "about", whether or not explicitly indicated. As used herein, the term "about" refers to variation in the numerical quantity that can occur. Whether or not modified by the term "about" the claims include equivalents to the quantities.

It should be noted that, as used in this specification and the appended claims, the singular forms "a", "an", and "the" include plural referents unless the content clearly dictates otherwise.

The term "free of", "does not contain", "does not comprise" does not exclude impurities. Impurities have no technical effect with respect to the object achieved by the present invention.

In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

Preferably, the organic semiconducting layer comprising the compound of Formula (II) is essentially non-emissive or non-emitting.

The operating voltage, also named U, is measured in Volt (V) at 10 milliampere per square centimeter (mA/cm2).

The candela per ampere efficiency, also named cd/A efficiency or Ceff, is measured in candela per ampere at 10 milliampere per square centimeter (mA/cm2).

The external quantum efficiency, also named EQE, is measured in percent (%).

The color space is described by coordinates CIE-x and CIE-y (International Commission on Illumination 1931). For blue emission the CIE-y is of particular importance. A smaller CIE-y denotes a deeper blue color. Efficiency values are compared at the same CIE-y.

The highest occupied molecular orbital, also named HOMO, and lowest unoccupied molecular orbital, also named LUMO, are measured in electron volt (eV).

The term "OLED", "organic light emitting diode", "organic light emitting device", "organic optoelectronic device" and "organic light-emitting diode" are simultaneously used and have the same meaning.

The term "life-span" and "lifetime" are simultaneously used and have the same meaning.

The anode and cathode may be described as anode electrode / cathode electrode or anode electrode / cathode electrode or anode electrode layer / cathode electrode layer.

Room temperature, also named ambient temperature, is 23° C.

Hereinafter, the embodiments are illustrated in more detail with reference to examples. However, the present disclosure is not limited to the following examples. Reference will now be made in detail to the exemplary aspects.

DESCRIPTION OF THE DRAWINGS

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures, which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed

FIG. 1 is a schematic sectional view of an organic electronic device, according to an exemplary embodiment of the present invention;

FIG. 2 is a schematic sectional view of an organic light-emitting diode (OLED), according to an exemplary embodiment of the present invention;

FIG. 3 is a schematic sectional view of an OLED, according to an exemplary embodiment of the present invention.

FIG. 4 is a schematic sectional view of an OLED comprising a charge generation layer and two emission layers, according to an exemplary embodiment of the present invention.

Hereinafter, the figures are illustrated in more detail with reference to examples. However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

FIG. 1 is a schematic sectional view of an organic electronic device 100, according to an exemplary embodiment of the present invention. The organic electronic device 100 includes a substrate 110, an anode 120, an emission layer (EML) 125, a second electron transport layer comprising the compound of formula (II) 160. The second electron transport layer comprising the compound of formula (II) 160 is formed on the EML 125. Onto the electron transport layer comprising the compound of formula (II) 160, a cathode 190 is disposed.

FIG. 2 is a schematic sectional view of an organic light-emitting diode (OLED) 100, according to an exemplary embodiment of the present invention. The OLED 100 includes a substrate 110, an anode 120, a hole injection layer (HIL) 130, a hole transport layer (HTL) 140, an emission layer (EML) 150, a second electron transport layer (ETL) 160. The second electron transport layer (ETL) 160 is formed on the EML 150. Onto the second electron transport layer (ETL) 160, an electron injection layer (EIL) 180 is disposed. The cathode 190 is disposed directly onto the electron injection layer (EIL) 180.

Fig. 3 is a schematic sectional view of an OLED 100, according to another exemplary embodiment of the present invention. Fig. 3 differs from Fig. 2 in that the OLED 100 of Fig. 3 comprises an electron blocking layer (EBL) 145 and a first electron transport layer 155.

Referring to Fig. 3, the OLED 100 includes a substrate 110, an anode 120, a hole injection layer (HIL) 130, a hole transport layer (HTL) 140, an electron blocking layer (EBL) 145, an emission layer (EML) 150, a first electron transport layer 155, a second electron transport layer comprising the compound of formula (II) 160, an electron injection layer (EIL) 180 and a cathode electrode 190.

Fig. 4 is a schematic sectional view of an OLED 100, according to another exemplary embodiment of the present invention. Fig. 4 differs from Fig. 3 in that the OLED 100 of Fig. 4 further comprises a charge generation layer (CGL) and a second emission layer (151).

Referring to Fig. 4, the OLED 100 includes a substrate 110, an anode 120, a first hole injection layer (HIL) 130, a first hole transport layer (HTL) 140, a first electron blocking layer (EBL) 145, a first emission layer (EML) 150, a first electron transport layer (HBL) 155, a second electron transport layer comprising the compound of formula (II) 160, an n-type charge generation layer (n-type CGL) 185, a hole generating layer (p-type charge generation layer; p-type GCL) 135, a second hole transport layer (HTL) 141, a second electron blocking layer (EBL) 146, a second emission layer (EML) 151, a hole blocking layer (HBL) 156, a further electron transport layer 161, a second electron injection layer (EIL) 181 and a cathode 190.

While not shown in Fig. 1, Fig. 2, Fig. 3 and Fig. 4, a sealing layer may further be formed on the cathode electrodes 190, in order to seal the OLEDs 100. In addition, various other modifications may be applied thereto.
Hereinafter, the embodiments are illustrated in more detail with reference to examples. However, the present disclosure is not limited to the following examples.

EXPERIMENTAL PART

Melting point

The melting point (mp) is determined as peak temperatures from the DSC curves of the above TGA-DSC measurement or from separate DSC measurements (Mettler Toledo DSC822e, heating of samples from room temperature to completeness of melting with heating rate 10 K/min under a stream of pure nitrogen. Sample amounts of 4 to 6 mg are placed in a 40 μL Mettler Toledo aluminum pan with lid, a <1 mm hole is pierced into the lid).

Glass transition temperature

The glass transition temperature (Tg) is measured under nitrogen and using a heating rate of 10 K per min in a Mettler Toledo DSC 822e differential scanning calorimeter as described in DIN EN ISO 11357, published in March 2010.

Rate onset temperature

The rate onset temperature ($T_{RO}$) is determined by loading 100 mg compound into a VTE source. As VTE source a point source for organic materials may be used as supplied by Kurt J. Lesker Company (www.lesker.com) or CreaPhys GmbH (http://www.creaphys.com). The VTE source is heated at a constant rate of 15 K/min at a pressure of less than $10^{-5}$ mbar and the temperature inside the source measured with a thermocouple. Evaporation of the compound is detected with a QCM detector which detects deposition of the compound on the quartz crystal of the detector. The deposition rate on the quartz crystal is measured in Angstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.
To achieve good control over the evaporation rate of an organic compound, the rate onset temperature may be in the range of 200 to 255 °C. If the rate onset temperature is below 200 °C the evaporation may be too rapid and therefore difficult to control. If the rate onset temperature is above 255 °C the evaporation rate may be too low which may result in low tact time and decomposition of the organic compound in VTE source may occur due to prolonged exposure to elevated temperatures.
The rate onset temperature is an indirect measure of the volatility of a compound. The higher the rate onset temperature the lower is the volatility of a compound.

Reduction potential

The reduction potential is determined by cyclic voltammetry with potenioststic device Metrohm $PGSTAT_{30}$ and software Metrohm Autolab GPES at room temperature. The redox potentials given at particular compounds were measured in an argon de-aerated, dry 0.1M THF solution of the tested substance, under argon atmosphere, with 0.1M tetrabutylammonium hexafluorophosphate supporting electrolyte, between platinum working electrodes and with an Ag/AgCl pseudo-standard electrode (Metrohm Silver rod electrode), consisting of a silver wire covered by silver chloride and immersed directly in the measured solution, with the scan rate 100 mV/s. The first run was done in the broadest range of the potential set on the working electrodes, and the range was then adjusted within subsequent runs appropriately. The final three runs were done with the addition of ferrocene (in 0.1M concentration) as the standard. The average of potentials corresponding to cathodic and anodic peak of the studied compound, after subtraction of the average of cathodic and anodic potentials observed for the standard Fc+/Fc redox couple, afforded finally the values reported above. All studied compounds as well as the reported comparative compounds showed well-defined reversible electrochemical behaviour.

Dipole moment

The dipole moment $|\vec{\mu}|$ of a molecule containing N atoms is given by:

$$\vec{\mu} = \sum_{i}^{N} q_i \vec{r_i}$$

$$|\vec{\mu}| = \sqrt{\mu_x^2 + \mu_y^2 + \mu_z^2}$$

where $q_i$ and $\vec{r_i}$ are the partial charge and position of atom i in the molecule.

The dipole moment is determined by a semi-empirical molecular orbital method.

The geometries of the molecular structures are optimized using the hybrid functional B3LYP with the 6-31G* basis set in the gas phase as implemented in the program package TURBOMOLE V6.5 (TURBOMOLE GmbH, Litzenhardtstrasse 19, 76135 Karlsruhe, Germany). If more than one conformation is viable, the conformation with the lowest total energy is selected to determine the bond lengths of the molecules.

Calculated HOMO, LUMO, S1 (singlet) and T1 (triplett) energy levels

The HOMO, LUMO, S1 and T1 are calculated with the program package ORCA V5.0.3 (Max Planck Institute für Kohlenforschung, Kaiser Wilhelzn Platz 1, 45470, Muelheim/Ruhr, Germany) and WEASEL 1.9.2 (FAccTs GmbH, Rolandstrasse 67, 50677 Köln, Germany). The HOMO, LUMO, S1 and T1 energy levels of the molecular structures are determined by applying the hybrid functional B3LYP with a 6-31G* basis set from the optimized geometries obtained by applying the functional B3LYP with a 6-31G* basis set. All the calculations were performed in the gas phase. If more than one conformation is viable, the conformation with the lowest total energy is selected.

Table 4:

| Material | Tg / °C | Tm / °C | Rate Onset Temperature / °C |
|---|---|---|---|
| B-1 | 109 | 295 | 234 |
| B-4 | 86 | 272 | 226 |

Table 5.

| Structure | HOMO / eV | LUMO / eV | E_gap/ eV | S1 / eV | T1 / eV |
|---|---|---|---|---|---|
| 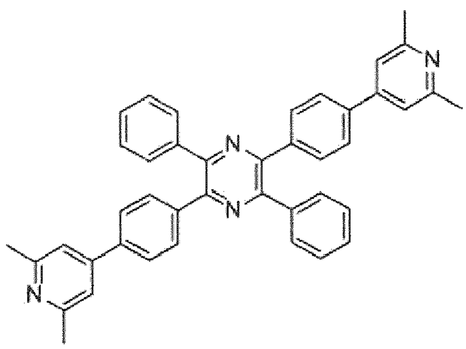 | -5,68 | -1,91 | 3,77 | 3,42 | 2,63 |

(continued)

| Structure | HOMO / eV | LUMO / eV | E_gap/ eV | S1 / eV | T1 / eV |
|---|---|---|---|---|---|
| 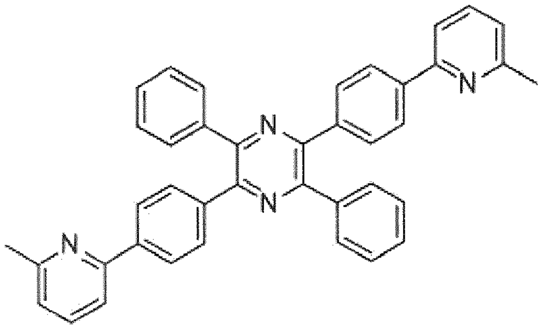 | -5,42 | -1,76 | 3,66 | 3,32 | 2,56 |
| 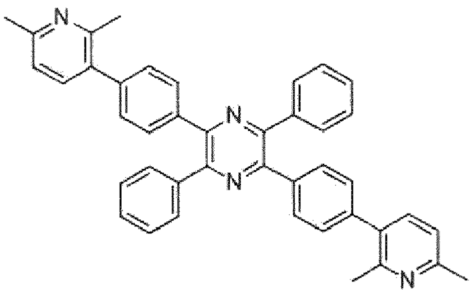 | -5,57 | -1,75 | 3,82 | 3,47 | 2,66 |
| 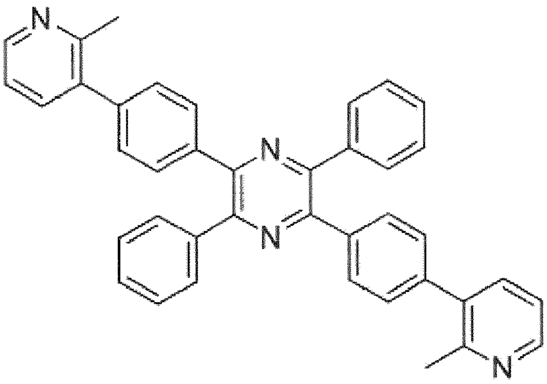 | -5,66 | -1,8 | 3,86 | 3,50 | 2,69 |
| 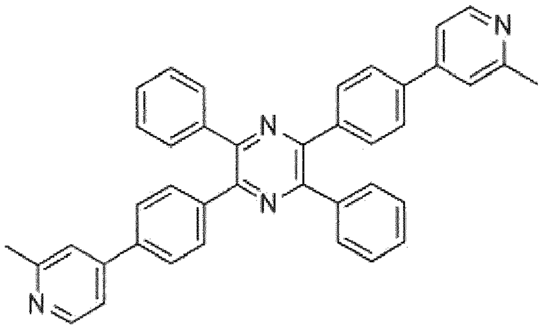 | -5,73 | -1,95 | 3,77 | 3,42 | 2,64 |

(continued)

| Structure | HOMO / eV | LUMO / eV | E_gap/ eV | S1 / eV | T1 / eV |
|---|---|---|---|---|---|
| 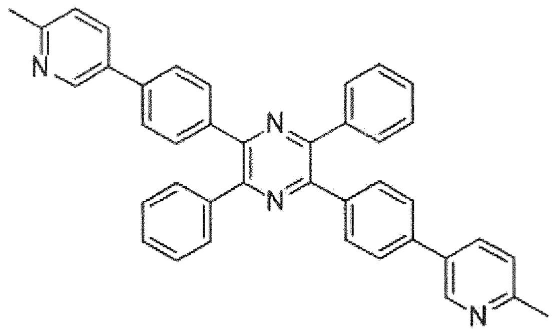 | -5,58 | -1,81 | 3,76 | 3,41 | 2,64 |
| 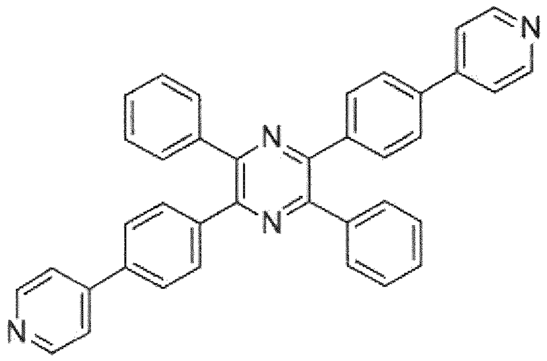 | -5,79 | -2,01 | 3,77 | 3,42 | 2,65 |
| 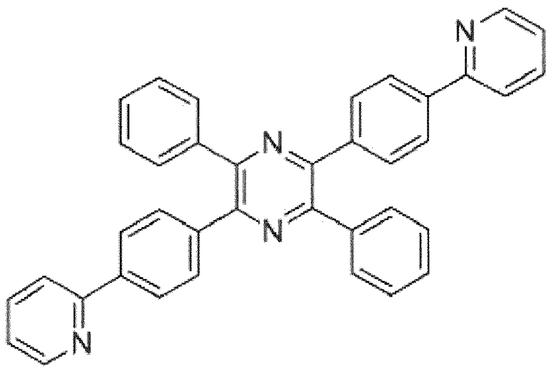 | -5,48 | -1,8 | 3,68 | 3,34 | 2,58 |
| 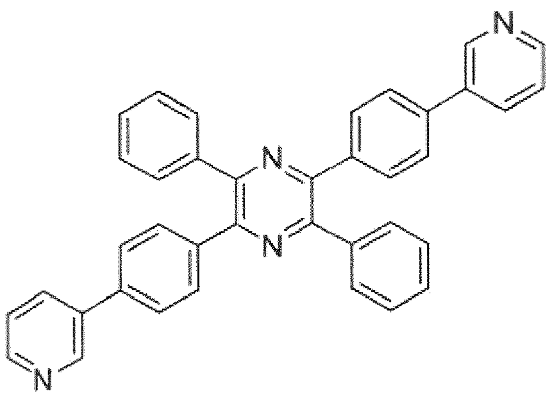 | -5,66 | -1,88 | 3,78 | 3,43 | 2,64 |

(continued)

| Structure | HOMO / eV | LUMO / eV | E_gap/ eV | S1 / eV | T1 / eV |
|---|---|---|---|---|---|
| 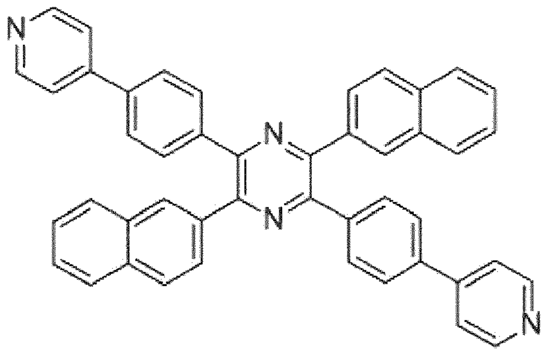 | -5,62 | -2,05 | 3,57 | 3,21 | 2,51 |
| 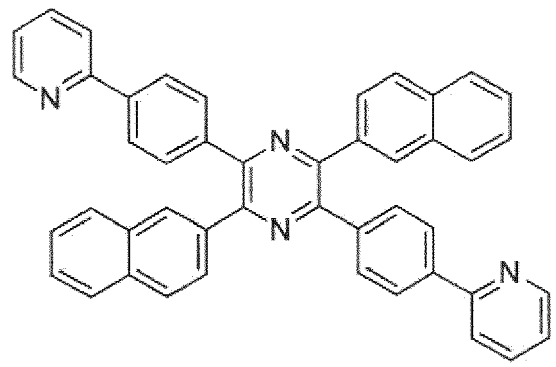 | -5,38 | -1,84 | 3,54 | 3,19 | 2,49 |
| 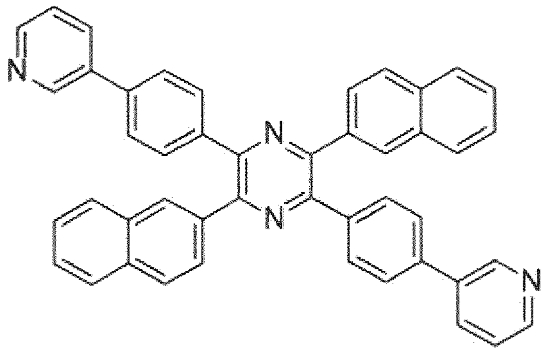 | -5,51 | -1,96 | 3,55 | 3,19 | 2,47 |
| 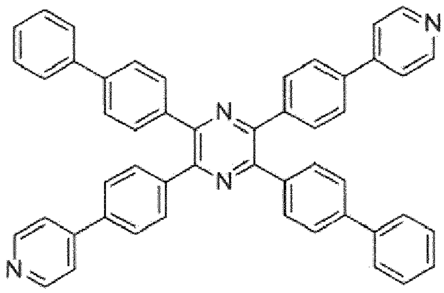 | -5,63 | -2,03 | 3,59 | 3,25 | 2,56 |

(continued)

| Structure | HOMO / eV | LUMO / eV | E_gap/ eV | S1 / eV | T1 / eV |
|---|---|---|---|---|---|
| 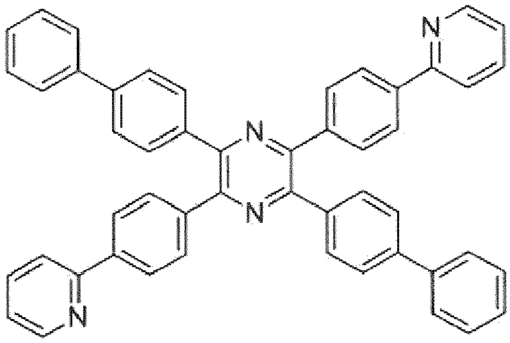 | -5,37 | -1,86 | 3,52 | 3,19 | 2,49 |
| 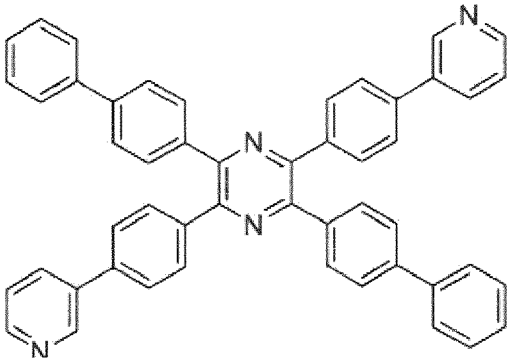 | -5,54 | -1,92 | 3,63 | 3,29 | 2,57 |
| 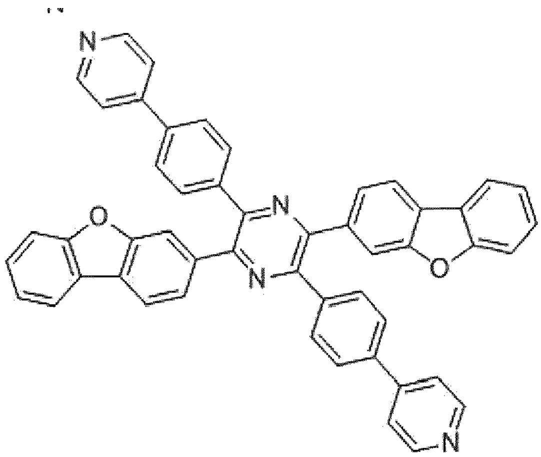 | -5,64 | -2,09 | 3,55 | 3,21 | 2,54 |
| 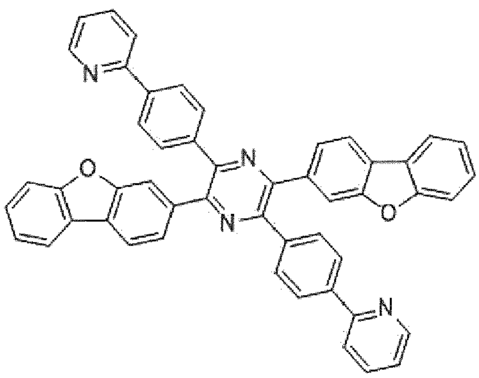 | -5,38 | -1,89 | 3,49 | 3,17 | 2,48 |

(continued)

| Structure | HOMO / eV | LUMO / eV | E_gap/ eV | S1 / eV | T1 / eV |
|---|---|---|---|---|---|
| 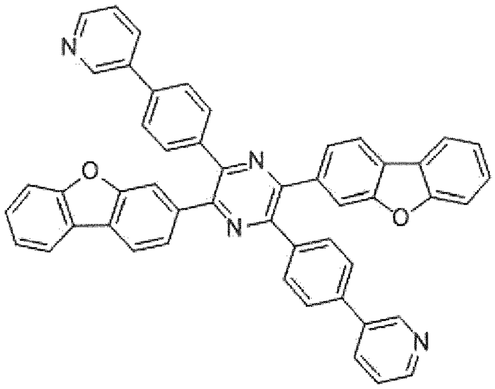 | -5,53 | -1,98 | 3,55 | 3,22 | 2,52 |
| 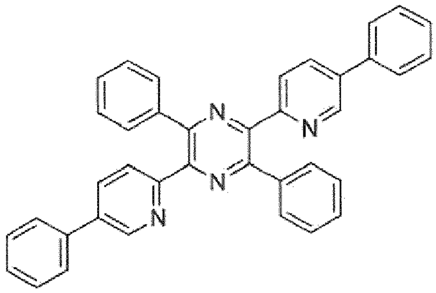 | -5,58 | -1,9 | 3,68 | 3,31 | 2,59 |
| 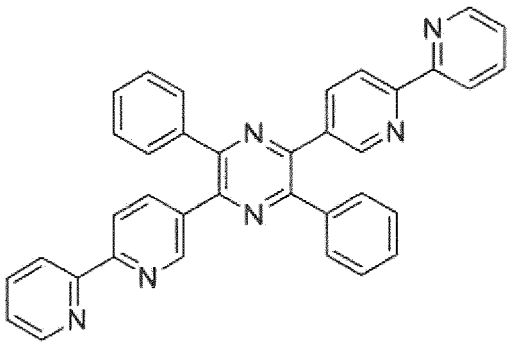 | -5,71 | -2,04 | 3,67 | 3,31 | 2,59 |
| 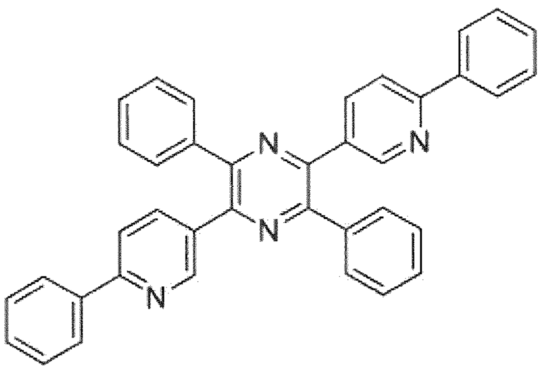 | -5,62 | -1,97 | 3,65 | 3,30 | 2,57 |

(continued)

| Structure | HOMO / eV | LUMO / eV | E_gap/ eV | S1 / eV | T1 / eV |
|---|---|---|---|---|---|
| 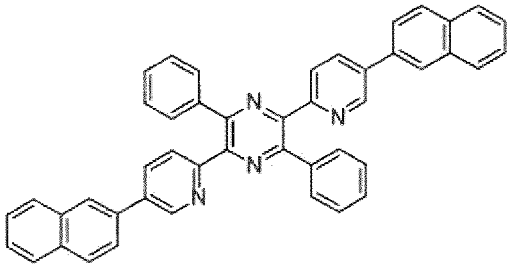 | -5,52 | -1,94 | 3,58 | 3,21 | 2,53 |
| 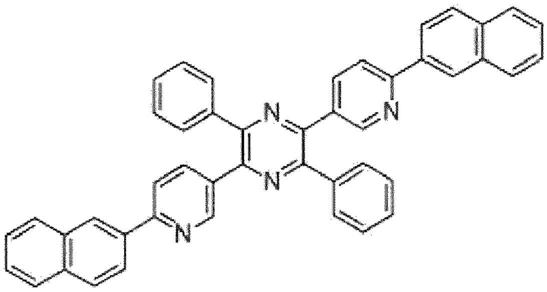 | -5,48 | -2,01 | 3,47 | 3,13 | 2,48 |
| 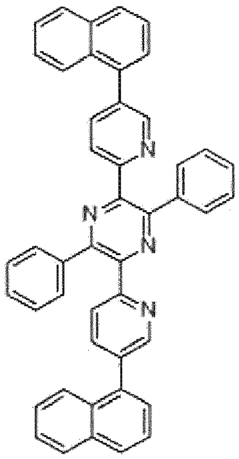 | -5,54 | -1,9 | 3,63 | 3,24 | 2,59 |
| 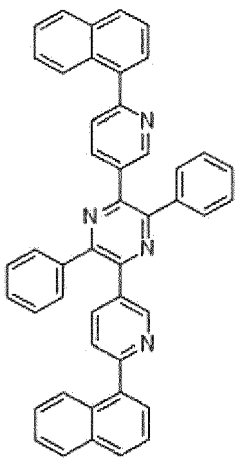 | -5,52 | -1,97 | 3,55 | 3,18 | 2,56 |

(continued)

| Structure | HOMO / eV | LUMO / eV | E_gap/ eV | S1 / eV | T1 / eV |
|---|---|---|---|---|---|
| 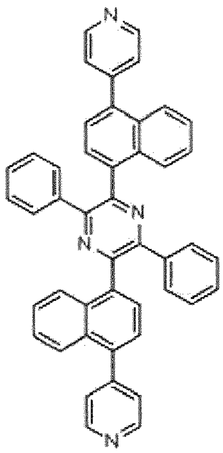 | -5,65 | -1,89 | 3,76 | 3,35 | 2,61 |
| 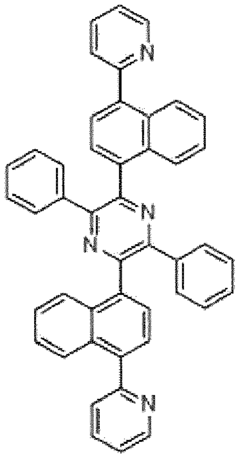 | -5,46 | -1,58 | 3,88 | 3,43 | 2,66 |
| 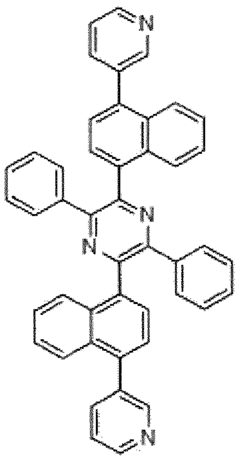 | -5,54 | -1,79 | 3,74 | 3,34 | 2,60 |

(continued)

| Structure | HOMO / eV | LUMO / eV | E_gap/ eV | S1 / eV | T1 / eV |
|---|---|---|---|---|---|
| 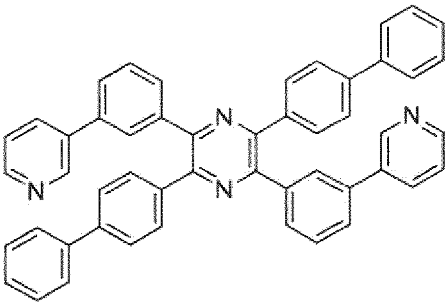 | -5,58 | -1,81 | 3,77 | 3,42 | 2,64 |
| 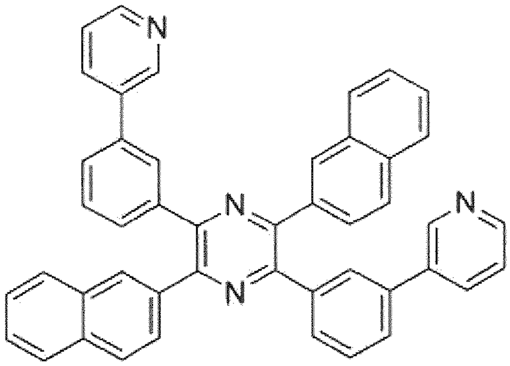 | -5,73 | -1,78 | 3,94 | 3,56 | 2,73 |
| 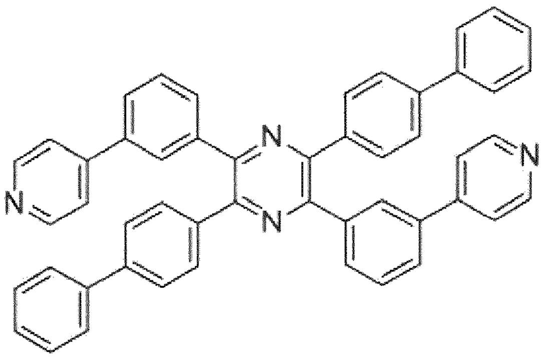 | -5,69 | -1,92 | 3,77 | 3,43 | 2,65 |
| 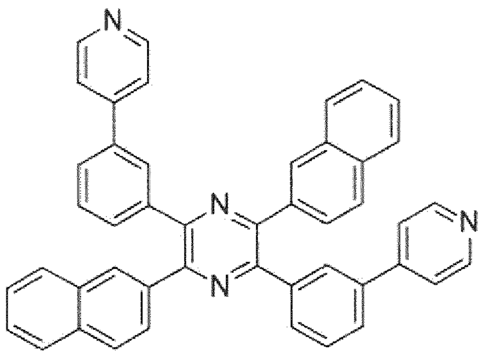 | -5,66 | -1,91 | 3,75 | 3,42 | 2,58 |

(continued)

| Structure | HOMO / eV | LUMO / eV | E_gap/ eV | S1 / eV | T1 / eV |
|---|---|---|---|---|---|
| 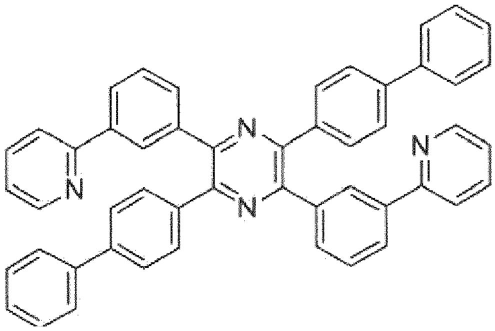 | -5,5 | -1,76 | 3,75 | 3,39 | 2,62 |
| 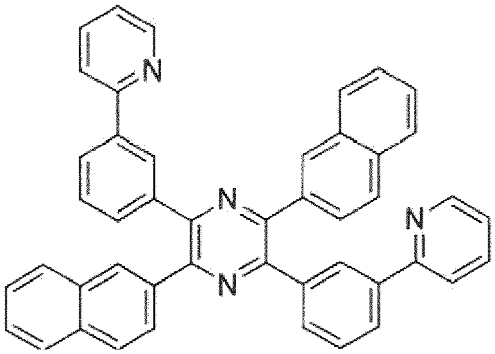 | -5,51 | -1,53 | 3,78 | 3,46 | 2,61 |
| 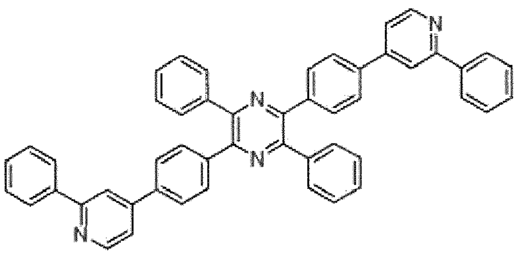 | -5,76 | -1,99 | 3,77 | 3,40 | 2,64 |
| 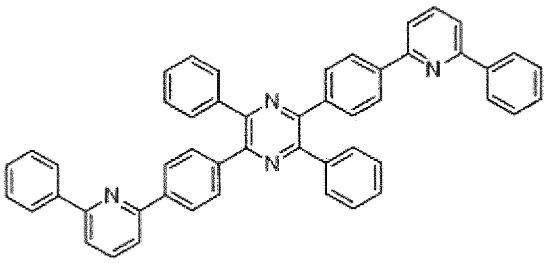 | -5,47 | -1,79 | 3,68 | 3,32 | 2,58 |
| 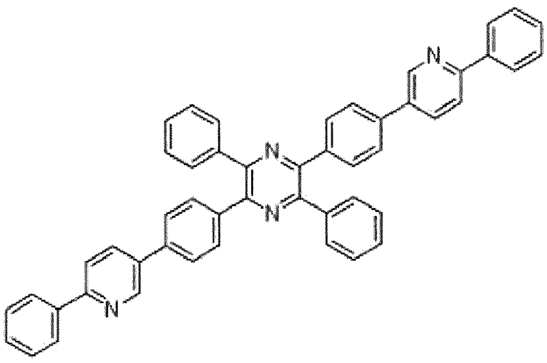 | -5,55 | -1,92 | 3,63 | 3,26 | 2,59 |

(continued)

| Structure | HOMO / eV | LUMO / eV | E_gap/ eV | S1 / eV | T1 / eV |
|---|---|---|---|---|---|
| 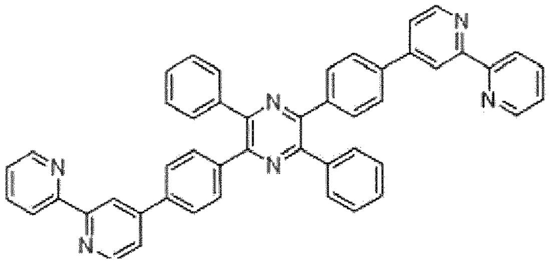 | -5,67 | -1,92 | 3,75 | 3,38 | 2,64 |
| 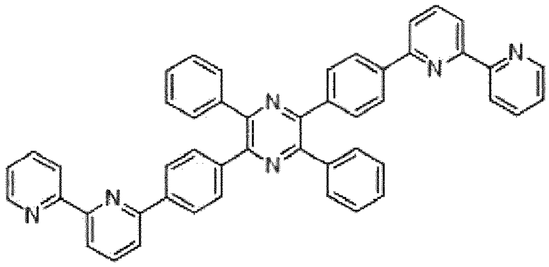 | -5,5 | -1,8 | 3,7 | 3,34 | 2,60 |
| 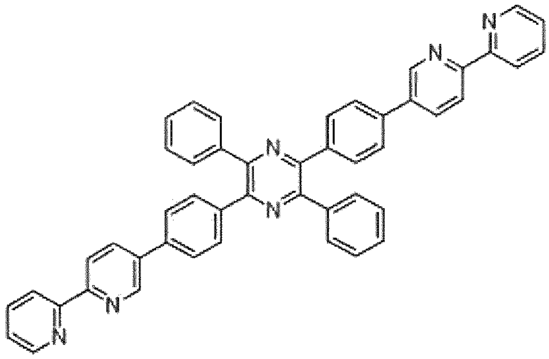 | -5,56 | -1,95 | 3,61 | 3,24 | 2,58 |
| 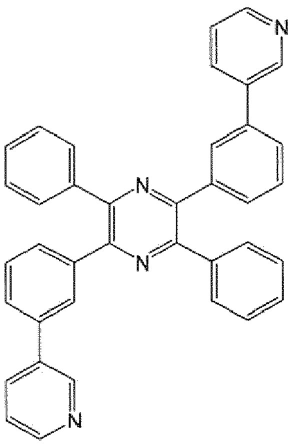 | -5,88 | -1,6 | 4,28 | 3,82 | 2,99 |

(continued)

| Structure | HOMO / eV | LUMO / eV | E_gap/ eV | S1 / eV | T1 / eV |
|---|---|---|---|---|---|
| 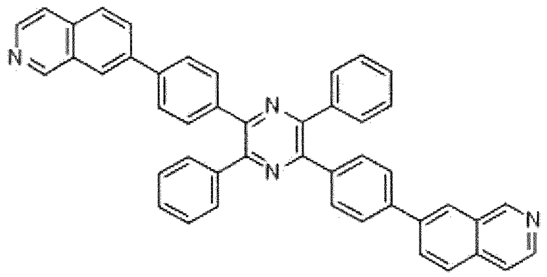 | -5,62 | -1,94 | 3,69 | 3,33 | 2,61 |
| 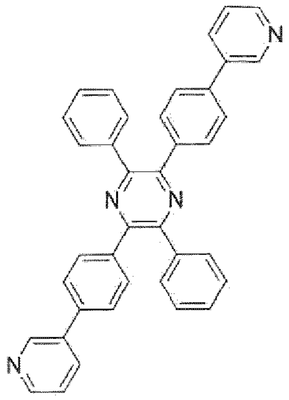 | -5,66 | -1,87 | 3,79 | 3,44 | 2,65 |
| 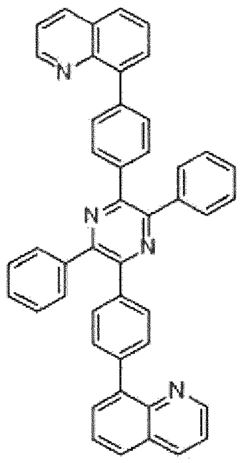 | -5,32 | -1,68 | 3,63 | 3,26 | 2,60 |
| 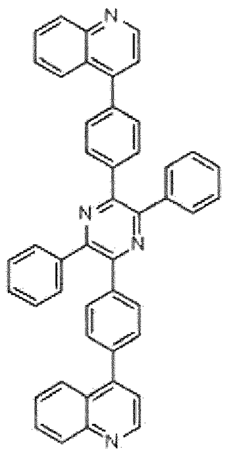 | -5,75 | -1,97 | 3,77 | 3,39 | 2,66 |

(continued)

| Structure | HOMO / eV | LUMO / eV | E_gap/ eV | S1 / eV | T1 / eV |
|---|---|---|---|---|---|
| 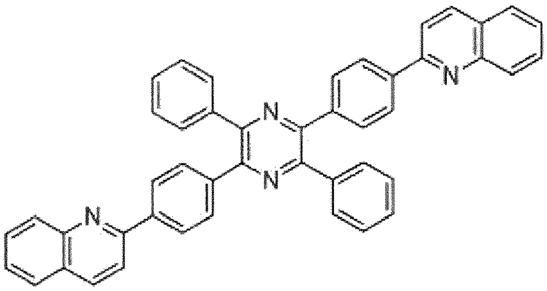 | -5,49 | -1,92 | 3,57 | 3,21 | 2,54 |
| 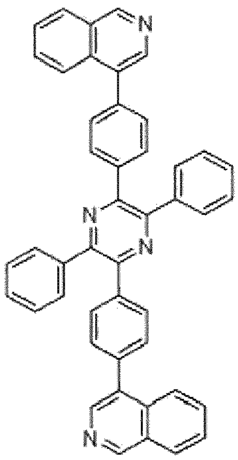 | -5,62 | -1,85 | 3,77 | 3,38 | 2,66 |

## Synthesis Procedures

*Synthesis of compound B-1*

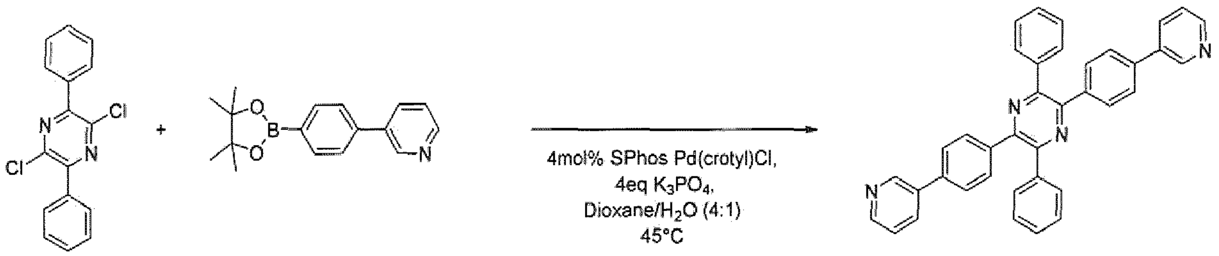


Under Ar-atmosphere 15g (49.8 mmol, 1 eq.) 2,5-dichloro-3,6-diphenylpyrazine [CAS 74134-61-5] were reacted together with 33.6g (119.5 mmol, 2.4 eq.) 3-(4-(4,4,5,5-tetramethyl-1,3,2-dioxaborolan-2-yl)phenyl)pyridine [CAS 929203-04-3], 42.3g (200 mmol, 4 eq.) $K_3PO_4$ and 1.2g (2 mmol, 0.04 eq.) SPhos Pd(crotyl)Cl [CAS 1798781-99-3] in a dioxane:water (4:1) mixture at 45°C for 18h. For purification the reactions crude product was recrystallized two times from chlorbenzene. A total of 15.9g (59% yield) product was obtained.

HPLC 99.8%, ESI-MS m/z 539 [M+H].

Further purification was achieved by high vaccum sublimation.

*Synthesis of compound B-4*

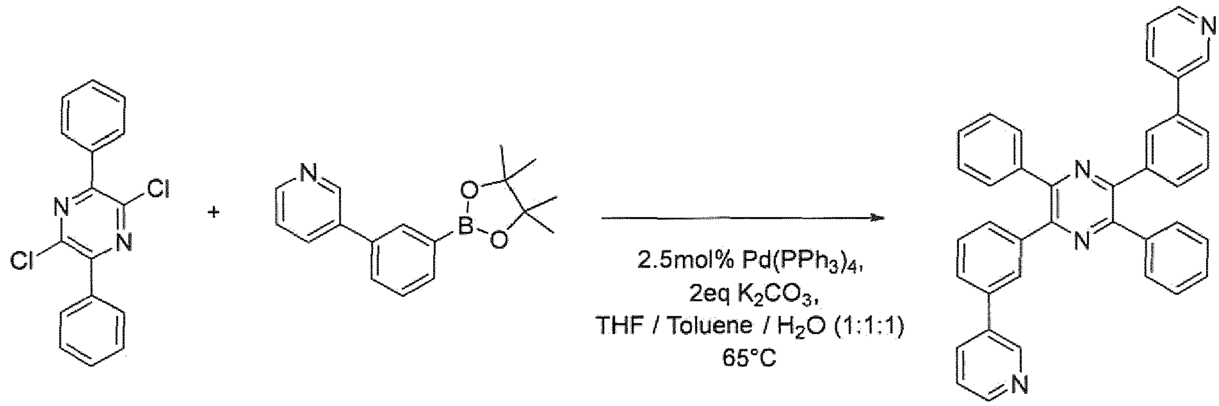


Under Ar-atmosphere 20g (66.4 mmol, 1 eq.) 2,5-dichloro-3,6-diphenylpyrazine [CAS 74134-61-5] were reacted together with 21.5g (76.4 mmol, 1.15 eq.) 3-(3-(4,4,5,5-tetramethyl-1,3,2-dioxaborolan-2-yl)phenyl)pyridine [CAS 939430-30-5], 18.4g (132.8 mmol, 2 eq.) $K_2CO_3$ and 1.9g (1.6 mmol, 0.025 eq.) $Pd(PPh_3)_4$ [CAS 14221-01-3] in a toluene:THF:water (1:1:1) mixture at 65°C for 4 days. After aqueous work-up the crude was filtered through a Florisil® pad using dichloromethane. To the obtained solution hexane was added, the solid filtered off and recrystallized from toluene. The obtained solid was chromatographed on silica gel using dichlormethane and dichlormethane:ethyl acetate mixture. After collecting the fractions 5.3g (19% yield) product were obtained.

HPLC 99.9%, ESI-MS m/z 539 [M+H].

Further purification was achieved by high vaccum sublimation.

## General procedure for fabrication of Top-emission OLEDs

The device was made by depositing a 10 nm hole injection layer of HT-1 doped with D-1 (matrix to dopant ratio of 92:8 vol%) onto an glass substrate provided with the silver anode, followed by an undoped hole transport layer of HT-1. A 5 nm layer electron blocking layer of HT-2 was deposited onto the HTL. Subsequently, a blue fluorescent emitting layer of emitter host H09 (Sun Fine Chemicals) doped with BD200 (Sun Fine Chemicals) (97:3 vol%) was deposited. On the emission layer, a layer made of ET-1 was deposited as a hole blocking layer. Then, the electron transport layer was deposited. onto the HBL. Subsequently, a first electron injection layer made of LiQ was deposited for OLED device type A. Or a first electron injection layer made of ET-2 doped with Li for device type B was deposited. Then, a second electron injection layer of Yb was deposited onto the ETL for both OLED device types A and B, followed by a cathode consisting of a silver-magnesium alloy in a volume ratio 90:10. Finally, a capping layer made of HT-3 was deposited on top of the cathode. All depositions were made by vacuum thermal evaporation.

Table 6: List of compounds used

| | IUPAC name | Reference |
|---|---|---|
| HT-1 | N-([1,1'-biphenyl]-2-yl)-N-(9,9-dimethyl-9H-fluoren-2-yl)-9,9'-spirobi[fluoren]-2-amine CAS 1364603-07-5 | - |
| HT-2 | N-([1,1'-biphenyl]-4-yl)-9,9-diphenyl-N-(4-(triphenylsilyl) phenyl)-9H-fluoren-2-amine [CAS 1613079-70-1] | WO2014088047 |
| D-1 | 4,4',4"-((1E,1'E,1"E)-cyclopropane-1,2,3-triylidenetris (cyanomethanylylidene))tris(2,3,5,6-tetrafluorobenzonitrile) [CAS 1224447-88-4] | US2008265216 |
| HOST-1 | H09 (Fluorescent-blue host material) | Commercially available from Sun Fine Chemicals, Inc, S. Korea |

(continued)

| | IUPAC name | Reference |
|---|---|---|
| EMITTER-i | BD200 (Fluorescent-blue emitter material) | Commercially available from Sun Fine Chemicals, Inc, S. Korea |
| ET-1 | 2,4-diphenyl-6-(4',5',6'-triphenyl-[1,1':2',1":3",1‴:3‴,1⁗-quinquephenyl]-3⁗-yl)-1,3,5-triazine [CAS 2032364-64-8] | WO2016171358 |
| ET-2 | 2,2'-(1,3-Phenylene)bis[9-phenyl-1,10-phenanthroline] [CAS 721969-94-4] | JP2004281390 |
| LiQ | 8-Hydroxyquinolinolato-lithium [CAS 850918-68-2] | WO2013079217 |
| HT-3 | N-([1,1'-biphenyl]-4-yl)-9,9-dimethyl-N-(4-(9-phenyl-9H-carbazol-3-yl)phenyl)-9H-fluoren-2-amine [CAS 1242056-42-3] | US2016322581 |
| ET-3 (comparative compound 1) | 1-(pyridin-2-yl)-3-(4-(3,5,6-triphenylpyrazin-2-yl)phenyl) imidazo[1,5-a]pyridine | WO2021105518 |
| ET-4 (comparative compound 2) | ((3,6-diphenylpyrazine-2,5-diyl)bis(3,1-phenylene))bis (dimethylphosphine oxide) | |
| C-3 | 2-([1,1'-biphenyl]-3-yl)-4-phenyl-6-(3-(10-phenylanthracen-9-yl)phenyl)-1,3,5-triazine [CAS 2437303-42-7] | WO20120794 |

Structure of ET-3 (comparative compound 1):

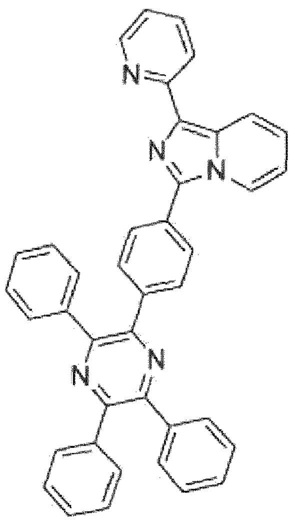

Structure of ET-4 (comparative compound 2):

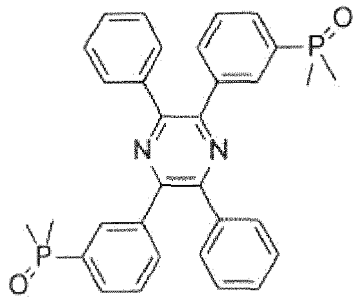

Table 7 schematically describes the OLED device type A with LiQ EIL1.

Table 7:

| Layer | Material | c [vol%] | d [nm] |
|---|---|---|---|
| anode | Ag | 100 | 100 |
| HIL | HT-1:D-1 | 92:8 | 10 |
| HTL | HT-1 | 100 | 130 |
| EBL | HT-2 | 100 | 5 |
| EML | H09:BD200 | 97:3 | 20 |
| HBL | ET-1 | 100 | 5 |
| ETL | Inventive compound or comparative compound optional with compound (III) | 100 | 30 |
| EIL1 | LiQ | 100 | 1 |
| EIL2 | Yb | 100 | 2 |
| cathode | Ag:Mg | 90:10 | 13 |
| Cap layer | HT-3 | 100 | 75 |

Table 8 shows the device performance of the OLED device type A

Table 8:

| ETL | Relative V [%] | Relative CEff/CIEy [%] |
|---|---|---|
| ET-3 | 100 | 100 |
| B-1 | **102** | **114** |
| B-1:C-3 (3:7) | **105** | **124** |
| B-4 | **103** | **129** |
| B-4:C-3 (3:7) | **102** | **128** |

Table 9 schematically describes the OLED device type B with ET-2:Li EIL1.

Table 9:

| Layer | Material | concentration [vol%] | Layer thickness [nm] |
|---|---|---|---|
| anode | A g | 100 | 100 |
| HIL | HT-1:D-1 | 92:8 | 10 |
| HTL | HT-1 | 100 | 130 |
| EBL | HT-2 | 100 | 5 |
| EML | H09:BD200 | 97:3 | 20 |

(continued)

| Layer | Material | concentration [vol%] | Layer thickness [nm] |
|---|---|---|---|
| HBL (1st ETL) | ET-1 | 100 | 5 |
| ETL | Inventive compound or comparative compound optional with compound (III) | 100 | 30 |
| EIL1 | ET-2:Li | 99:1 | 15 |
| EIL2 | Yb | 100 | 2 |
| cathode | Ag:Mg | 90:10 | 13 |
| Cap layer | HT-3 | 100 | 75 |

Table 10 shows the device performance of the OLED device type B.

Table 10:

| ETL | Relative V [%] | Relative CEff/CIEy [%] |
|---|---|---|
| ET-3 | 100 | 100 |
| ET-4:C-3 (3:7) | 112 | 100 |
| B-1 | **104** | **103** |
| B-1 : C-3 (3:7) | **99** | **111** |
| B-4 | **105** | **116** |
| B-4:C-3 (3:7) | **99** | **114** |

Technical Effect of the invention

The OLED devices comprising the inventive compounds, such as the exemplary compounds E1, E20 and E28, show an improved current efficiency at comparable operating voltage.

The features disclosed in the foregoing description and in the dependent claims may, both separately and in any combination thereof, be material for realizing the aspects of the disclosure made in the independent claims, in diverse forms thereof.

## Claims

**1.** Organic light emitting diode comprising a non-transparent substrate, an anode, a cathode, an emission layer, and an electron transport layer stack;
wherein

- the electron transport layer stack is arranged between the emission layer and the cathode;
- the electron transport layer stack comprises optionally a first electron transport layer;
- the electron transport layer stack comprises a second electron transport layer;
- the second electron transport layer is arranged between the emission layer and the cathode;
- the second electron transport layer comprises a compound of formula (II)

$$(Ar^2)_m\text{-}(Z_k\text{-}G)_n \qquad (II)$$

- n is 2 or more;
- m is 1 or 2;
- k is o, 1 or 2;
- $Ar^2$ is independently selected from the group consisting of substituted or unsubstituted $C_2$ to $C_{42}$ heteroaryl and substituted or unsubstituted $C_6$ to $C_{60}$ aryl;

- wherein, if $Ar^2$ is substituted, the one or more substituent(s) are independently selected from the group consisting of D, $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R_{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;
- wherein each $C_6$ to $C_{12}$ aryl substituent on $Ar^2$ and each $C_3$ to $C_{11}$ heteroaryl substituent on X may be substituted with D, $C_1$ to $C_4$ alkyl or halogen;

- Z is independently selected from substituted or unsubstituted $C_6$ to $C_{30}$ aryl and substituted or unsubstituted $C_2$ to $C_{42}$ heteroaryl;

- wherein, if Z is substituted, the one or more substituent(s) are independently selected from the group consisting of D, $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;
- wherein each $C_6$ to $C_{12}$ aryl substituent on Z and each $C_3$ to $C_{11}$ heteroaryl substituent on X may be substituted with D, $C_1$ to $C_4$ alkyl or halogen;

- G comprises at least one 6-membered heteroaromatic ring, wherein the 6-membered heteroaromatic ring comprises one N-atom;
- G is chosen so that the dipole moment of a compound G-phenyl is $\geq$ 1 D and $\leq$ 7 D; and
- the first electron transport layer and the second electron transport layer are free of an electrical dopant.

**2.** Organic light emitting diode according to claim 1, wherein

- $Ar^2$ is independently selected from the group consisting of pyridinyl, triazinyl, pyrimidinyl, pyrazinyl, quinoxalinyl; quinazolinyl, benzoquinazolinyl, benzimidazolyl, quinolinyl, benzoquinolinyl, benzoacridinyl, dibenzoacridinyl, fluoranthenyl, anthracenyl, naphthyl, triphenylenyl, phenathrolinyl, and dinaphthofuranyl, which may be substituted or unsubstituted, respectively;
- if the respective group is substituted, the one or more substituent(s) are independently selected from the group consisting of D, $C_6$ to $C_{24}$ aryl, $C_3$ to $C_{21}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy; and
- each $C_6$ to $C_{24}$ aryl substituent on $Ar_2$ and each $C_3$ to $C_{21}$ heteroaryl substituent on $Ar^2$ may be substituted with D, $C_1$ to $C_4$ alkyl or halogen.

**3.** Organic light emitting diode according to claim 1 or 2, wherein Z is independently selected from the following groups

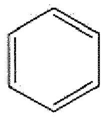 ; 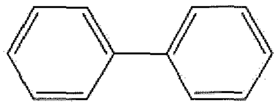 ; 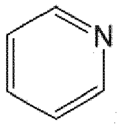 ; 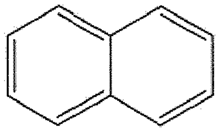 ; 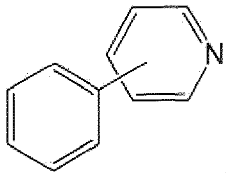 ;

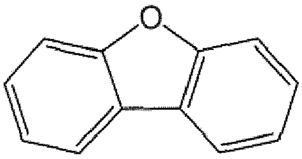 ;

wherein the respective structure may be unsubstituted or substituted with one or more substituent(s) selected from the group consisting of D, $C_1$ to $C_{12}$ alkyl and phenyl.

**4.** Organic light emitting diode according to any of the preceding claims, wherein

- G is selected from the group consisting of

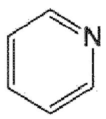 ; 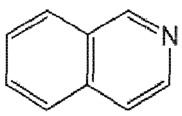 ; and 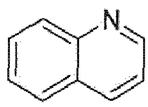 ,

and
- the respective structure may be unsubstituted or substituted with one or more substituents independently selected from the group consisting of D, phenyl, naphthyl, $C_1$ to $C_{12}$ alky, and pyridyl.

**5.** Organic light emitting diode according to any of the preceding claims, wherein the compound of formula (II) has the following structure (IIa)

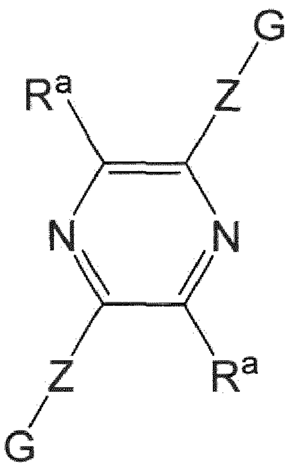

(IIa)

wherein

$R^a$ is independently selected from the group consisting of substituted or unsubstituted $C_6$ to $C_{24}$ aryl and substituted or unsubstituted $C_3$ to $C_{21}$ heteroaryl, wherein if $R^a$ is substituted, the one or more substituents are independently elected from the group consisting of D, phenyl, and $C_1$ to $C_{12}$ alkyl;
Z is independently selected from the group consisting of a single bond and $C_6$ to $C_{24}$ arylene;
G is independently selected from (IIa-1) to (IIa-3)

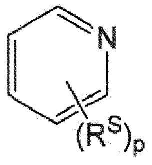 (IIa-1) 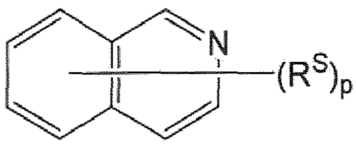 (IIa-2) 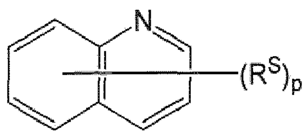 (IIa-3);

p is an integer from o to 4 for IIa-1;
p is an integer from o to 6 for IIa-2 and IIa-3; and
$R^S$ is independently selected from the group consisting of D, $C_1$ to $C_{12}$ aryl, $C_1$ to $C_{12}$ alkyl, and pyridyl, wherein the respective $C_1$ to $C_{12}$ aryl and pyridyl can be unsubstituted or substituted with one or more of D and $C_1$ to $C_4$ alkyl.

**6.** Organic light emitting diode according to any of the preceding claims, wherein the compound of formula (II) is selected from the following structures B-1 to B-44

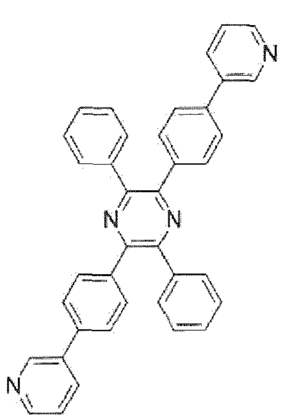 B-1 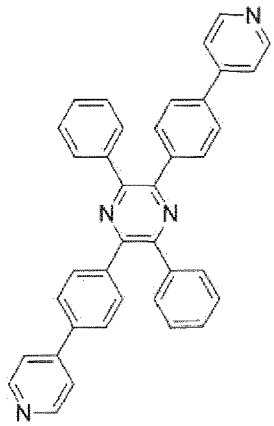 B-2 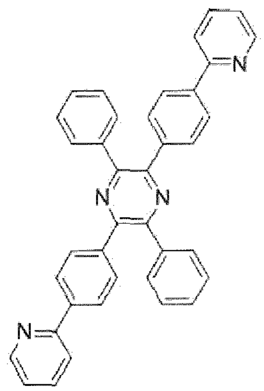 B-3

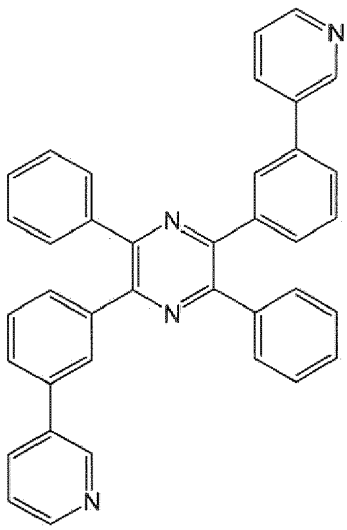

B-4

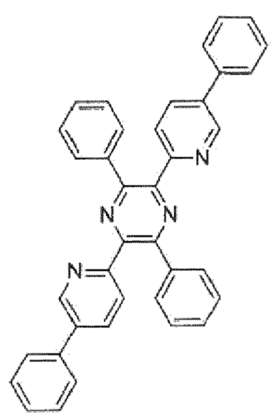
B-5

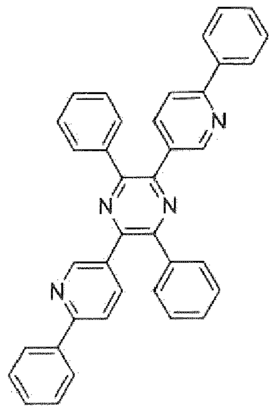
B-6

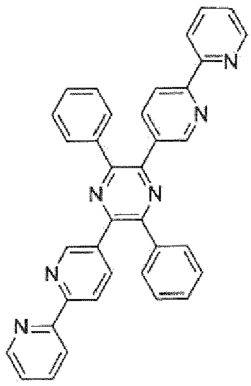
B-7

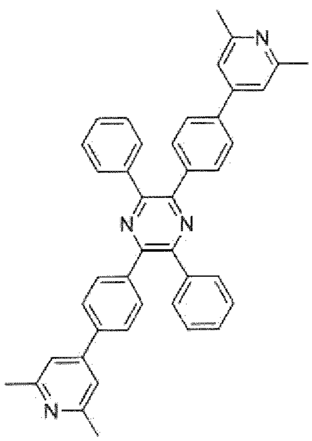
B-8

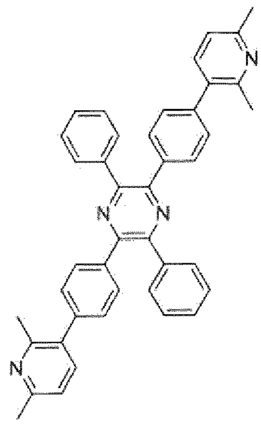
B-9

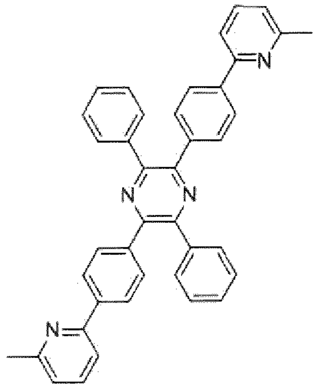
B-10

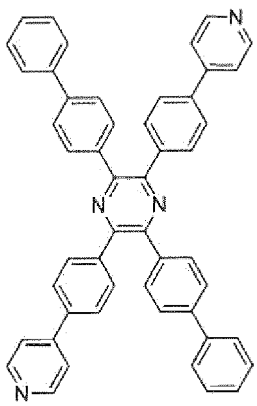
B-11

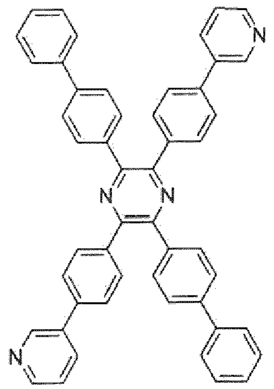
B-12

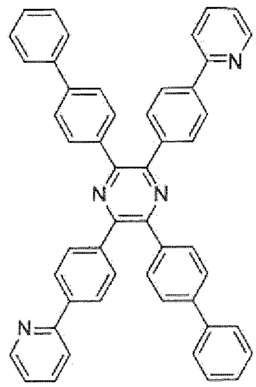
B-13

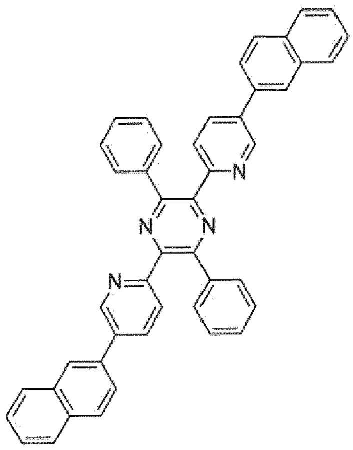
B-14

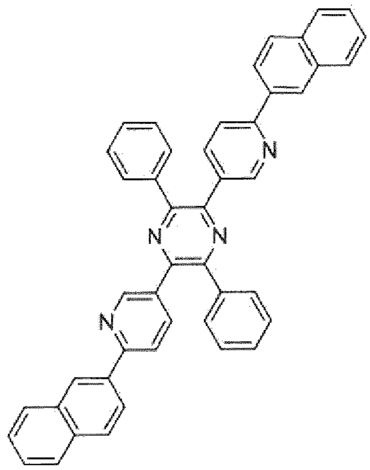
B-15

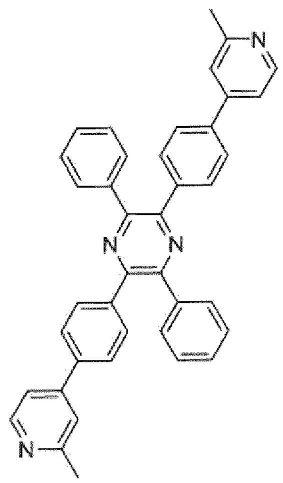
B-16

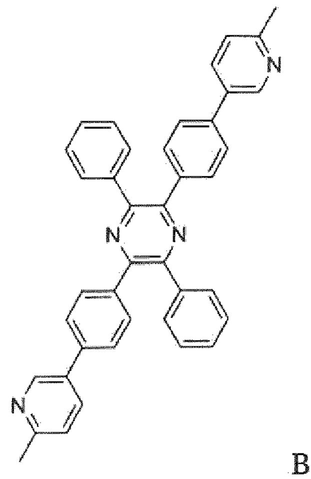
B-17

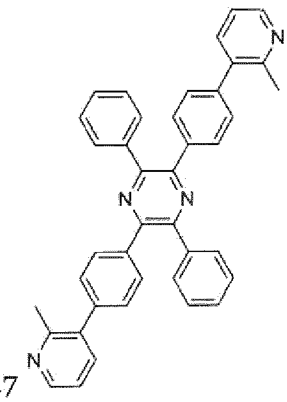
B-18

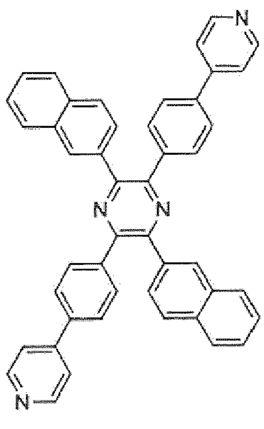
B-19

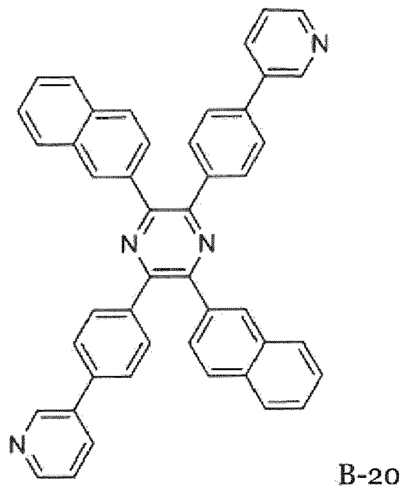
B-20

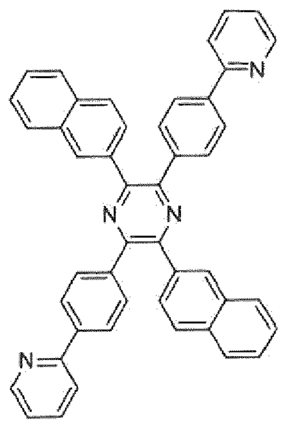
B-21

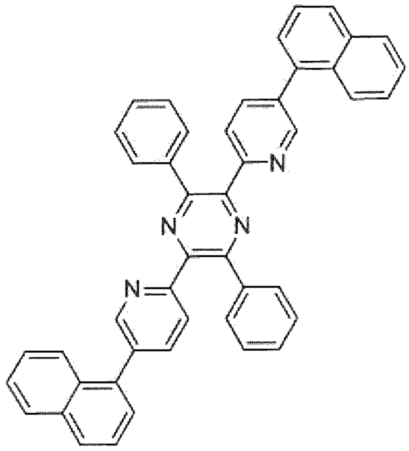

B-22

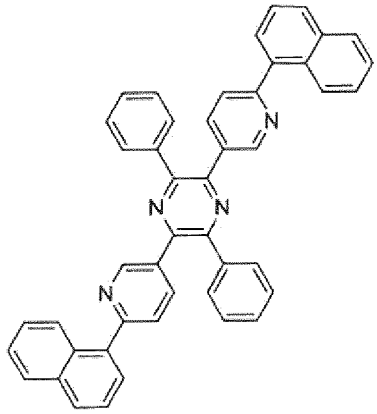

B-23

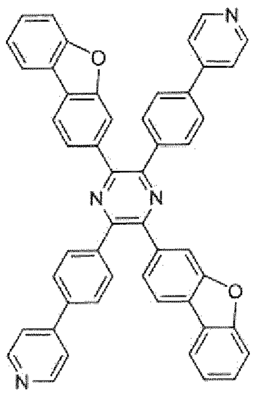

B-24

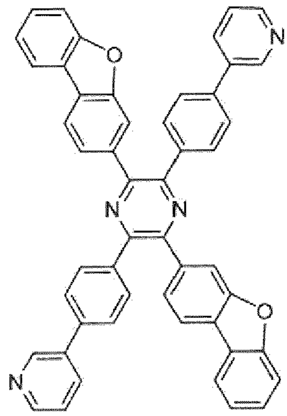

B-25

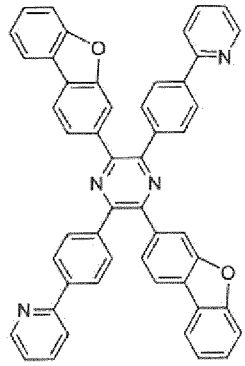

B-26

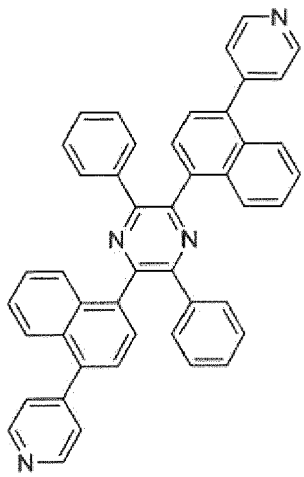

B-27

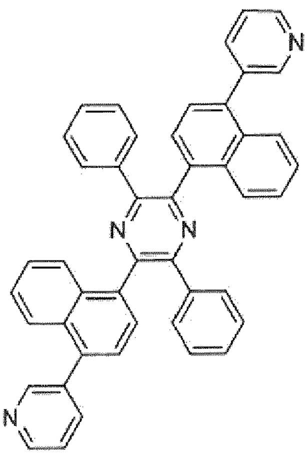

B-28

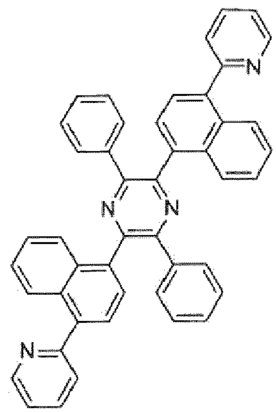

B-29

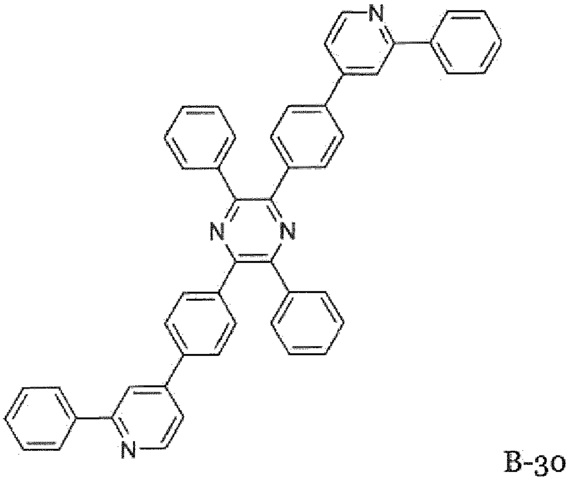

B-30

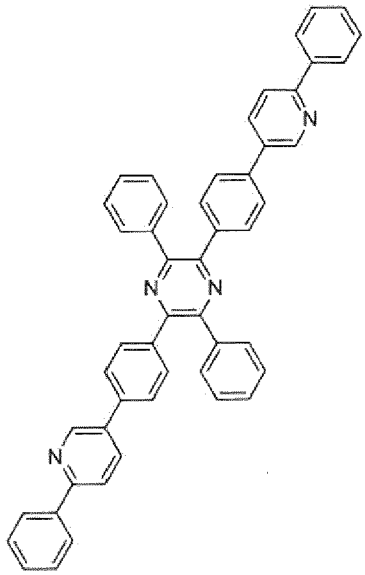

B-31

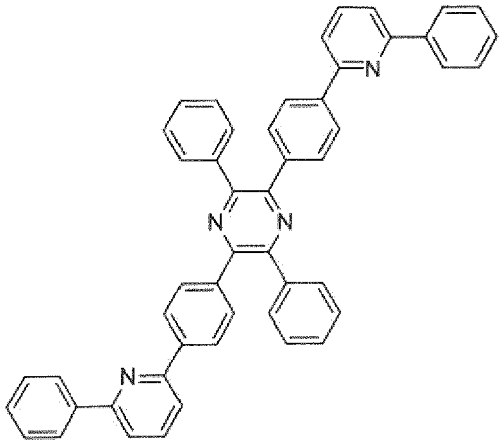

B-32

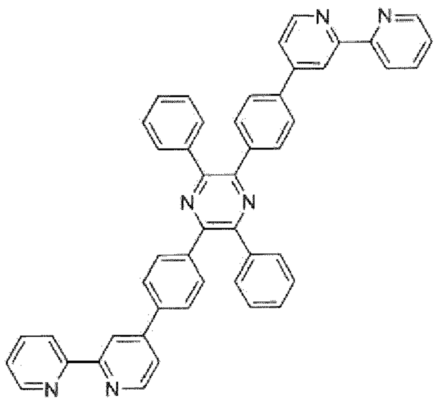

B-33

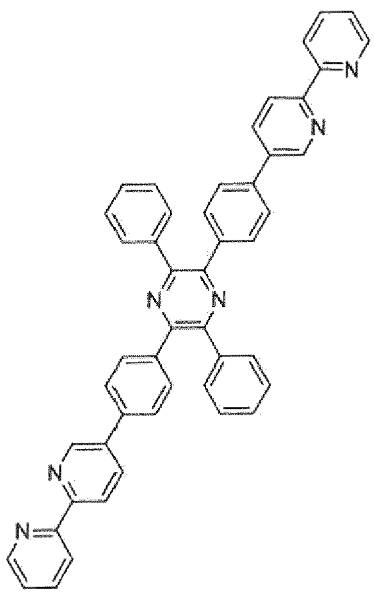

B-34

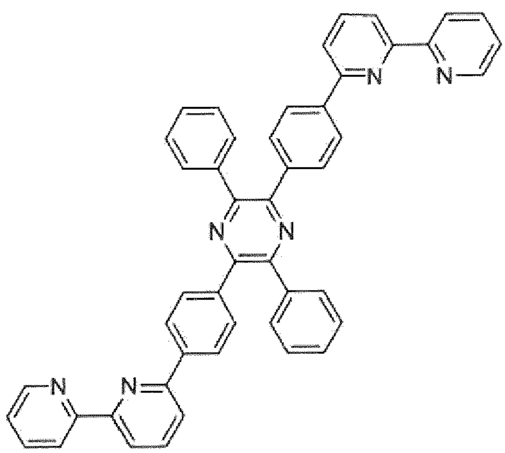

B-35

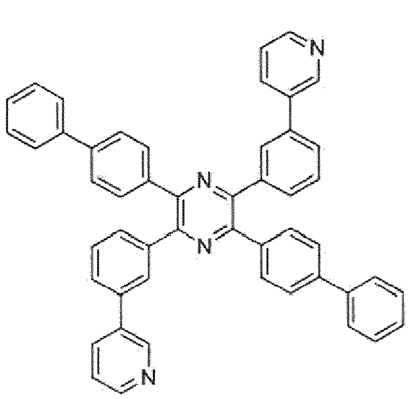

B-36

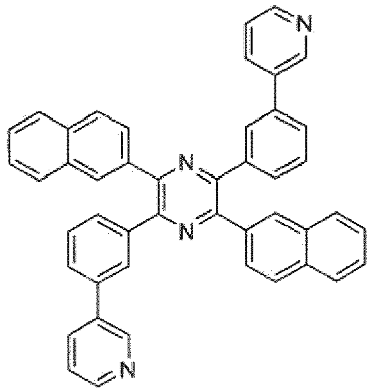

B-37

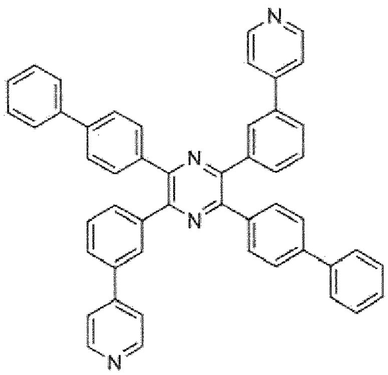

B-38

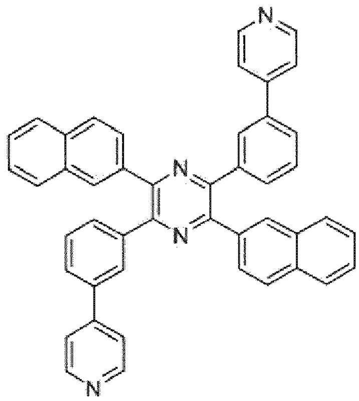

B-39

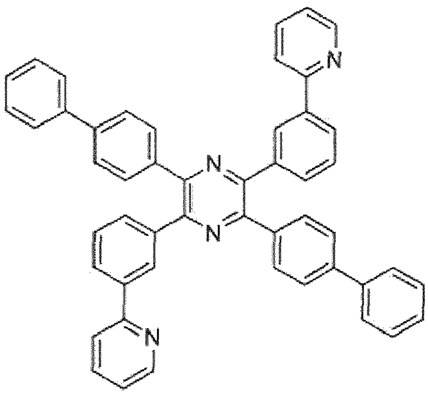

B-40

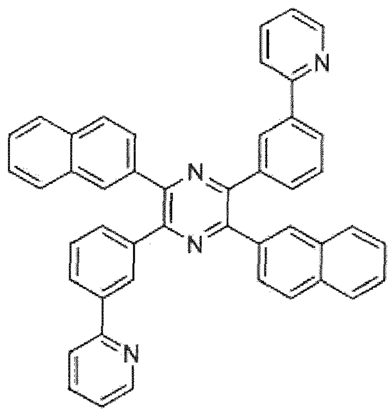

B-41

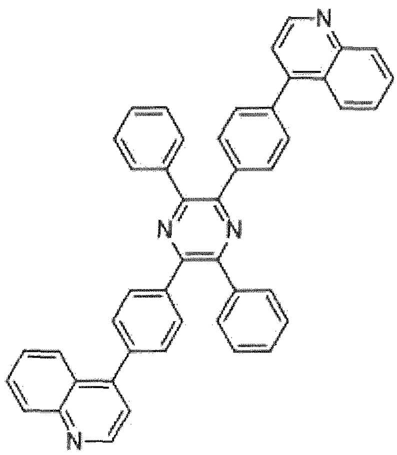

B-42

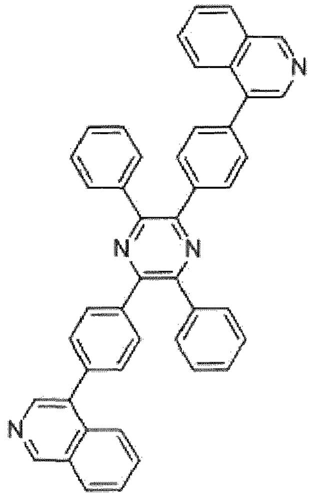

B-43

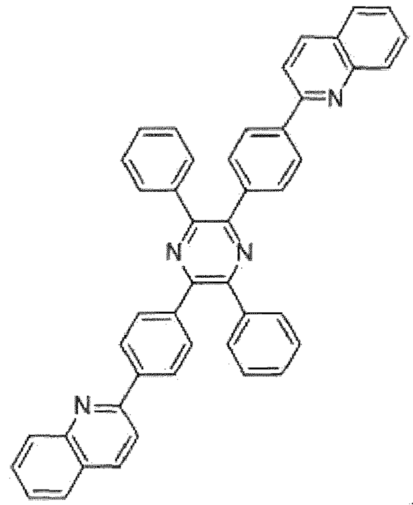

B-44.

**7.** Organic light emitting diode according to any of the preceding claims, wherein the first electron transport layer comprises a compound of formula (I)

$$(Ar^1\text{-}A_c)_a\text{-}X_b \qquad \text{(I)}$$

- a is 1 or 2;
- b is 1 or 2;
- c is o or 1;
- $Ar^1$ is independently selected from substituted or unsubstituted $C_6$ to $C_{60}$ aryl or substituted or unsubstituted $C_2$ to $C_{42}$ heteroaryl;

  - wherein, if $Ar^1$ is substituted, the one or more s ubstituent(s) are independently selected from the group consisting of D, $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;
  - wherein each $C_6$ to $C_{12}$ aryl substituent on $Ar^1$ and each $C_3$ to $C_{11}$ heteroaryl substituent on $Ar^1$ may be substituted with D, $C_1$ to $C_4$ alkyl or halogen;

- A is independently selected from substituted or unsubstituted $C_6$ to $C_{30}$ aryl;

  - wherein, if A is substituted, the one or more substituent(s) are independently selected from the group consisting of D, $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;
  - wherein each $C_6$ to $C_{12}$ aryl substituent on A and each $C_3$ to $C_{11}$ heteroaryl substituent on X may be substituted with D, $C_1$ to $C_4$ alkyl or halogen;

- X is independently selected from the group consisting of substituted or unsubstituted $C_2$ to $C_{42}$ heteroaryl and substituted or unsubstituted $C_6$ to $C_{60}$ aryl;

  - wherein, if X is substituted, the one or more substituent(s) are independently selected from the group consisting of D, $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, 5 or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;
  - wherein each $C_6$ to $C_{12}$ aryl substituent on X and each $C_3$ to $C_{11}$ heteroaryl substituent on X may be substituted with D, $C_1$ to $C_4$ alkyl or halogen;

- the compound of formula (I) has a dipole moment of ≥ 0 D and ≤ 4 D;

**8.** Organic light emitting diode according to claim 7, wherein $Ar^1$ is independently selected from the group consisting of phenyl, naphthyl, anthracenyl, fluoranthenyl, xanthenyl, spiro-xanthenyl, fluorenyl, spiro-fluorenyl, triphenylsilyl, tetraphenylsilyl or a group having the formula (Ia)

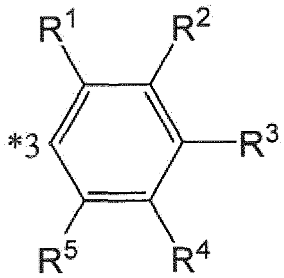
(Ia)

wherein

- the $Ar^1$ having the formula (Ia) is bonded at *1 to A;
- $R^1$ to $R^5$ are independently selected from the group consisting of H, substituted or unsubstituted $C_6$ to $C_{12}$ aryl and substituted or unsubstituted $C_4$ to $C_{10}$ heterorayl;
- wherein the one or more substituents, if present, are independently selected from D, $C_1$ to $C_4$ alkyl or halogen.

**9.** Organic light emitting diode according to any of the claims 7 or 8, wherein A is selected from the group consisting of phenylene, naphthylene, biphenyl-ylene and terphenylene which may be substituted or unsubstituted, respectively.

**10.** Organic light emitting diode according to any of the claims 7 to 9, wherein X is independently selected from the group consisting of triazinyl, pyrimidinyl, pyrazinyl quinazolinyl, benzoquinazolinyl, benzimidazolyl, quinolinyl, benzoquinolinyl benzoacridinyl, dibenzoacridinyl, fluoranthenyl, anthracenyl, naphthyl, triphenylenyl, phenathrolinyl, and dinaphthofuranyl which may be substituted or unsubstituted, respectively.

**11.** Organic light emitting diode according to any of the preceding claims, wherein the second electron transport layer further comprises a compound (III), wherein the compound (III) comprises 8 to 13 aromatic or heteroaromatic rings.

**12.** Organic light emitting diode according to claim 11, wherein the compound (III) comprises two or more heteroaromatic rings and the heteroaromatic rings are separated from each other by at least one aromatic ring which is free of a heteroatom.

**13.** Organic light emitting diode according to any of the preceding claims, wherein the organic light emitting diode further comprises an electron injection layer and the electron injection layer is arranged between the second electron transport layer and the cathode.

**14.** Device comprising the organic light emitting diode according to any of the preceding claims, wherein the device is a display device or a lighting device.

**15.** Compound of formula (IV)

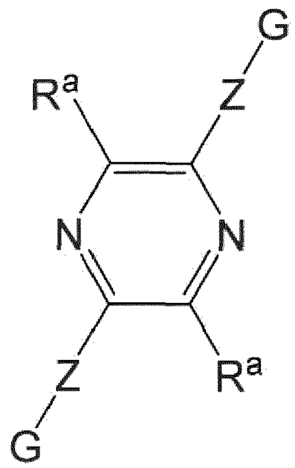

(IV)

wherein

$R^a$ is independently selected from the group consisting of substituted or unsubstituted $C_6$ to $C_{24}$ aryl and substituted or unsubstituted $C_3$ to $C_{18}$ heteroaryl, wherein if $R^a$ is substituted, the one or more substituents are independently elected from the group consisting of D, phenyl, and $C_1$ to $C_{12}$ alkyl;
Z is independently selected from the group consisting of a single bond and $C_6$ to $C_{24}$ arylene;
G is independently selected from (IVa-1) to (IVa-3)

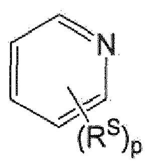 (IVa-1) 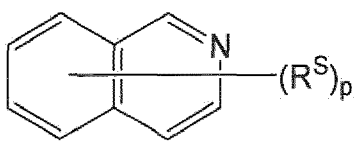 (IVa-2) 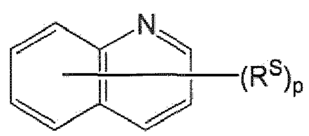 (IVa-3);

p is an integer from o to 4 for IVa-1;
p is an integer from o to 6 for IVa-2 and IVa-3; and
$R^S$ is independently selected from the group consisting of D, $C_1$ to $C_{12}$ aryl, $C_1$ to $C_{12}$ alkyl, and pyridyl, wherein the respective $C_1$ to $C_{12}$ aryl and pyridyl can be unsubstituted or substituted with one or more of D and $C_1$ to $C_4$ alkyl.

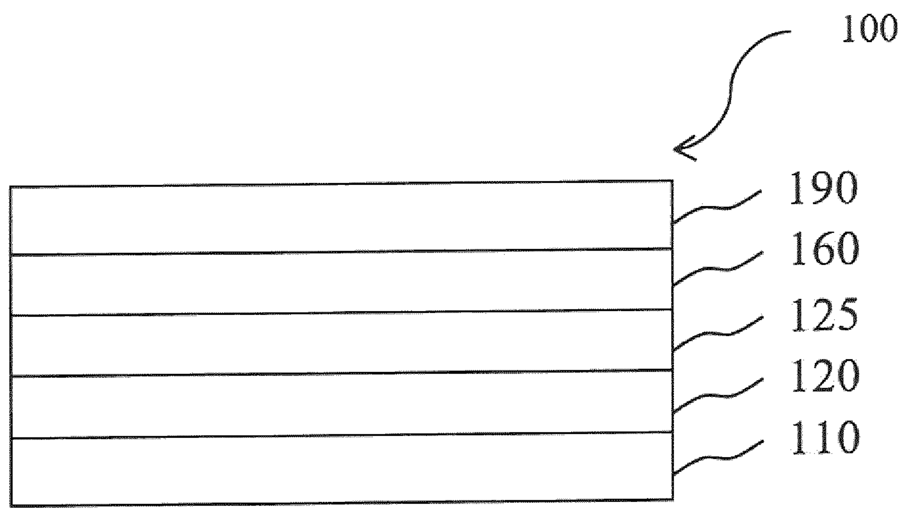


Fig.1

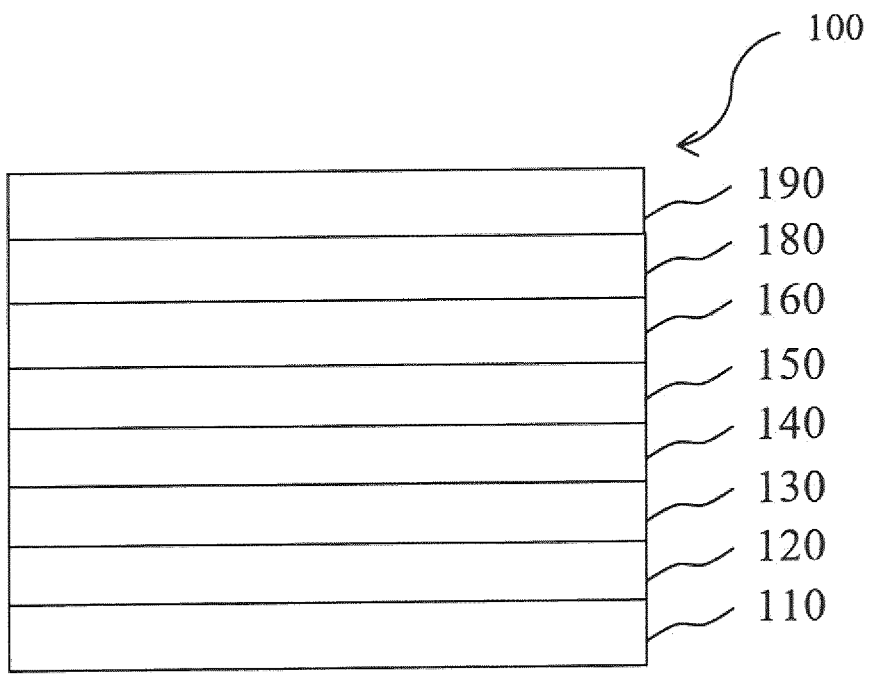


Fig.2

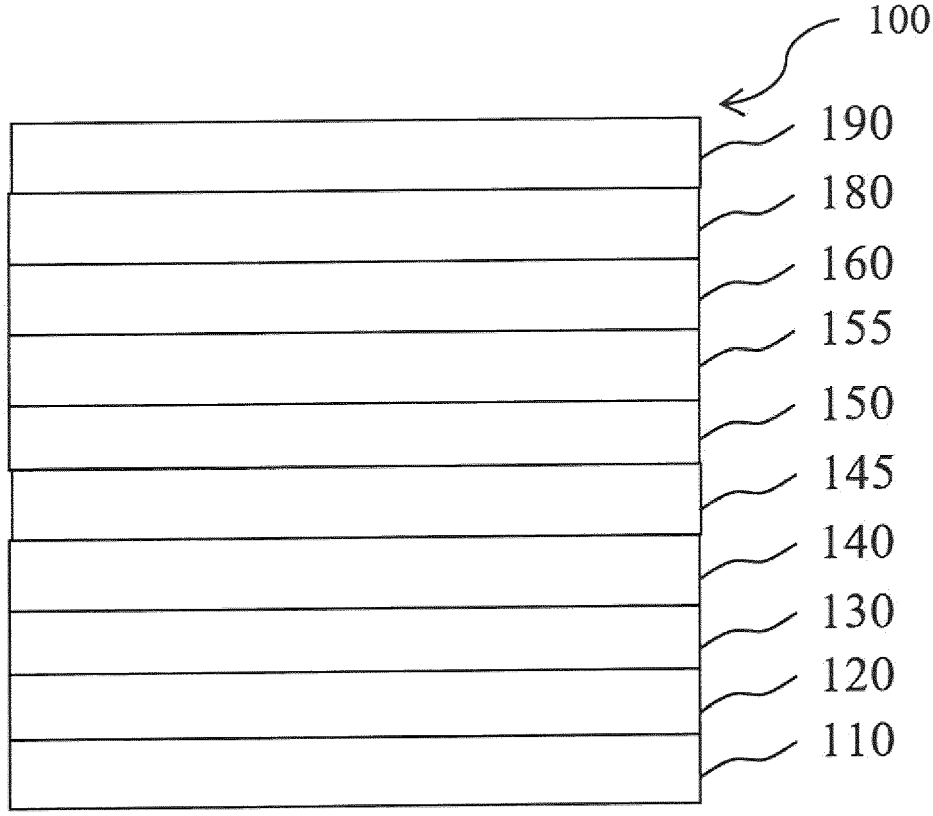


Fig.3

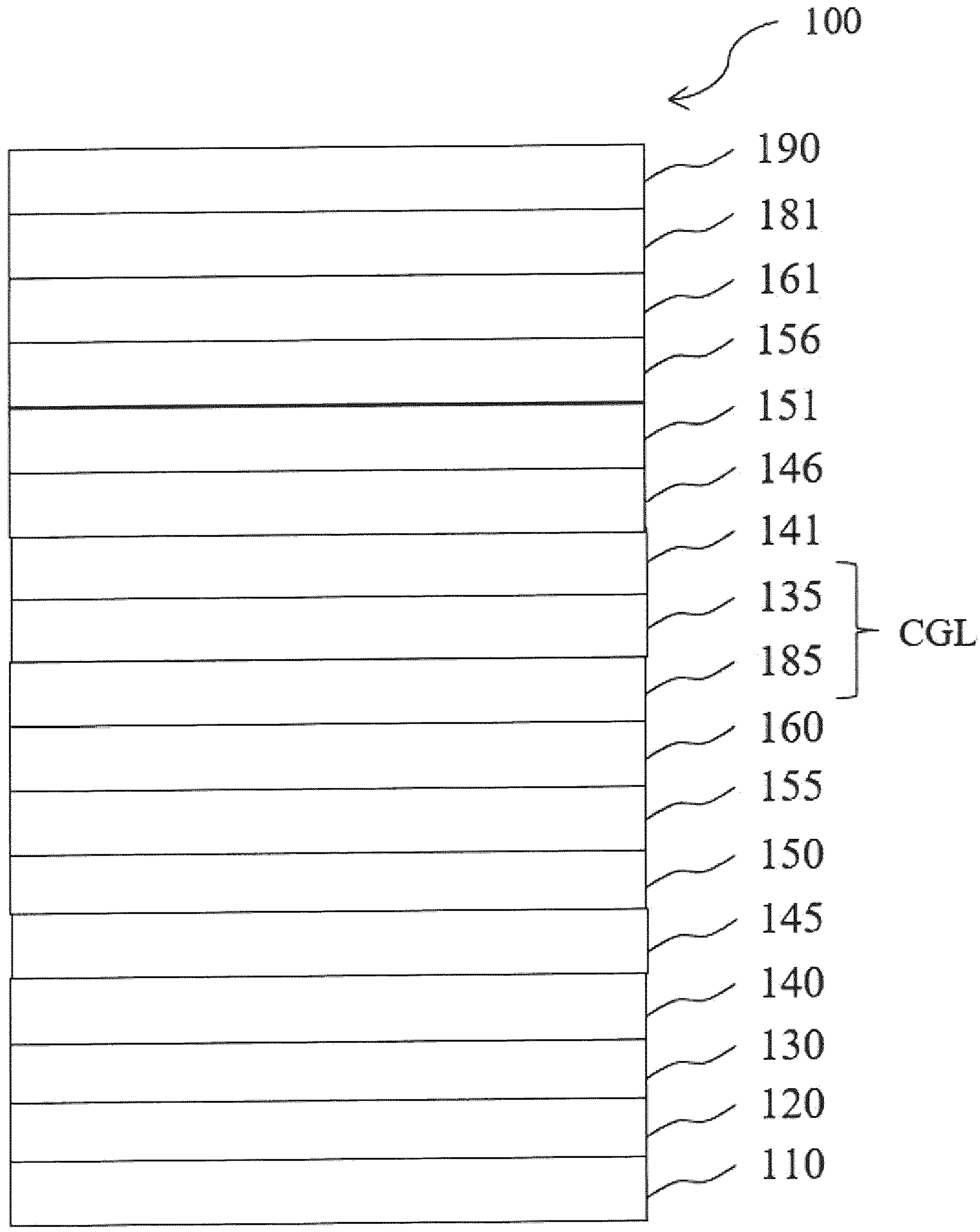
100
190
181
161
156
151
146
141
135
185
CGL
160
155
150
145
140
130
120
110

Fig.4

European Patent Office

# EUROPEAN SEARCH REPORT

Application Number
EP 23 15 6500

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/250279 A1 (NOVALED GMBH [DE]) 16 December 2021 (2021-12-16) * claims 1,7-9,15,17,21; table 3; compounds B-8, B-2 * * page 125, line 8 * ----- | 1,5-14 | INV. H10K85/60 ADD. H10K50/16 |
| X | EP 3 182 478 A1 (NOVALED GMBH [DE]) 21 June 2017 (2017-06-21) * paragraphs [0095], [0096], [0147], [0188], [0275], [0277], [0278], [0280]; examples 11-15; table 9; compounds ETM-49 * ----- | 1-4 | |
| X | EP 3 798 213 A1 (NOVALED GMBH [DE]) 31 March 2021 (2021-03-31) * compounds G29, G36 * ----- | 15 | |
| X | CN 110 003 186 A (JIANGSU SUNERA TECH CO LTD) 12 July 2019 (2019-07-12) * page 10; compound 16 * ----- | 15 | TECHNICAL FIELDS SEARCHED (IPC) |
| X | CN 107 629 011 A (JIANGSU SANYUE PHOTOELECTRIC TECH CO LTD) 26 January 2018 (2018-01-26) * compounds 10,24 * ----- | 15 | H10K |

The present search report has been drawn up for all claims

| Place of search | Date of completion of the search | Examiner |
|---|---|---|
| The Hague | 4 July 2023 | Fratiloiu, Silvia |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 23 15 6500

This annex lists the patent family members relating to the patent documents cited in the above-mentioned European search report. The members are as contained in the European Patent Office EDP file on
The European Patent Office is in no way liable for these particulars which are merely given for the purpose of information.

04-07-2023

| Patent document cited in search report | Publication date | Patent family member(s) | Publication date |
|---|---|---|---|
| WO 2021250279 A1 | 16-12-2021 | CN 115868257 A | 28-03-2023 |
| | | EP 4165694 A1 | 19-04-2023 |
| | | KR 20230023005 A | 16-02-2023 |
| | | WO 2021250279 A1 | 16-12-2021 |
| EP 3182478 A1 | 21-06-2017 | CN 108463898 A | 28-08-2018 |
| | | EP 3182478 A1 | 21-06-2017 |
| | | JP 7022063 B2 | 17-02-2022 |
| | | JP 2019500752 A | 10-01-2019 |
| | | KR 20180097653 A | 31-08-2018 |
| | | US 2019006589 A1 | 03-01-2019 |
| | | WO 2017102822 A1 | 22-06-2017 |
| EP 3798213 A1 | 31-03-2021 | CN 114502547 A | 13-05-2022 |
| | | EP 3798213 A1 | 31-03-2021 |
| | | EP 4034533 A1 | 03-08-2022 |
| | | KR 20220069958 A | 27-05-2022 |
| | | US 2022393112 A1 | 08-12-2022 |
| | | WO 2021058759 A1 | 01-04-2021 |
| CN 110003186 A | 12-07-2019 | CN 106967052 A | 21-07-2017 |
| | | CN 110003186 A | 12-07-2019 |
| CN 107629011 A | 26-01-2018 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005086251 A
- EP 1837926 B1
- WO 2007107306 A
- WO 2007107356 A
- EP 2722908 A1
- EP 1970371 A1
- WO 2013079217 A1
- WO 2014088047 A
- US 2008265216 A
- WO 2016171358 A
- JP 2004281390 B
- WO 2013079217 A
- US 2016322581 A
- WO 2021105518 A
- WO 20120794 A

### Non-patent literature cited in the description

- **YASUHIKO SHIROTA ; HIROSHI KAGEYAMA.** *Chem. Rev.*, 2007, vol. 107, 953-1010
- *CHEMICAL ABSTRACTS,* 74134-61-5
- *CHEMICAL ABSTRACTS,* 929203-04-3
- *CHEMICAL ABSTRACTS,* 1798781-99-3
- *CHEMICAL ABSTRACTS,* 939430-30-5
- *CHEMICAL ABSTRACTS,* 14221-01-3
- *CHEMICAL ABSTRACTS,* 1364603-07-5
- *CHEMICAL ABSTRACTS,* 1613079-70-1
- *CHEMICAL ABSTRACTS,* 1224447-88-4
- *CHEMICAL ABSTRACTS,* 2032364-64-8
- *CHEMICAL ABSTRACTS,* 721969-94-4
- *CHEMICAL ABSTRACTS,* 850918-68-2
- *CHEMICAL ABSTRACTS,* 1242056-42-3
- *CHEMICAL ABSTRACTS,* 2437303-42-7